(19) Europäisches Patentamt
European Patent Office
Office européen des brevets

(11) Publication number: **0 350 163 A2**

(12) **EUROPEAN PATENT APPLICATION**

(21) Application number: 89305691.1

(51) Int. Cl.[4]: C07K 5/02 , A61K 37/64

(22) Date of filing: 06.06.89

Claims for the following Contracting State: ES.

The title of the invention has been amended (Guidelines for Examination in the EPO, A-III, 7.3).

(30) Priority: 09.06.88 GB 8813671
13.12.88 GB 8829065
18.03.89 GB 8906262

(43) Date of publication of application:
10.01.90 Bulletin 90/02

(84) Designated Contracting States:
AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Smith, Stephen Allan
Beecham Pharmaceuticals Coldharbour Road
The Pinnacles Harlow Essex CM19 5AD(GB)
Inventor: Ham, Peter
Beecham Pharmaceuticals Coldharbour Road
The Pinnacles Harlow Essex CM19 5AD(GB)
Inventor: Nash, David John
Beecham Pharmaceuticals Coldharbour Road
The Pinnacles Harlow Essex CM19 5AD(GB)

(74) Representative: Jones, Pauline et al
Beecham Pharmaceuticals Patents & Trade Marks Dept. Great Burgh Yew Tree Bottom Road
Epsom Surrey KT18 5XQ(GB)

(54) **Renin inhibitory peptides.**

(57) Compounds of formula (I), and pharmaceutically acceptable salts thereof:

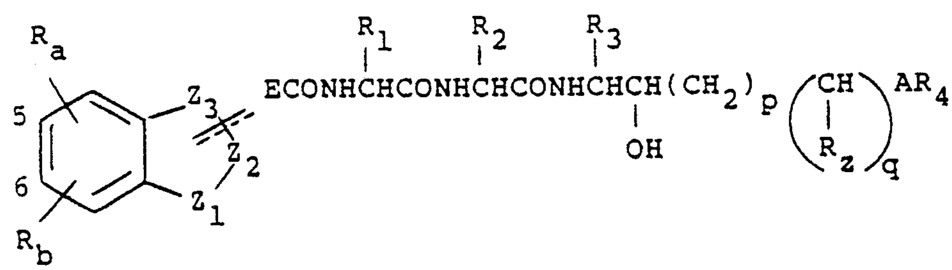

(I)

(I)

wherein
$Z_1$, $Z_2$, $Z_3$ and the carbon atoms to which $Z_1$ and $Z_3$ are attached, form a 5-membered non-aromatic heterocyclic ring;
E is absent or is $(CH_2)_n$ or $CH(CH_2)_{n-1}$ wherein n is 1 to 4;
A is -CONH-, -NHCO-, -COO-, $-S(O)_r-$ wherein r is 0, 1 or 2, or $-CH_2-$;

EP 0 350 163 A2

p is 0, 1 or 2;
q is 0 or 1;
$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl or $C_{3-8}$ cycloalkyl;
$R_4$ is $C_{1-6}$ alkyl or $C_{3-8}$ cycloalkyl; and
the dashed line represents an optional bond (when E is present); which are renin inhibitors, useful in the treatment of hypertension.

## NOVEL COMPOUNDS

The invention relates to novel compounds having pharmacological activity, to processes for their preparation, to pharmaceutical preparations containing them and to their use in medicine.

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in plasma, and in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have been described as useful in the treatment of hypertension.

A group of compounds has now been discovered, which inhibit the enzyme renin and therefore have potential blood pressure lowering activity, useful in the treatment of hypertension. Compounds having an associated mechanism of action have also been described as possessing anti-retroviral activity and the present compounds may therefore be of potential use in the treatment of diseases caused by retroviruses including human immunodeficiency virus (HIV-I and II) and HTLV I and II. They may also be of potential use in the treatment of other cardiovascular disorders, such as congestive heart failure, and have beneficial effects on learning and memory and mood elevation activity of potential use in the treatment of CNS disorders such as Alzheimer's disease and depression.

Accordingly, the present invention provides a compound of formula (I), or a pharmaceutically acceptable salt thereof:

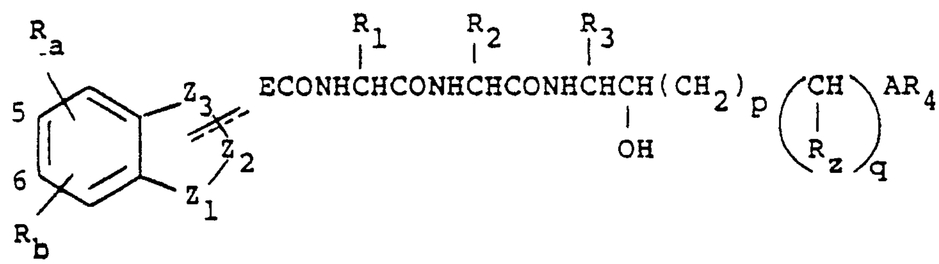

(I)

wherein

$Z_1$, $Z_2$, $Z_3$ and the carbon atoms to which $Z_1$ and $Z_3$ are attached, form a 5-membered non-aromatic heterocyclic ring;

E is absent or is $(CH_2)_n$ or $CH(CH_2)_{n-1}$ wherein n is 1 to 4;

A is -CONH-, -NHCO-, -COO-, $-S(O)_r-$ wherein r is 0, 1 or 2, or $-CH_2-$;

p is 0, 1 or 2;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl or $C_{3-8}$ cycloalkyl;

$R_4$ is $C_{1-6}$ alkyl or $C_{3-8}$ cycloalkyl; and

the dashed line represents an optional bond (when E is present).

Values of $Z_1$, $Z_2$ and $Z_3$ include wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, and one of $Z_2$ and $Z_3$, is $CH_2$, CH or C when the other is S or O, or is $CH_2$, CH, C, NR or N when the other is SO or $SO_2$; or $Z_2$ is SO, $SO_2$ or C=O when one of $Z_1$ and $Z_3$ is N and the other is O, S or NR; or $Z_1$ is CO, $Z_2$ is O and $Z_3$ is CH.

Examples of $Z_1$, $Z_2$ and $Z_3$ therefore include

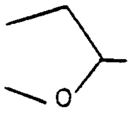 , 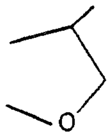 , 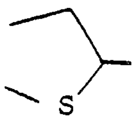 , 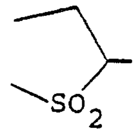 ,

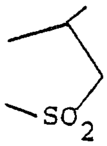 , 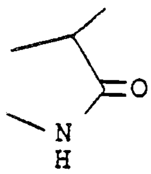 , 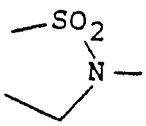 , 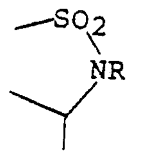

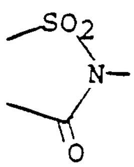 , 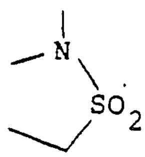 , 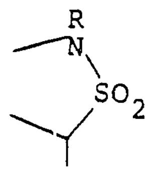 , 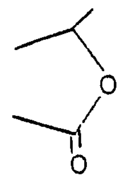

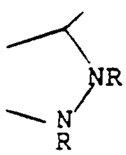 , 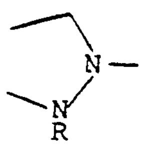 , 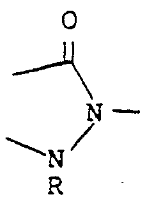 , 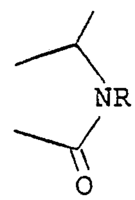

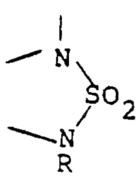 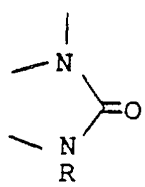 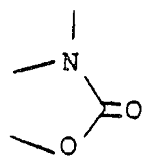

Preferably $Z_1$ is $SO_2$ or O (more preferably $SO_2$), $Z_2$ is $CH_2$, $Z_3$ is CH and E is attached at $Z_3$.

Suitable examples of $R_a$ and $R_b$ include a moiety selected from halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy. $R_a$ or $R_b$ may also be a mercapto substituent such that the resulting compound is a disulphide as described in Example 42.

Suitable examples of substituents in optionally substituted amino groups in $R_a/R_b$ then include one or two groups independently selected from $C_{1-6}$ alkyl or $C_{1-6}$ alkyl substituted by carboxy, $C_{1-6}$ alkoxycarbonyl hydroxy or amino group.

Alternatively, $NR_5R_6$ or other substituted amino groups in $R_a/R_b$ may be pyrrolidinyl, piperidinyl, morpholinyl or piperazinyl optionally N-substituted by $C_{1-6}$ alkyl.

Suitable acyl groups in $R_7$ when $C_{1-6}$ alkyl substituted by acylamino, include $C_{1-7}$ alkanoyl, or

optionally substituted benzoyl. Suitable protecting groups in protected amino include benzyloxycarbonyl and t-butyloxycarbonyl.

Preferably one of $R_a$ and $R_b$ is hydrogen and the other is 5- or 6- $CH_2NH_2$ or $CO_2H$, 5-$CH_2NHCH_2CO_2H$ or 5-$SO_2CH_2CO_2H$. (It will be appreciated that the 5/6 numbering depicted in formula (I) may be reversed for certain values of $Z_1$, $Z_2$, $Z_3$).

In regard to values of A and p and q, reference is hereby made to the following literature:-

1. J. Med. Chem. 1988, 31, 1918
(p = 1, q = 0, A = -CONH-)
2. J. Med Chem. 1987, 30, 1224
(p = 2, q = 0, A = -NHCO-)
3. J. Med. Chem. 1987, 31, 701
(p = O, q = 0, A = -COO-)
4. J. Med. Chem. 1987, 31, 1839
(p = 1, q = 1, A = -CONH-)
5. J. Med. Chem. 1987, 30, 1729 and refs. therein
(p = 2, q = 1, A = $S(O)_r$)
6. WO 88/05050
(p = 0, q = 1, $R_2$ = OH, A = $CH_2$, $R_z$ = OH)
7. EP-A-172346
(p = 0, q = 1, $R_2$ = OH, A = $CH_2$, $R_z$ = H)

Suitable values of $R_9$ and $R_{11}$ when aryl include phenyl or naphthyl and when heteroaryl, include a 5- or 6-membered monocyclic or 9- or 10- membered bicyclic of which 5- or 6- membered monocyclic heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5-or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furanyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Examples of such groups include 2- and 3-benzofuranyl, 2- and 3-benzothienyl, and 2- and 3-indolyl, and 2- and 3-quinolyl and, for $R_{11}$, 4-benzimidazolyl.

Suitable examples of groups or atoms for optional substitution of $R_5$ when benzyl and $R_9$ and $R_{11}$ include one, two or three substituents independently selected from $C_{1-4}$ alkyl, $C_{1-4}$ alkoxy, halo (such as fluoro, chloro, bromo), hydroxy, nitro, and cyano.

Preferred examples of $R_9$ include phenyl and naphthyl, and preferred examples of $R_{11}$ when aryl or heteroaryl include phenyl, imidazol-4-yl and -2-yl, pyrazol-1-yl and 4-methylpyrazol-1-yl.

Preferably the amino acid residue containing $R_2$ is Leu, $\beta$-pyrazolylalanine or His.

There is a group of compounds within formula (I) wherein

E is $(CH_2)n$ wherein n is 0, 1 or 2;

$Z_1$ is O, S, SO, $SO_2$ or NR wherein R is hydrogen or $C_{1-6}$ alkyl;

$Z_2$ is $CR_xR_y$ wherein $R_x$ is hydrogen and $R_y$ is hydrogen or $R_y$ is attached to E in formula (I), or $R_x$ and $R_y$ together are an oxo group;

$Z_3$ is $CH_2$ or is CH attached to E in formula (I);

$R_{11}$ is $C_{1-6}$ alkyl, $C_{3-8}$ cycloalkyl, or optionally substituted aryl or heteroaryl; and

the remaining variables are as defined in respect of formula (I).

Abbreviations used herein are as follows:

| Amino Acid Residue | Abbreviations |
|---|---|
| histidine | His |
| isoleucine | Ile |
| leucine | Leu |
| 1-naphthylalanine | NAla |
| norleucine | Nle |
| phenylalanine | Phe |

The α-amino acid components are in the (S)-configuration (or L-form).

In a particular aspect, the present invention provides a compound of formula (IA) or a pharmaceutically acceptable salt thereof:

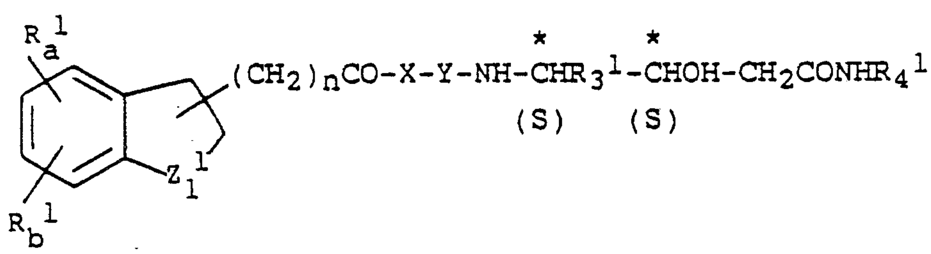

wherein (IA)
X is Phe or NAla;
Y is Leu, Ile, Nle or His;
$Z_1{}^1$ is O, S, SO or $SO_2$;
n is 0, 1 or 2;
$R_a{}^1$ and $R_b{}^1$ are independently selected from hydrogen, halogen, $C_{1-6}$ alkyl, $C_{1-6}$ alkoxy, nitro, cyano, $SO_3H$, CHO, $C_{1-6}$ alkyl substituted by hydroxy or amino optionally substituted by a $C_{1-7}$ alkanoyl group or by one or two $C_{1-6}$ alkyl groups, or a group $NR_5{}^1R_6{}^1$, $SO_2NR_5{}^1R_6{}^1$, $S(O)_mR_5{}^1$, $CO_2R_5{}^1$, $NHCONR_5{}^1R_6{}^1$ or $NHCOR_5{}^1$ wherein $R_5{}^1$ and $R_6{}^1$ are independently selected from hydrogen or $C_{1-6}$ alkyl and m is 0, 1 or 2;
$R_3$ is cyclohexylmethyl; and
$R_4$ is $C_{4-5}$ alkyl.

Often, in formula (IA), X is Phe and Y is Leu.

Suitable values for alkyl groups in $R_a$, $R_b$, $R_z$, R and $R_2$ to $R_{12}$ in formulae (I) and (IA) include methyl, ethyl, n- and iso-propyl, n-, sec-, iso- and tert-butyl, n-, iso-, sec-, tert- and neo-pentyl. $C_{3-8}$ cycloalkyl groups are cyclopropyl, cyclobutyl, cyclopentyl, cyclohexyl, cycloheptyl and cyclooctyl.

Suitable values for $R_a$ and $R_b$ halo include fluoro, chloro, bromo and iodo, preferably chloro or bromo.

Preferably $R_4$ is iso-butyl.

The right hand sequence in formula (I) and (IA) is preferably 4(S)-amino-5-cyclohexyl-3-(S)-hydroxypentanoic acid isobutylamide (ACHPAA), from the amino acid containing $R_3$ or $R_3{}^1$.

Preferably n is 1, 2 or 3.

Preferably E is attached at the 3-position with respect to $Z_1$.

Pharmaceutically acceptable salts include acid addition salts which may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, trifluoroacetic acid, propionic acid, lactic acid, citric acid, tartaric acid, fumaric acid, malic acid, succinic acid, salicylic acid or acetylsalicylic acid.

Phamaceutically acceptable salts may also include alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)amine.

It will be appreciated that the compounds of the invention may exist as solvates, such as hydrates, and these are included whenever, a compound of formula (I) or a salt thereof, is herein referred to.

The compounds of formula (I) wherein E is attached to $Z_1/Z_2/Z_3$ when CH, and (IA) have at least one asymmetric centre in addition to those attached to $R_1$ and $R_2$ (in X and Y) and $R_3$ and those indicated in formula (IA), and are therefore capable of existing in more than one stereoisomeric form. The invention

extends to each of these forms and to mixtures thereof. Preferably the configuration at the carbon atoms bearing $R_1$, $R_2$ and $R_3$ is the (S)-configuration.

It will be appreciated that, when the optional bond depicted in formula (I) is present, the compounds are capable of existing in E and Z (trans and cis) forms. The invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

A compound of the invention may be prepared by those methods known in the art for the synthesis of compounds of analogous structure, such as peptides, and in this regard reference is made, by way of illustration only, to the literature reference: S.R. Pettit, "Synthetic Peptides", (Elsevier Scientific Publishing Co. 1976).

The present invention also provides a compound of the present invention which has been prepared synthetically.

A compound of the present invention may, for example, be formed by the sequential coupling of appropriate amino acids with the acid of formula (II):

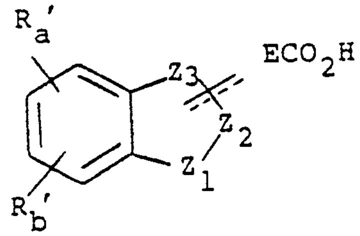

(II)

wherein $R_a'$ and $R_b'$ are $R_a$ and $R_b$ respectively or groups or atoms convertible thereto; or by the initial preparation and subsequent coupling of peptide subunits with the acid of formula (II), the subunits themselves prepared in stepwise manner; in either case classical solution chemistry methods analogous to those used in peptide synthesis, may be employed.

The coupling reactions may be effected by, for example, activating the reacting carboxyl group of the acid of formula (II) or amino acid, and reacting this with the amino group of the substrate unit. Details of suitable, optional activating and protecting (masking) groups and of suitable reaction conditions (for the coupling reactions and for the introduction and removal of protecting groups) giving, preferably, the minimum of racemisation, may be found in the above-referenced literature.

It will be appreciated that, when A is other than CONH- or -COO-, the C-terminus group is other than an amino acid, in which case the coupling is carried out with an appropriate other precursor, as described in the aforementioned literature references 2., 5., 6. and 7.

Accordingly, the present invention further provides a process for the preparation of a compound of formula (I) or a pharmaceutically acceptable salt thereof which comprises reacting a reagent of the formula (III):

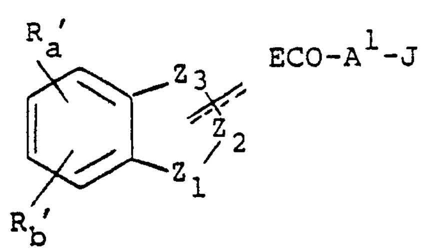

(III)

wherein $R_a'$ and $R_b'$ are $R_a$ and $R_b$ respectively or groups or atoms convertible thereto; $A^1$ is absent or represents an appropriate amino acid or dipeptide unit; J is OH or a leaving group; and the remaining variables are as hereinbefore defined; with a reagent of the formula (IV):

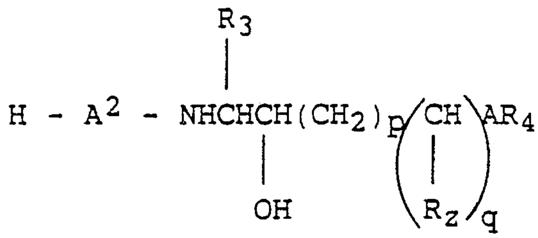

(IV)

wherein

$A^2$ is absent or represents an appropriate amino acid or dipeptide unit, such that $A^1$ + $A^2$ is $-NHCHR_1CONHCHR_2CO-$, and the remaining variables are as hereinbefore defined; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'/R_b'$ to $R_a$ and/or $R_b$ and/or forming a pharmaceutically acceptable salt thereof.

Suitable examples of J when a leaving group include halo, such as chloro or bromo, and other suitable groups which are displaceable by an amino nucleophile, such as $C_{1-6}$ alkoxycarbonyloxy.

It is generally preferred, especially when $A^1$ is not absent, however, that J is OH, and a suitable coupling reagent or dehydrating catalyst is used to effect the reaction, such as dicyclohexylcarbodiimide and those described in the Descriptions and Examples hereinafter.

Deprotection of $A^1$ and/or $A^2$ takes place conventionally, in accordance with the particular protecting group(s) employed.

Pharmaceutically acceptable salts may be formed conventionally.

$Z_1$, $Z_2$ or $Z_3$ when S or SO may be converted to SO or $SO_2$ respectively (or S to $SO_2$) by conventional oxidation methods, such as those described hereinafter for conversion (vi) for $R_a/R_b$.

It will be apparent that compounds of the formula (I) containing an $R_a'/R_b'$ group which is other than $R_a$, $R_b$ and which is convertible to $R_a/R_b$ group, are useful novel intermediates. A number of such conversions is possible, not only for the final product compounds of formula (I) when $R_a'/R_b'$ are other than $R_a$, $R_b$, but also within $R_a/R_b$, and also for their intermediates as follows:

(i) a hydrogen substituent is convertible to a nitro substituent by nitration;

(ii) a nitro substituent is convertible to an amino substituent by reduction;

(iii) a $C_{1-7}$ acylamino substituent is convertible to an amino substituent by deacylation;

(iv) an amino substituent is convertible to a $C_{1-7}$ acylamino substituent by acylation with a carboxylic acid derivative;

(v) a hydrogen substituent is convertible to a halogen substituent by halogenation;

(vi) an $C_{1-6}$ alkylthio or a $C_{1-6}$ alkylsulphinyl substituent is convertible to a $C_{1-6}$ alkylsulphinyl or $C_{1-6}$ alkylsulphonyl substituent respectively, by oxidation;

(vii) an amino, aminosulphonyl, or $NHCONH_2$ substituent is convertible to a corresponding substituent which is substituted by one or two alkyl groups, by N-alkylation;

(viii) an amino substituent is convertible to a group $NHCONH_2$, by reaction with potassium cyanate and acid;

(ix) a hydrogen substituent is convertible to an aminosulphonyl substituent by treatment with $ClSO_3H$ followed by $HNR_5R_6$;

(x) a cyano substituent may be converted to an aminomethyl substituent by reduction;

(xi) a bromo substituent may be converted to a cyano substituent by reaction with copper(I) cyanide.

Conversions (i) to (xi) are only exemplary and are not exhaustive of the possibilities. It will be appreciated that it is often desirable to carry out these conversions at an earlier stage, in the intermediate acid of formula (II), especially in regard to conversion (iii).

In regard to (i), nitration is carried out in accordance with known procedures.

In regard to (ii), the reduction is carried out with a reagent suitable for reducing nitroanisole to aminoanisole.

In regard to (iii), deacylation is carried out by treatment with a base, such as an alkali metal hydroxide.

In regard to (iv), the acylation is carried out with an acylating agent, such as the corresponding acid or acid chloride. Formylation is carried out with the free acid.

In regard to (v), halogenation is carried out with conventional halogenating agents.

In regard to (vi), oxidation is carried out at below ambient temperatures in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or

with aqueous hydrogen peroxide.

In regard to (vii), alkylation is carried out with a corresponding alkylating agent such as the chloride or bromide under conventional conditions.

In regard to (viii), conversion to a ureido derivative is carried out by reaction with potassium cyanate in acidic methanol, at ambient temperature.

In regard to (ix), the mixing with $ClSO_3H$ takes place at low temperatures around 0°C and allowed to warm to ambient temperature, as described in Description 38(a) hereinafter. The subsequent reaction with the amine takes place at ambient temperature, in a solvent such as ethanol.

In regard to (x), the reduction takes place by reaction with sodium borohydride/cobalt chloride as described in Example 20a hereinafter.

In regard to (xi), the reaction takes place under conventional conditions, as described in Description 25 hereinafter.

Compounds of the general formulae (II) and (III) may themselves be prepared by standard techniques analogous to those described above.

The acids of formula (II) wherein n is 0 are either known compounds or are prepared by analogous methods to these and for structurally similar known compounds, for example, as in the Descriptions hereinafter.

Some of the acids of formula (II) wherein n is 1 or 2, in particular, wherein $Z_1$ is S, SO or $SO_2$, are believed to be novel, and form an aspect of the present invention. They may be prepared from the corresponding acids wherein n is 0 or (when n is 1) from the corresponding malonic acid derivative, which may then be converted to the corresponding n = 2 derivative by conventional methods of homologation, such as the Grignard and nitrile methods. Other novel acids of formula (II) may be prepared by methods described in the Descriptions, such as 10, 40 and 41.

When n is 3, the acids may be prepared from the corresponding n=1 aldehyde by reduction, followed by Wittig homologation, as described in Description 53 hereinafter.

The preparation of the amino acid of formula (V):

$$H_2N-\underset{(S)}{\overset{*}{C}}HR_3^1-\underset{(S)}{\overset{*}{C}}HOH-CH_2CO_2H \qquad (V)$$

is described in J. Med. Chem 1985, <u>28</u>, 1779-1790.

As regards the appropriate intermediates wherein A is other than -CONH-, reference is hereby made to the literature references 2., 3., 5., 6. and 7. listed hereinbefore.

It will be appreciated that protected forms of compounds of the present invention are novel intermediates and form an aspect of the invention.

Particularly suitable methods of preparation of the compounds of the present invention are as described in the Descriptions and Examples hereinafter. The couplings may be carried out sequentially beginning by coupling the acid of formula (II) with, for example, phenylalanine or 1-naphthylalanine, followed by coupling with Y, for example, leucine or histidine, and finally, coupling with the amino acid of formula (V). In a preferred aspect, however, either the acid of formula (II) is coupled with a tripeptide unit formed between the amino acid of formula (V); leucine, histidine or other $R_2$ containing amino acid; and phenylalanine or naphthylalanine; or alternatively the acid of formula (II) is coupled with phenylalanine or naphthylalanine and this is coupled with a dipeptide unit formed between the $R_2$ containing amino acid and the amino acid of formula (V).

As mentioned previously the compounds of the invention have been found to be renin inhibitors, and therefore they are of potential use in the treatment of hypertension. They may also be of potential use in the other diseases and disorders hereinbefore referred to.

The present invention also provides a pharmaceutical composition which comprises a compound of formula (I) or a pharmaceutically acceptable salt thereof, and a pharmaceutically acceptable carrier. In particular, the present invention provides an anti-hypertensive pharmaceutical composition which comprises an anti-hypertensive effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

The present invention further provides a method of prophylaxis or treatment of hypertension in mammals including man, which comprises administering to the suffering mammal an anti-hypertensively effective amount of a compound of formula (I) or a pharmaceutically acceptable salt thereof.

An effective amount will depend on the relative efficacy of the compounds of the present invention, the severity of the hypertension being treated and the weight of the sufferer. However, a unit dose form of a composition of the invention may contain from 0.1 to 500 mg of a compound of the invention and more usually from 1 to 100 mg, for example 2 to 50 mg such as 2, 3, 4, 5, 10, 20 or 30mg. Such compositions may be administered from 1 to 6 times a day, more usually from 2 to 4 times a day, in a manner such that the daily dose is from 1 to 1000mg for a 70 kg human adult and more particularly from 5 to 500 mg.

No toxicological effects are indicated at the aforementioned dosage ranges.

The present invention further provides a compound of formula (I) or a pharmaceutically acceptable salt thereof for use in the treatment or prophylaxis of hypertension.

The following descriptions relate to the preparation of intermediates and the following examples (and Descriptions 56(e) and 58(e) relate to the preparation of compounds of formula (I). Tables I to VI show the structures of the intermediates and compounds of formula (I).

N.B. The prefix 'D' denotes a description number and the prefix 'E' denotes an example number.

In the following, the abbreviations used are:

| Abbreviation | Definition |
|---|---|
| ACHPAA | 4(S)-amino-5-cyclohexyl-3(S)-hydroxypentanoic acid isobutylamide |
| ACHPA | 4(S)-amino-5-cyclohexyl-3(S)-hydroxypentanoic acid |
| BOC | tert-butoxycarbonyl |
| CBZ | benzyloxycarbonyl |
| Phth | phthaloyl |

Table I

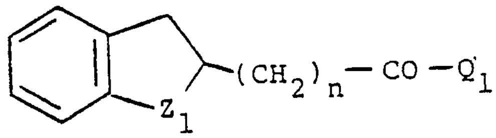

| Compound No. | $Z_1$ | n | $Q_1$ | Salt/Solvate (prepared from+) |
|---|---|---|---|---|
| D1(a) | O | 0 | PheOMe | |
| D1(b) | O | 0 | PheOH | |
| D1(c) | O | 0 | PheLeuOMe | |
| D1(d) | O | 0 | PheLeuOH | |
| D2(a) | O | 2 | PheOMe | |
| D2(b) | O | 2 | PheOH | |
| D2(c) | O | 2 | PheLeuOMe | |
| D2(d) | O | 2 | PheLeuOH | |
| D9(a) | S | 0 | OH | |
| D9(b) | S | 0 | OH | |
| D9(c) | S | 0 | PheOMe | |
| D9(d) | S | 0 | PheOH | |
| D9(e) | S | 0 | PheLeuOMe | |
| D9(f) | S | 0 | PheLeuOH | |
| D28 | $SO_2$ | 1 | OH | (D27) |
| E1 | O | 0 | PheLeuACHPAA | $0.5H_2O$ |
| E2 | O | 2 | PheLeuACHPAA | $0.5H_2O$ |
| E10 | S | 0 | PheLeuACHPAA | $0.5H_2O$ |
| E11 | $SO_2$ | 0 | PheLeuACHPAA | |
| E32 | $SO_2$ | 1 | PheLeuACHPAA | $0.5H_2O$ |
| E51 | $SO_2$ | 2 | PheLeuACHPAA | $0.5H_2O$ |

+ Descriptions only

Table II

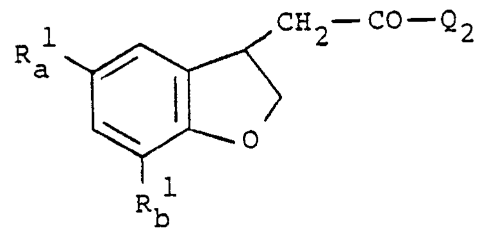

| Compound No. | $R_a^1$ | $R_b^1$ | $Q_2$ | Salt/Solvate (prepared from+) |
|---|---|---|---|---|
| D3(a) | H | OMe | PheOMe | |
| D3(b) | H | OMe | PheLeuOMe | |
| D3(c) | H | OMe | PheLeuOH | |
| D4(a) | H | H | PheOMe | |
| D4(b) | H | H | PheOH | |
| D4(c) | H | H | PheLeuOH | |
| D5(a) | $NO_2$ | H | OEt | |
| D5(b) | $NO_2$ | H | OH | |
| D5(c) | $NO_2$ | H | PheLeuOMe | |
| D5(d) | $NO_2$ | H | PheLeuOH | |
| D6(a) | $NH_2$ | H | PheLeuOMe | $CH_3CO_2H$ (D5c) |
| D6(b) | $H_2NCONH$ | H | PheLeuOMe | |
| D6(c) | $H_2NCONH$ | H | PheLeuOH | |
| D7(a) | H | $NO_2$ | OEt | |
| D7(b) | H | $NO_2$ | OH | |
| D7(c) | H | $NO_2$ | PheLeuOMe | |
| D7(d) | H | $NO_2$ | PheLeuOH | |
| D8(a) | $MeNHO_2S$ | H | OEt | |
| D8(b) | $MeNHO_2S$ | H | OH | |
| D8(c) | $MeNHO_2S$ | H | PheLeuOMe | |
| D8(d) | $MeNHO_2S$ | H | PheLeuOH | |
| D11(a) | OHC | H | OEt | |
| D11(b) | OHC | H | OH | |
| D11(c) | OHC | H | PheLeuOMe | |
| D11(d) | OHC | H | PheLeuOH | |

Table II (Cont'd.)

| Compound No. | $R_a^1$ | $R_b^1$ | $Q_2$ | Salt/ Solvate (prepared from+) |
|---|---|---|---|---|
| D12(a) | $HO_2C$ | H | OEt | (D11a) |
| D12(b) | $MeO_2C$ | H | $NHNH_2$ | |
| D14(a) | NC | H | OEt | (D11a) |
| D14(b) | NC | H | OH | |
| D14(c) | NC | H | PheLeuOMe | |
| D14(d) | NC | H | PheLeuOH | |
| D15(a) | $PhCH_2O_2C$ | H | OEt | (D12a) |
| D15(b) | $PhCH_2O_2C$ | H | OH | |
| D16(f) | $MeO_2S$ | H | OEt | |
| D16(g) | $MeO_2S$ | H | OH | |
| D36(a) | $NO_2$ | H | PheOH | |
| D36(b) | $NO_2$ | H | PheHisOMe | |
| D36(c) | $NO_2$ | H | PheHisOH | |
| D38(a) | $ClO_2S$ | H | OEt | |
| D38(b) | HS | H | OEt | |
| D39(a) | $^tBuO_2CCH_2S$ | H | OEt | (D38b) |
| D39(b) | $^tBuO_2CCH_2S$ | H | OH | |
| D39(c) | $^tBuO_2CCH_2S$ | H | $PheLeuOCH_2Ph$ | |
| D39(d) | $^tBuO_2CCH_2O_2S$ | H | $PheLeuOCH_2Ph$ | |
| D39(e) | $^tBuO_2CCH_2O_2S$ | H | PheLeuOH | |
| D48(a) | NC | H | PheHisOMe | (D14b) |
| D48(b) | NC | H | PheHisOH | |
| D50(a) | $PhthNCH_2CH_2S$ | H | OEt | (D38b) |
| D50(b) | $PhthNCH_2CH_2O_2S$ | H | OEt | |
| D50(c) | $H_2NCH_2CH_2O_2S$ | H | OEt | HCl |
| D50(d) | $CBZ-NHCH_2CH_2O_2S$ | H | OEt | |
| D50(e) | $CBZ-NHCH_2CH_2O_2S$ | H | OH | |

Table II (Cont'd.)

| Compound No. | $R_a^1$ | $R_b^1$ | $Q_2$ | Salt/ Solvate (prepared from+) |
|---|---|---|---|---|
| D56(a) | NC | H | PheOMe | (D14b) |
| D56(b) | $H_2NCH_2$ | H | PheOMe | |
| D56(c) | BOC-$NHCH_2$ | H | PheOMe | |
| D56(d) | BOC-$NHCH_2$ | H | PheOH | |
| D57(a) | OHC | H | PheHisOMe | (D11b) |
| D57(b) | OHC | H | PheHisOH | |
| D59 | $HO_2C$ | H | OH | (D12a) |
| E3 | H | OMe | PheLeuACHPAA | $0.5H_2O$ |
| E4 | H | H | PheLeuACHPAA | $H_2O$ |
| E5 | $NO_2$ | H | PheLeuACHPAA | $0.5H_2O$ |
| E6 | $NH_2$ | H | PheLeuACHPAA | $0.5H_2O$ $.CF_3CO_2H$ |
| E7 | $H_2NCONH$ | H | PheLeuACHPAA | $2H_2O$ |
| E8 | H | $NO_2$ | PheLeuACHPAA | |
| E9 | $MeNHO_2S$ | H | PheLeuACHPAA | |
| E13 | OHC | H | PheLeuACHPAA | $0.5H_2O$ |
| E14/E15 | $HOCH_2$ | H | PheLeuACHPAA | |
| E16 | $MeO_2C$ | H | PheLeuACHPAA | $0.5H_2O$ |
| E17a | $HO_2C$ | H | PheLeuACHPAA | |
| E17b | $NaO_2C$ | H | PheLeuACHPAA | |
| E19 | NC | H | PheLeuACHPAA | $0.5H_2O$ |
| E20a | $H_2NCH_2$ | H | PheLeuACHPAA | $0.5CHCl_3$ |
| E20b | $H_2NCH_2$ | H | PheLeuACHPAA | $2H_2O.HCl$ |
| E21 | $PhCH_2O_2C$ | H | PheLeuACHPAA | |
| E22 | $MeO_2S$ | H | PheLeuACHPAA | $0.5H_2O$ |
| E23 | $PhCH_2O_2C$ | H | PheHisACHPAA | $0.5H_2O$ |

Table II (Cont'd.)

| Compound No. | $R_a^1$ | $R_b^1$ | $Q_2$ | Salt/ Solvate (prepared from+) |
|---|---|---|---|---|
| E24a | $HO_2C$ | H | PheHisACHPAA | |
| E24b | $HO_2C$ | H | PheHisACHPAA | $3H_2O.HCl$ |
| E26 | $HOCH_2CH_2N{=}CH$ | H | PheLeuACHPAA | $1.5H_2O$ |
| E27a | $HOCH_2CH_2NHCH_2$ | H | PheLeuACHPAA | |
| E27b | $HOCH_2CH_2NHCH_2$ | H | PheLeuACHPAA | HCl |
| E34 | $EtO_2CCH_2NHCH_2$ | H | PheLeuACHPAA | $0.5H_2O$ |
| E35 | $PhCH_2O_2CCH_2NHCH_2$ | H | PheLeuACHPAA | $H_2O$ |
| E36a | $HO_2CCH_2NHCH_2$ | H | PheLeuACHPAA | |
| E36b | $NaO_2CCH_2NHCH_2$ | H | PheLeuACHPAA | |
| E39 | $NO_2$ | H | PheHisACHPAA | $H_2O$ |
| E40 | $NH_2$ | H | PheHisACHPAA | $0.5CHCl_3$ |
| E42 | -S | H | PheLeuACHPAA | disulphide |
| E43 | $^tBuO_2CCH_2O_2S$ | H | PheLeuACHPAA | $0.5H_2O$ |
| E54 | NC | H | PheHisACHPAA | $H_2O$ |
| E56 | $NaO_2CCH_2O_2S$ | H | PheLeuACHPAA | |
| E57 | $CBZ\text{-}NHCH_2CH_2O_2S$ | H | PheLeuACHPAA | $0.5H_2O$ |
| E66 | OHC | H | PheHisACHPAA | $1.5H_2O$ |

Table III

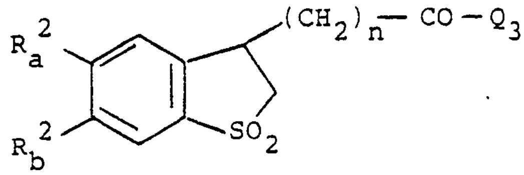

| Compound No. | $R_a^2$ | $R_b^2$ | n | $Q_3$ | Salt/ Solvate (prepared from+) |
|---|---|---|---|---|---|
| D10(b) | H | H | 1 | OH | |
| D10(c) | H | H | 1 | PheLeuOMe | |
| D10(d) | H | H | 1 | PheLeuOH | |
| D21 | H | $NO_2$ | 1 | OH | (D10b) |
| D22 | H | $NO_2$ | 1 | OEt | (D21) |
| D23 | H | $NH_2$ | 1 | OEt | (D22) |
| D24 | H | Br | 1 | OEt | (D23) |
| D25 | H | NC | 1 | OEt | (D24) |
| D26 | H | NC | 1 | OH | (D25) |
| D40(c) | H | H | 2 | OH | |
| D41(e) | NC | H | 1 | OEt | |
| D41(f) | NC | H | 1 | OH | |
| D49(a) | H | BOC-$NHCH_2$ | 1 | OEt | (D25) |
| D49(b) | H | BOC-$NHCH_2$ | 1 | OH | |
| D53(a) | H | H | 1 | OEt | |
| D53(b) | H | H | 1 | H | |
| D53(d) | H | H | 3 | OEt | |
| D53(e) | H | H | 3 | OH | |

Table III Contd.

| Compound No. | $R_a^2$ | $R_b^2$ | n | $Q_3$ | Salt/ Solvate (prepared from$^+$) |
|---|---|---|---|---|---|
| E12 | H | H | 1 | PheLeuACHPAA | |
| E18 | H | H | 1 | PheLeuACHPA-NH$^i$Am | $0.5H_2O$ |
| E25 | H | H | 1 | PheHisACHPAA | $1.5H_2O$ |
| E28 | H | $NO_2$ | 1 | PheLeuACHPAA | $H_2O$ |
| E29 | H | $NH_2$ | 1 | PheLeuACHPAA | $2H_2O$ |
| E30 | H | NC | 1 | PheLeuACHPAA | |
| E31a | H | $H_2NCH_2$ | 1 | PheLeuACHPAA | $2H_2O$ |
| E31b | H | $H_2NCH_2$ | 1 | PheLeuACHPAA | HCl |
| E44 | H | H | 2 | PheLeuACHPAA | $0.5H_2O$ |
| E45 | NC | H | 1 | PheLeuACHPAA | |
| E50a | $H_2NCH_2$ | H | 1 | PheLeuACHPAA | |
| E50b | $H_2NCH_2$ | H | 1 | PheLeuACHPAA | $3H_2O.HCl$ |
| E55 | H | BOC-$NHCH_2$ | 1 | PheHisACHPAA | $1.5H_2O$ |
| E60 | H | $H_2NCH_2$ | 1 | PheHisACHPAA | $1.5H_2O.$ $2CF_3CO_2H$ |
| E61 | H | H | 3 | PheLeuACHPAA | |

Table IV

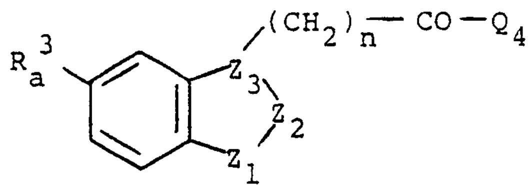

| Compound No. | $R_a^3$ | $Z_1$ | $Z_2$ | $Z_3$ | n | $Q_4$ | Salt/ Solvate |
|---|---|---|---|---|---|---|---|
| D37(b) | H | CO | NH | CH | 1 | OEt | |
| D37(c) | H | CO | NH | CH | 1 | OH | |
| D51(a) | H | NH | CO | N | 1 | OEt | |
| D51(b) | H | NH | CO | N | 1 | OH | |
| D52(a) | H | O | CO | N | 1 | OEt | |
| D52(b) | H | O | CO | N | 1 | OH | |
| D54(a) | H | O | CO | N | 2 | OMe | |
| D54(b) | H | O | CO | N | 2 | OH | |
| D55 | NC | NH | CO | CH | 1 | OH | |
| D60(b) | H | CO | O | CH | 1 | OH | |
| E33 | H | NH | CO | CH | 1 | PheLeuACHPAA | $H_2O$ |
| E41 | H | CO | NH | CH | 1 | PheLeuACHPAA | $H_2O$ |
| E58 | H | NH | CO | N | 1 | PheLeuACHPAA | $1.5H_2O$ |
| E59 | H | O | CO | N | 1 | PheLeuACHPAA | $0.5H_2O$ |
| E62 | H | O | CO | N | 2 | PheLeuACHPAA | $H_2O$ |
| E63 | NC | NH | CO | CH | 1 | PheLeuACHPAA | $H_2O$ |
| E67 | $H_2NCH_2$ | NH | CO | CH | 1 | PheLeuACHPAA | $2.5H_2O$. HCl |
| E68 | H | CO | O | CH | 1 | PheLeuACHPAA | $H_2O$ |

Table V

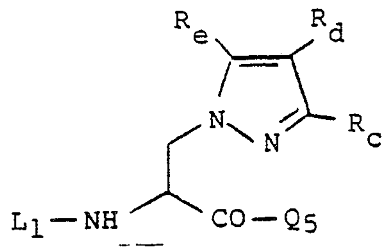

| Compound No. | $L_1$ | $R_c$ | $R_d$ | $R_e$ | $Q_5$ | Salt/ Solvate |
|---|---|---|---|---|---|---|
| D29 | BOC | H | H | H | ACHPAA | |
| D30 | H | H | H | H | ACHPAA | $2CF_3CO_2H$ |
| D31 | CBZ-Phe | H | H | H | ACHPAA | $0.5H_2O$ |
| D32 | Phe | H | H | H | ACHPAA | 2HBr |
| D33 | CBZ | H | Me | H | OH | |
| D34 | CBZ | H | Me | H | ACHPAA | |
| D35 | H | H | Me | H | ACHPAA | 2HBr |
| D43(a) | CBZ | Me | H | Me | OH | |
| D43(b) | CBZ | Me | H | Me | ACHPAA | |
| D43(c) | H | Me | H | Me | ACHPAA | 2HBr |
| D56(e) | 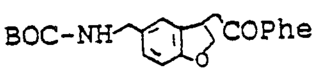 | H | H | H | ACHPAA | $H_2O$ |
| D58(b) | CBZ | H | NH-BOC | H | OH | |
| D58(c) | CBZ | H | NH-BOC | H | ACHPAA | |
| D58(d) | H | H | NH-BOC | H | ACHPAA | |
| D58(e) | 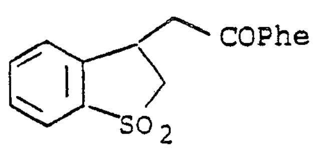 | H | NH-BOC | H | ACHPAA | |
| E37 | 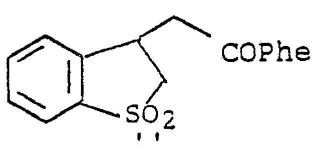 | H | H | H | ACHPAA | $0.5H_2O$ |
| E38 | '' | H | Me | H | ACHPAA | $H_2O$ |
| E47 | '' | Me | H | Me | ACHPAA | $2H_2O$ |
| E64 | '' | H | $NH_2$ | H | ACHPAA | |
| E65 | 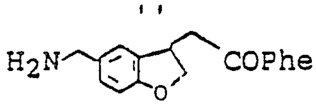 | H | H | H | ACHPAA | $1.5H_2O.$ $CF_3CO_2H$ |

Table VI

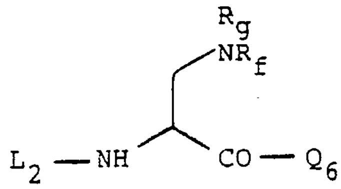

| Compound No. | $L_2$ | $R_f$ | $R_g$ | $Q_6$ | Salt/ Solvate (prepared from +) |
|---|---|---|---|---|---|
| D44(a) | CBZ | BOC | H | OH | |
| D44(b) | CBZ | BOC | H | ACHPAA | |
| D44(c) | H | BOC | H | ACHPAA | |
| D46(a) | CBZ | BOC | H | OMe | (D44a) |
| D46(b) | CBZ | H | H | OMe | $CF_3CO_2H$ |
| D46(c) | CBZ | Ac | H | OMe | |
| D46(d) | CBZ | Ac | H | OH | |
| D46(e) | CBZ | Ac | H | ACHPAA | |
| D47(a) | CBZ | $CO(CH_2)_3Cl$ | H | OMe | (D46b) |
| D47(b) | CBZ | $-COCH_2CH_2CH_2-$ | | OMe | |
| D47(c) | CBZ | $-COCH_2CH_2CH_2-$ | | OH | |
| D47(d) | CBZ | $-COCH_2CH_2CH_2-$ | | ACHPAA | |
| D47(e) | H | $-COCH_2CH_2CH_2-$ | | ACHPAA | |
| E48 | 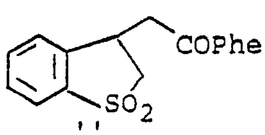 | BOC | H | ACHPAA | $1.5H_2O$ |
| E49 | '' | H | H | ACHPAA | $H_2O.CF_3CO_2H$ |
| E52 | '' | Ac | H | ACHPAA | |
| E53 | '' | $-COCH_2CH_2CH_2-$ | | ACHPAA | |

Remaining Structures

D10(a)

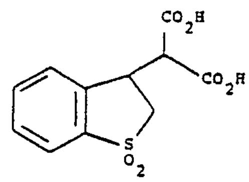

D13(a) BOC-ACHPAA D13(b) CBZ-PheLeuACHPAA

D16(a)

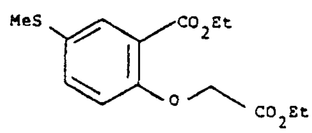

D16(b)

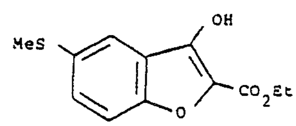

D16(c)

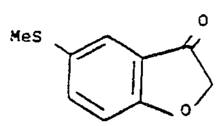

D16(d)

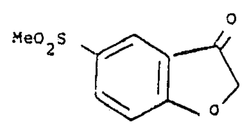

D16(e)

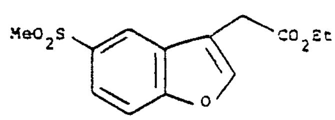

D17 CBZ-PheHisACHPAA
D18 PheHisACHPAA
D19 BOC-PheLeuACHPAA
D20 PheLeuACHPAA. $CF_3CO_2H$

D27

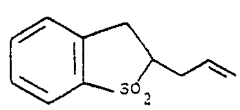

D37

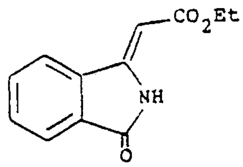

D38(c)

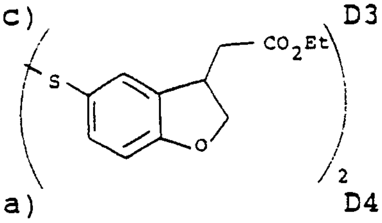

D38(d)

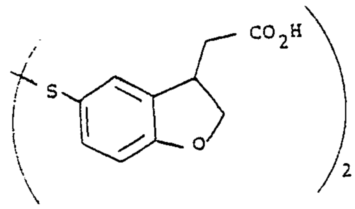

D40(a)

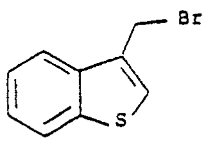

D40(b)

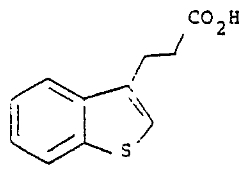

D41(a)

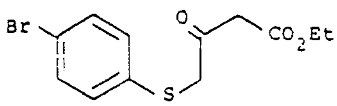

D41(b)

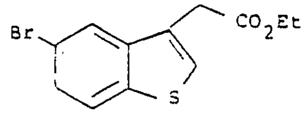

D41(c)

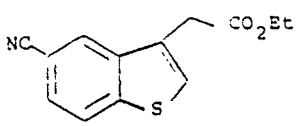

D41(d)

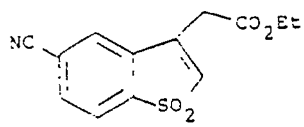

D42

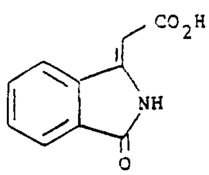

D45(a)

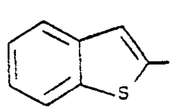

D45(b)

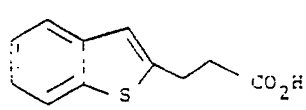

D45(c)

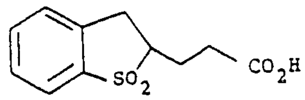

D53(c)

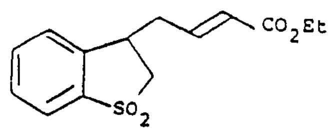

D58(a)

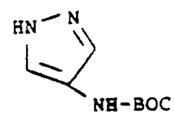

D60(a)

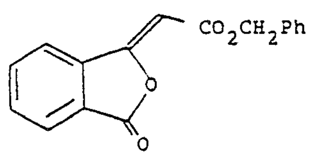

E46

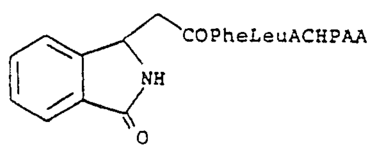

½$H_2O$

## Starting materials and Mass spectroscopic characterisation for Examples

| Compound | Prepared from | M+1(m.w.) |
|---|---|---|
| E1 | D1(d) | 677(676) |
| E2 | D2(d) | 705(704) |
| E3 | D3(c) | 721(720) |
| E4 | D4(c) | 691(690) |
| E5 | D5(d) | 736(735) |
| E6 | E5 | 706(705) |
| E7 | D6(c) | 749(748) |
| E8 | D7(d) | 736(735) |
| E9 | D8(d) | 784(783) |
| E10 | D9(f) | 692($M^+$) |
| E11 | E10 | 724($M^+$) |
| E12 | D10(d) | 739(738) |
| E13 | D11(d) | 719(718) |
| E14 | E13 | 721(720) |
| E15 | E13 | 721(720) |
| E16 | D12(b) | 749(748) |
| E17a/1 | E16 ) | |
| /2 | D59 ) | 735(734) |
| /3 | E21 ) | |
| E17b | E17a | |
| E18 | (D19)* | 753(752) |
| E19 | D14(d) | 716(715) |
| E20a | E19 | 720(719) |
| E20b | E20a | - |
| E21 | D15(b) | 825(824) |
| E22 | D16(a) | 769(768) |
| E23 | E15(b)/D18 | 849(848) |
| E24a | E23 | |
| E24b | E24a | 759(758) |
| E25 | D10(b)/D18 | 763(762) |
| E26 | E13 | 762(761) |

* Prepared analogously to

Starting materials and Mass spectroscopic characterisation for Examples

| Compound | Prepared from | M+1(m.w.) |
|---|---|---|
| E27a | E26 | 764(763) |
| E27b | E27a | 764(763) |
| E28 | D21 | 784(783) |
| E29 | E28 | 754(753) |
| E30 | D26 | 764(763) |
| E31a | E30 | 768(767) |
| E31b | E31a | - |
| E32 | D28 | 739(738) |
| E33 | D20 | 704(703) |
| E34 | E13 | 806(805) |
| E35 | E13 | 869(868) |
| E36a | E34 | 778(777) |
| E36b | E36a | - |
| E37 | D10(b)/D32 | 763(762) |
| E38 | (D2)*/D35 | 777(776) |
| E39 | D36(c) | 760(759) |
| E40 | E39 | 730(729) |
| E41 | D37(c) | 704(703) |
| E42 | D38(d) | 1443(1442) |
| E43 | D39(e) | 869(868) |
| E44 | D40(c) | 753(752) |
| E45 | D41(f) | 764(763) |
| E46 | D42 | 702(701) |
| E47 | (D2)*/D43(c) | 791(790) |
| E48 | (D2)*/D44(c) | 812(811) |
| E49 | E48 | 712(711) |
| E50a | E45 | - |
| E50b | E50a | 769(768) |
| E51 | D20/D40(c) | 753(752) |
| E52 | D46(e) | 754(753) |
| E53 | D47(d) | 780(779) |
| E54 | D48(b) | 740(739) |

* Prepared analogously to

## Starting materials and Mass spectroscopic characterisation for Examples

| Compound | Prepared from | M+1(m.w.) |
|---|---|---|
| E55 | D49(b) | 892(891) |
| E56 | E43 | 813(812) |
| E57 | D50(e) | 932(931) |
| E58 | D51(b) | 705(704) |
| E59 | D52(b) | 706(705) |
| E60 | E55 | 792(791) |
| E61 | D53(e) | 767(766) |
| E62 | D54(b) | 720(719) |
| E63 | D55 | 729(728) |
| E64 | D58(e) | 778(777) |
| E65 | D56(e) | 744(743) |
| E66 | D57(b) | 743(742) |
| E67 | E63 | 733(732) |
| E68 | D60(b) | 705(704) |

### Description 1

a) (2,3-Dihydrobenzofuran-2-carbonyl)-(S)-phenylalanine methyl ester

To a solution of 2,3-dihydrobenzofuran-2-carboxylic acid * (3.62 g) in dry tetrahydrofuran (THF) (100 ml) was added phenylalanine methyl ester hydrochloride (4.76 g) and hydroxybenzotriazole (2.98 g). The mixture was stirred at 0° and dicyclohexylcarbodiimide (4.55 g) was added in one portion. After 1 h at 0°, the mixture was warmed to room temperature for 1 h and filtered. The solvent was removed under reduced pressure and the residue partitioned between ethyl acetate and saturated sodium bicarbonate. The organic layer was washed with 10% citric acid, saturated sodium bicarbonate, water and brine. The solution was dried ($MgSO_4$) and the solvent was removed under reduced pressure to leave a yellow solid that was chromatographed on silica gel using 5% methanol/chloroform. This gave (2,3-dihydrobenzofuran-2-carbonyl)-(S)-phenylalanine methyl ester (4.31g).
NMR (δ) ($CDCl_3$) 2.6-3.8(7H,m), 4.7-5.3(2H,m), 6.5-7.7(10H,m).

b) (2,3-Dihydrobenzofuran-2-carbonyl)-(S)-phenylalanine

To a solution of (2,3-dihydrobenzofuran-2-carbonyl)-(S)-phenylalanine methyl ester (3.83 g) in a mixture of dioxan (60 ml) and water (30 ml) at 0° was added lithium hydroxide (1.73 g). The mixture was kept at 0° for 10 min and then stirred at room temperature overnight. Water was added and the solution was acidified to pH2.5 (with 10% potassium hydrogen sulphate). The oily suspension was extracted with ethyl acetate (x3) and the organic layers were dried ver magnesium sulphate. Removal of the solvent under reduced

* See Org. Syn. Coll. Vol. III, 209 and J. Med. Chem. 1963, 6, 315-9.

pressure gave (2,3-dihydrobenzofuran-2-carbonyl)-(S)-phenylalanine (3.4 g).
NMR (δ) ($CDCl_3$) 2.6-3.5(4H,m), 4.5-5.2(2H,m), 6.6-7.4(10H,m), 11.0(1H,bs).

c) (2,3-Dihydrobenzofuran-2-carbonyl)-(S)-phenylalanyl-(S)-leucine methyl ester

This material was formed from (2,3-dihydrobenzofuran-2-carbonyl)-(S)-phenylalanine, (3.92 g) and (S)-leucine methyl ester hydrochloride (2.3 g) using the method in Description 1(a). This gave (2,3-dihydrobenzofuran-2-carbonyl)-(S)-phenylalanyl-(S)-leucine methyl ester (2.69 g).
NMR (δ) ($CDCl_3$) 2.7-5.3(10H,m), 6.5-7.8(11H,m).

d) (2,3-Dihydrobenzofuran-2-carbonyl)-(S)-phenylalanyl-(S)-leucine

This material was formed from (2,3-dihydrobenzofuran-2-carbonyl)-(S)-phenylalanine-(S)-leucine methyl ester (2.69 g) using the method of Description 1(b). This gave (2,3-dihydrobenzofuran-2-carbonyl)-(S)-phenylalanyl-(S)-leucine (0.59 g).
NMR (δ) ($DMSOd_6$) 0.7-1.0(6H,m), 1.4-1.7(3H,m), 2.7-3.4-4H,m), 4.2(1H,m), 4.6(1H,m), 5.1(1H,m), 6.7-7.4-(9H,m), 7.9(1H,m), 8.4(1H,m), 12.6(1H,bs).
H.R.M.S. m/z $C_{24}H_{28}N_2O_5$ requires 424.1998.
Found 424.1997.

Description 2

a) 3-(2,3-Dihydrobenzofuran-2-yl)propanoyl-(S)-phenylalanine methyl ester

To a solution of 3-(2,3-dihydrobenzofuran-2-yl) propanoic acid * (5.0 g), (S)-phenylalanine methyl ester hydrochloride (5.62 g), hydroxybenzotriazole (3.52 g) and N-ethyl morpholine (3.3 ml) in dry dichloromethane (85 ml) at 0° was added dicyclohexylcarbodiimide (5.62 g). After 1 h at 0°, the mixture was stirred overnight at room temperature. The mixture was filtered and washed with 10% citric acid (2x80 ml), water (50 ml), saturated sodium bicarbonate (2x70 ml) and dried over sodium sulphate. Removal of the solvent under reduced pressure gave 3-(2,3-dihydrobenzofuran-2-yl)propanoyl-(S)-phenylalanine methyl ester (6.9 g).
NMR (δ) ($DMSOd_6$) 1.5-2.5(4H,m), 2.7-3.2(4H,m), 3.7(3H,s), 4.3-4.7(2H,m), 6.5-7.2(9H,m), 8.3(1H,bd).

b) 3-(2,3-Dihydrobenzofuran-2-yl)propanoyl-(S)-phenylalanine

To a solution of 3-(2,3-dihydrobenzofuran-2-yl)propanoyl-(S)-phenylalanine methyl ester (6.9 g) in methanol (70 ml) at 0° was added a solution of sodium hydroxide (1.0 g) in water (10 ml). The mixture was allowed to warm up to room temperature and stirred overnight. The methanol was removed under reduced pressure and the residue was washed with ethyl acetate, acidified with 10% citric acid and the oily solid was extracted with dichloromethane (3x80 ml). The combined dichloromethane extracts were washed with brine and dried over sodium sulphate. Removal of the solvent under reduced pressure gave 3-(2,3-dihydrobenzofuran-2-yl)propanoyl -(S)-phenylalanine (5.8 g).
NMR (δ) ($CDCl_3/CD_3OD$) 1.5-2.1(2H,m), 2.1-2.5(2H,m), 2.7-3.2(4H,m), 4.2-4.8(2H,m), 6.5-7.1(9H,m).

c) 3-(2,3-Dihydrobenzofuran-2-yl)propanoyl-(S)-phenylalanyl-(S)-leucine methyl ester

This material was formed from 3-(2,3-dihydrobenzofuran-2-yl)propanoyl-(S)-phenylalanine (5.3 g) and (S)-leucine methyl ester hydrochloride (2.85 g) as for Description 2(a) except that 1-(3-dimethylaminopropyl)-3-ethyl carbodiimide hydrochloride was substituted for dicyclohexylcarbodiimide. This gave 3-(2,3-dihydrobenzofuran-2-yl)propanoyl-(S)-phenylalanyl-(S)-leucine methyl ester (6.8 g).

* U.S. Patent 4,428,940.

NMR (δ) ($CDCl_3$) 0.8-1.0(6H,2xd), 1.3-1.6(3H,m), 1.6-2.6(4H,m), 2.8-3.3(4H,m), 3.6(3H,s), 4.3-5.0(3H,m), 6.3-7.2(11H,m).

d) 3-(2,3-Dihydrobenzofuran-2-yl)propanoyl-(S)-phenylalanyl-(S)-leucine

This material was formed from 3-(2,3-dihydrobenzofuran-2-yl)propanoyl-(S)-phenylalanyl-(S)-leucine methyl ester (6.7 g) as in Description 2(b). This gave 3-(2,3-dihydrobenzofuran-2-yl)propanoyl-(S)-phenylalanyl-(S)-leucine (5.4 g).
NMR (δ) ($DMSOd_6$) 0.7-0.9(6H,2xd), 1.4-1.8(5H,m), 2.1-2.3(2H,m), 2.6-2.8(2H,m), 2.9-3.2(2H,m), 4.2(1H,m), 4.4-4.7(2H,m), 6.6-6.8(2H,m), 7.0-7.3(7H,m), 8.1(1H,m), 8.2(1H,m), 12.4(1H,bs).
M.S. (m/z) (FAB) (M+1) = 453 (consistent with m.w. = 452).

Description 3

a) (7-Methoxy-2,3-dihydrobenzofuran-3-yl)acetyl-(S)-phenylalanine methyl ester

This material was formed from (7-methoxy-2,3-dihydrobenzofuran-3-yl)acetic acid * (10 g) and (S)-phenylalanine methyl ester hydrochloride (10.4 g) as in Description 2(a). This gave (7-methoxy-2,3-dihydrobenzofuran-3- yl)acetyl-(S)-phenylalanine methyl ester (16.9 g).
NMR (δ) ($DMSOd_6$) 3.5(3H,s), 3.7(3H,s), 6.5-6.8(3H,m), 7.2(5H,s), 8.4(1H,d).

b) (7-Methoxy-2,3-dihydrobenzofuran-3-yl)acetyl-(S)-phenylalanyl-(S)-leucine methyl ester

This material was formed from (7-methoxy-2,3-dihydrobenzofuran-3-yl)acetyl-(S)-phenylalanine methyl ester (16.9 g) following the method of Description 2(a) and (b). This gave (7-methoxy-2,3-dihydrobenzofuran-3-yl)acetyl-(S)-phenylalanyl-(S)-leucine methyl ester (21.7 g).
NMR (δ) ($CDCl_3$) 0.7-1.0(6H,m), 1.4-2.0(3H,m), 3.6(3H,s), 3.7(3H,s), 6.2-6.7(5H,m), 7.1(5H,s).

c) (7-Methoxy-2,3-dihydrobenzofuran-3-yl)acetyl-(S)-phenylalanyl-(S)-leucine

This material was prepared from (7-methoxy-2,3-dihydrobenzofuran-3-yl)acetyl-(S)-phenylalanyl-(S)-leucine methyl ester (22.7 g) as in Description 2(b). This gave (7-methoxy-2,3-dihydrobenzofuran-3-yl)-acetyl-(S)-phenylalanyl-(S)-leucine (17.2 g).
NMR (δ) ($DMSOd_6$) 0.7-0.9(6H,2xd), 1.4-1.8(3H,m), 3.7(3H,s), 6.5-6.8(3H,m), 7.1-7.3(5H,m), 8.1-8.4(2H,m), 12.6(1H,bs).
M.S. (m/z) (FAB) (M+1) = 469 (consistent with m.w. = 468).

Description 4

a) (2,3-Dihydrobenzofuran-3-yl)acetyl-(S)-phenylalanine methyl ester

This material was formed from (2,3-dihydrobenzofuran-3-yl)acetic acid (5.25 g) and (S)-phenylalanine methyl ester hydrochloride as in Description 2(a). This gave (2,3-dihydrobenzofuran-3-yl)acetyl-(S)-phenylalanine methyl ester (8.0 g).
NMR (δ) ($CDCl_3$) 2.5(2H,m), 3.1(2H,m), 3.7(3H,s), 6.6-7.3(9H,m).

b) (2,3-Dihydrobenzofuran-3-yl)acetyl-(S)-phenylalanine

* See J.O.C. 1962, 27, 586-91 and Helv. Chim. Acta. 1982, V65, 1837-52.

This material was formed from (2,3-dihydrobenzofuran-3-yl)acetyl-(S)-phenylalanine methyl ester (8.0 g) as in Description 2(b). This gave (2,3-dihydrobenzofuran-3-yl)acetyl-(S)-phenylalanine (5.9 g).
NMR (δ) ($CDCl_3/DMSOd_6$) 2.5(2H,m), 3.2(2H,m), 6.6-7.3(9H,m).

c) (2,3-Dihydrobenzofuran-3-yl)acetyl-(S)-phenylalanyl-(S)-leucine

This material was formed from (2,3-dihydrobenzofuran-3-yl)acetyl-(S)-phenylalanine (5.9 g) following the method of Descriptions 2(a) and 2(b). This gave (2,3-dihydrobenzofuran-3-yl)acetyl-(S)-phenylalanyl-(S)-leucine (2.9 g).
NMR (δ) ($CDCl_3$) 0.7-0.9(6H,m), 1.4-1.9(3H,m), 6.7-7.1(4H,m), 7.1-7.3(5H,m), 8.2-8.4(2H,m), 12.6(1H,b).
M.S. (m/z) (FAB) (M + 1) = 439 (consistent with m.w. = 438).

## Description 5

a) Ethyl (5-nitro-2,3-dihydrobenzofuran-3-yl)acetate

To a stirred, ice cooled solution of ethyl (2,3-dihydrobenzofuran-3-yl)acetate (10 g, 0.048 moles) in glacial acetic acid (50 ml) was added concentrated nitric acid (2.4 ml) dropwise. After stirring for 1 h at room temperature further concentrated nitric acid (7.6 ml) was added and stirring was continued for a further 18 h. The reaction mixture was poured into water (200 ml), basified with solid sodium carbonate and extracted with chloroform (3x100 ml). The chloroform extracts were combined, dried over anhydrous sodium sulphate and evaporated to dryness in vacuo. Purification by column chromatography on silica gel using dichloromethane as eluent afforded ethyl (5-nitro-2,3-dihydrobenzofuran-3-yl)acetate (6.3 g).
NMR (δ) ($CDCl_3$) 1.3(3H,t), 2.6-2.9(2H,m), 4.0(1H,m), 4.2(2H,g), 4.5(1H,dd), 5.0(1H,t), 6.9(1H,d), 8.1(2H,m).

b) (5-Nitro-2,3-dihydrobenzofuran-3-yl)acetic acid

Ethyl (5-nitro-2,3-dihydrobenzofuran-3-yl)acetate (6.85 g, 0.0273 moles) in ethanol (100 ml) was cooled in ice as a 10% aqueous sodium hydroxide solution (12 ml, 0.03 moles) was added slowly, with stirring. The ice was removed and stirring was continued for 19 h. The reaction mixture was evaporated in vacuo and the residue taken up in water and washed with chloroform. Acidification of the aqueous layer afforded (5-nitro-2,3-dihydrobenzofuran-3-yl)acetic acid as a yellow solid which was isolated by filtration (4.81 g, 79%).
NMR (δ) ($CDCl_3/CD_3OD/D_2O$) 2.5-2.9(2H,m), 3.5-4.2(1H,m), 4.3(2H,dd), 4.9(1H,t), 6.7(1H,d), 8.0(2H,m).

c) (5-Nitro-2,3-dihydrobenzofuran-3-yl)acetyl-(S)-phenylalanyl-(S)-leucine methyl ester

To a stirred, ice cooled mixture of (5-nitro-2,3-dihydrobenzofuran-3-yl)acetic acid (2.23 g, 0.01 moles) and 1-hydroxybenzotriazole (1.35 g, 0.01 moles) in dry tetrahydrofuran (80 ml) was added dicyclohexylcarbodiimide (2.06 g, 0.01 moles) in one portion. After 10 mins N-ethylmorpholine (1.15 g, 0.01 moles) was added, immediately followed by (S)-phenylalanyl-(S)-leucine methyl ester hydrochloride 3.52 g, 0.01 moles). The reaction mixture was stirred at room temperature for 18 h. The precipitate was filtered and the filtrate evaporated in vacuo. The residue was dissolved in ethyl acetate, filtered and washed with saturated sodium hydrogen carbonate (200 ml), 10% citric acid solution (100 ml) and saturated brine (200 ml). The organics were dried over sodium sulphate and evaporated to dryness in vacuo to yield (5-nitro-2,3-dihydrobenzofuran-3-yl)acetyl-(S)-phenylalanyl-(S)-leucine methyl ester (3.95 g, 79%).
NMR (δ) ($CDCl_3$) 0.7-1.7(9H,m), 2.4-3.2(4H,m), 3.4-5.1(8H,m), 6.4-8.3(10H,m).

d) (5-Nitro-2,3-dihydrobenzofuran-3-yl)acetyl-(S)-phenylalanyl-(S)-leucine

(5-Nitro-2,3-dihydrobenzofuran-3-yl)acetyl-(S)-phenylalanyl-(S)-leucine methyl ester (1.1 g, 2.21 mmoles) in methanol (20 ml) was cooled in ice, and a 10% aqueous sodium hydroxide solution (0.97 ml, 2.42 mmoles) was added slowly with stirring. The ice was removed and stirring was continued for 19 h. The

reaction mixture was evaporated in vacuo and the residue taken up in water and washed with ethyl acetate. The aqueous layer was acidified with 5M HCl and extracted with ethyl acetate (2x50 ml). Combined extracts were dried over anhydrous sodium sulphate and evaporated in vacuo to afford (5-nitro-2,3-dihydroben zofuran-3-yl)acetyl-(S)-phenylalanyl-(S)-leucine as a yellow solid (0.55 g, 51%).
NMR (δ) ($CD_3OD$) 1.0-1.9(9H,m), 2.4-3.4(4H,m), 3.8-4.9(4H,m), 6.8-8.3(10H,m).
M.S. (m/z) (FAB) (M + 1) = 484 (consistent with m.w. = 483).

## Description 6

### a) (5-Amino-2,3-dihydrobenzofuran-3-yl)acetyl-(S)-phenylalanyl-(S)-leucine methyl ester acetate salt

A solution of (5-nitro-2,3-dihydrobenzofuran-3-yl)acetyl-(S)-phenylalanyl-(S)-leucine methyl ester (1.51 g, 3 mmoles) in ethanol (50 ml) and glacial acetic acid (0.52 ml, 9 mmoles) was hydrogenated at room temperature and atmospheric pressure in the presence of 10% palladium on carbon for 18 h. Filtration through kieselguhr and evaporation to dryness in vacuo afforded (5-amino-2,3-dihydrobenzofuran-3-yl)-acetyl-(S)-phenylalanyl-(S)-leucine methyl ester acetate salt (1.55 g).
H.R.M.S. $C_{26}H_{33}N_3O_5$ requires 467.2420. Found, 467.2420.

### b) (5-Ureido-2,3-dihydrobenzofuran-3-yl)acetyl-(S)-phenylalanyl-(S)-leucine methyl ester

Potassium cyanate (0.27 g, 3.3 mmoles) in water (2 ml) was added dropwise to a stirred solution of (5-amino-2,3-dihydrobenzofuran-3-yl)acetyl-(S)-phenylalanyl-(S)-leucine methyl ester acetate salt (1.55 g, 2.9 mmol) in methanol (10 ml). After 1 h the reaction mixture was filtered to afford (5-ureido-2,3-dihydrobenzofuran-3-yl)acetyl-(S)-phenylalanyl-(S)-leucine methyl ester as a red/brown solid (1.20 g, 80%).
NMR (δ) ($CD_3OD$) 0.8-1.8(9H,m), 2.2-3.2(4H,m), 3.5-4.7(8H,m), 6.6-7.4(8H,m).
M.S. (m/z) (FAB) (M + 1) = 511 (consistent with m.w. = 510).

### c) (5-Ureido-2,3-dihydrobenzofuran-3-yl)acetyl-(S)-phenylalanyl-(S)-leucine

This material was prepared as described in Description 5(d) from (5-ureido-2,3-dihydrobenzofuran-3-yl)-acetyl-(S)-phenylalanyl-(S)-leucine methyl ester (1.15 g, 2.25 mmoles). This gave (5-ureido-2,3-dihydrobenzofuran-3-yl)acetyl-(S)-phenylalanyl-(S)-leucine (0.8 g).
NMR (δ) ($CDCl_3$) 0.7-1.0(6H,dd), 1.3-1.7(3H,m), 2.0-2.8(4H,m), 3.1(1H,m), 3.6-3.8(2H,m), 4.2(2H,m), 5.4-6.2-(1H,bs), 6.6(1H,dd), 7.0(1H,dd), 7.1-8.7(9H,m), 12.0-13.0(1H,bs).
M.S. (m/z) (FAB) (M + 1) = 497 (consistent with m.w. = 496).

## Description 7

### a) Ethyl (7-nitro-2,3-dihydrobenzofuran-3-yl)acetate

This material was formed as a by-product in the synthesis of ethyl (5-nitro-2,3-dihydrobenzofuran-3-yl)-acetate (Description 5(a)) and isolated by chromatography as a yellow solid. This gave ethyl (7-nitro-2,3-dihydrobenzofuran-3-yl)acetate (0.74 g).
NMR (δ) ($CDCl_3$) 1.3(3H,t ), 2.6-2.9(2H,m), 3.9 (1H,m), 4.2(2H,q), 4.6(1H,m), 5.0(1H,t), 7.0(1H,m), 7.4(1H,dd), 8.0(1H,dd).

### b) (7-Nitro-2,3-dihydrobenzofuran-3-yl)acetic acid

This material was prepared as described in Description 5(b) from ethyl (7-nitro-2,3-dihydrobenzofuran-3-yl)acetate (0.66 q, 2.63 mmoles). This gave (7-nitro-2,3-dihydrobenzofuran-3-yl)acetic acid (0.54 g).
NMR (δ) ($DMSOd_6$) 4.0(1H,m), 4.7(1H,m), 5.1(1H,t), 7.0(1H,m), 7.6(1H,dd), 8.0(1H,dd).

H.R.M.S. (m/z) $C_{10}H_9NO_5$ requires 223.0481. Found 223.0480.

c) (7-Nitro-2,3-dihydrobenzofuran-3-yl)acetyl-(S)-phenylalanyl-(S)-leucine methyl ester

This material was prepared as described in Description 5(c) from (7-nitro-2,3-dihydrobenzofuran-3-yl)-acetic acid (0.40 g, 1.79 mmoles). This gave (7-nitro-2,3-dihydro- benzofuran-3-yl)acetyl-(S)-phenylalanyl-(S)-leucine methyl ester (0.65 g).
NMR (δ) ($CDCl_3$) 0.7-2.0(9H,m), 2.4-2.7(2H,m), 3.1(2H,d), 3.7(3H,s), 4.0-5.0(5H,m), 6.3-8.0(10H,m).
M.S. (m/z) (FAB) (M + 1) = 498 (consistent with m.w. = 497).

d) (7-Nitro-2,3-dihydrobenzofuran-3-yl)acetyl-(S)-phenylalanyl-(S)-leucine

Prepared as described in Description 5(d) from (7-nitro-2,3-dihydrobenzofuran-3-yl)acetyl-(S)-phenylalanyl-(S)-leucine methyl ester (0.64 g, 1.2 mmoles). This gave (7-nitro-2,3-dihydrobenzofuran-3-yl)-acetyl-(S)-phenylalanyl-(S)-leucine (0.48 g).
NMR (δ) ($DMSOd_6$) 0.5-1.7(9H,m), 2.0-4.7(9H,m), 6.3-8.2(10H,m).

Description 8

a) Ethyl (5-(methylaminosulphonyl)-2,3-dihydrobenzofuran-3-yl)acetate

Ethyl (2,3-dihydrobenzofuran-3-yl)acetate (1.0 g) was added portionwise with stirring to chlorosulphonic acid 1.65 ml) at 0°C. The mixture was stirred at room temperature for 6 h, poured into water, and extracted with chloroform (x3). The extract was dried ($Na_2SO_4$) and evaporated under reduced pressure to leave a yellow oil (0.48 g), the crude ethyl (5-chlorosulphonyl-2,3-dihydrobenzofuran-3-yl)acetate intermediate.

This material was dissolved in ethanol (4 ml), and methylamine (2 ml of 33% methylamine in industrial methylated spirit) was added. This mixture was swirled, and left to stand at room temperature overnight. Removal of volatiles at reduced pressure gave a semi-solid that was chromatographed on silica gel using chloroform. This gave ethyl (5-(methylaminosulphonyl)-2,3-dihydrobenzofuran-3-yl)acetate (0.29 g).
NMR (δ) ($CDCl_3$) 1.3(3H,t), 2.2-2.8(5H,m), 3.5-5.3(6H,m), 6.6-7.7(3H,m).

b) (5-(Methylaminosulphonyl)-2,3-dihydrobenzofuran-3-yl)acetic acid

To a solution of ethyl (5-(methylaminosulphonyl)-2,3-dihydrobenzofuran-3-yl)acetate (0.28 g) in ethanol (5 ml) was added sodium hydroxide (0.53 ml of 10% aqueous solution). The mixture was swirled and left to stand at room temperature for 4 h, poured into water (100 ml), and washed with chloroform (x2). The aqueous solution was then acidified with 5 M hydrochloric acid and extracted with chloroform (x3). The combined extracts were dried ($Na_2SO_4$) and evaporated under reduced pressure, giving a colourless oil, (5-(methylaminosulphonyl)-2,3-dihydrobenzofuran-3-yl)acetic acid (0.12 g).
NMR (δ) ($CDCl_3$) 2.66(3H,d), 2.7-2.95(2H, ABX), 3.95(1H, quintet), 4.38(1H,dd), 4.66(1H,m), 4.87(1H,t), 6.89-(1H,d), 7.7(2H,m).

c) (5-(Methylaminosulphonyl)-2,3-dihydrobenzofuran-3-yl)acetyl-(S)-phenylalanyl-(S)-leucine methyl ester

To a solution of (5-(methylaminosulphonyl)-2,3-dihydrobenzofuran-3-yl)acetic acid (0.270 g) and hydroxybenzotriazole (0.136 g) in dry dimethylformamide (4 ml) at 0°C, was added, with stirring, dicyclohexylcarbodiimide (0.207 g). After 15 min at 0°C, diisopropylethylamine (0.21 ml) and (S)-phenylalanyl-(S)-leucine methyl ester acetate salt (0.353 g) were successively added, and the mixture was stirred overnight, rising slowly to room temperature. Ethyl acetate (50 ml) was added, and the mixture was filtered and washed with 5% citric acid (25 ml), water (25 ml) 5% sodium hydrogen carbonate (25 ml) and brine (25 ml), dried ($Na_2SO_4$) and evaporated under reduced pressure. The yellow solid so obtained was chromatographed on silica gel using chloroform. This gave (5-(methylaminosulphonyl)-2,3-dihydrobenzofuran-3-yl)acetyl-(S)-phenylalanyl-(S)-leucine methyl ester (0.28 g) as a semi-solid.
NMR (δ) ($CD_3OD/DMSOd_6$) 0.86(3H,d), 0.90(3H,d), 1.0-1.8(3H,m), 2.6-3.1(4H,m), 3.61(3H,m), 3.8-4.9(5H,m), 5.57(1H,d), 6.9(1H,m), 7.1-7.3(m), 7.5-7.65(2H,m), 8.0-8.55(4H,m). (Some portions of the spectrum were obscured by solvent peaks).

d) (5-(Methylaminosulphonyl)-2,3-dihydrobenzofuran-3-yl)acetyl-(S)-phenylalanyl-(S)-leucine

This material was formed from the corresponding methyl ester (0.27 g) and sodium hydroxide (0.28 ml of 10% aqueous solution) in ethanol (4 ml), using the method of Description 8(b). This gave (5-(methylaminosulphonyl)-2,3-dihydrobenzofuran-3-yl)acetyl-(S)-phenylalanyl-(S)-leucine (0.218 g).
NMR (δ) ($CDCl_3$) 0.8-1.0(6H,m), 1.0-1.8(3H,m), 2.3-4.9(12H,m), 6.8-7.0(1H,m), 7.25(m), 7.5-8.1(6H,m), 12.3-(1H,b).

Description 9

a) Methyl 2,3-dihydrobenzothiophene-2-carboxylate

Ethyl thianaphthene-2-carboxylate (21.74 g) was dissolved in 1000 ml of methanol and stirred at room temperature with magnesium turnings (12.69 g). After 5 h stirring the temperature of the reaction began to rise rapidly but was maintained at 30°C by external cooling. When all the magnesium turnings had reacted the solution was acidified (5 M HCl) and diluted with water (1000 ml). The solution was extracted with ether (3x500 ml) and the organic layers washed with water (3x800 ml) and brine (800 ml) and dried over magnesium sulphate. Removal of the solvent under reduced pressure gave methyl 2,3-dihydrobenzothiophene-2-carboxylate (20.07 g).
NMR (δ) ($CCl_4$) 2.2-3.4(2H,m), 3.5-3.9(3H,m), 4.0-4.7(1H,m), 6.5-7.8(4H,m).

b) 2,3-Dihydrobenzothiophene-2-carboxylic acid

Methyl 2,3-dihydrobenzothiophene-2-carboxylate (20.07 g) and sodium hydroxide (5.15 g in 20 ml of water) were stirred in 180 ml of methanol for 2 h. The solution was diluted with water (1000 ml) and washed with ether (2x300 ml). The aqueous layer was acidified (5 M HCl), extracted with chloroform (3x200 ml) and dried over magnesium sulphate. Removal of the solvent under reduced pressure gave 2,3-dihydrobenzothiophene-2-carboxylic acid (16.57 g).
NMR (6) ($CCl_4$) 2.2-3.4(2H,m), 4.0-4.6(1H,m), 6.5-7.7(4H,m).

c) (2,3-Dihydrobenzothiophene-2-carbonyl)-(S)-phenylalanine methyl ester

To a solution of 2,3-dihydrobenzothiophene-2-carboxylic acid (8.55 g), hydroxybenzotriazole (7.05 g), (S)-phenylalanine methyl ester hydrochloride (11.26 g) and diisopropylethylamine (9.1 ml) in dry dimethylformamide (100 ml) at 0°C, was added, with stirring, dicyclohexylcarbodiimide (10.76 g). The mixture was stirred, and allowed to warm to room temperature overnight. Ethyl acetate (500 ml) was added, and the mixture was filtered and washed with 10% citric acid (250 ml), water (250 ml), 5% sodium hydrogen carbonate (250 ml) and brine (250 ml), dried ($MgSO_4$) and evaporated under reduced pressure. This gave (2,3-dihydrobenzothiophene-2-carbonyl)-(S)-phenylalanine methyl ester (16.56 g).
NMR (δ) ($CDCl_3$) 2.7-3.2(2H,m), 3.3-3.8(4H,m), 3.9-5.0(3H,m), 6.5-7.6(10H,m).

d) (2,3-Dihydrobenzothiophene-2-carbonyl)-(S)-phenylalanine

(2,3-Dihydrobenzothiophene-2-carbonyl)-(S)-phenylalanine methyl ester (16.56 g) and sodium hydroxide (2.43 g in 20 ml of water) were stirred in 180 ml of methanol for 2 h. The solution was diluted with water (1000 ml) washed with chloroform (2x300 ml), acidified (5 M HCl), extracted with chloroform (3x300 ml) and dried ($MgSO_4$). Removal of the solvent under reduced pressure gave (2,3-dihydrobenzothiophene-2-carbonyl)-(S)-phenylalanine (15.43 g).
NMR (δ) ($CDCl_3$) 2.5-3.3(2H,m), 3.3-3.7(1H,m), 4.1-5.2(3H,m), 6.5-7.7(9H,m), 9.2-9.5(1H,m), 9.7-10.3(1H,m).

e) (2,3-Dihydrobenzothiophene-2-carbonyl)-L-phenylalanyl-(S)-leucine methyl ester

This material was formed from (2,3-dihydrobenzothiophene-2-carbonyl)-(S)-phenylalanine (3.62 g), (S)-leucine methyl ester hydrochloride (2.21 g), hydroxybenzotriazole (1.64 g), dicyclohexylcarbcdiimide (2.51 g) and diisopropylethylamine (2.1 ml) in dry dimethylformamide (25 ml) using the method of Description 9-(c). The yellow solid was chromatographed on silica gel using chloroform. This gave (2,3-dihydrobenzothiophene-2-carbonyl)-(S)-phenylalanyl-(S)-leucine methyl ester (1.60 g).
NMR (δ) ($CDCl_3$) 0.75-0.8(3H,m), 0.85-0.9(3H,m), 1.2-1.7(3H,m), 2.95-3.0(1H,d), 3.05-3.1(1H,t), 3.4-3.75-(5H,m), 4.2-4.3(1H,m), 4.4-4.7(2H,m), 6.08(d) and 6.3(d) (1H altogether), 6.9-7.0(1H,m), 7.0-7.4(9H, m).
M.S. (m/z) (EI pos.) M = 454.

f) (2,3-Dihydrobenzothiophene-2-carbonyl)-(S)-phenylalanyl-(S)-leucine

This material was obtained from (2,3-dihydrobenzothiophene-2-carbonyl)-(S)-phenylalanyl-(S)-leucine methyl ester (0.36 g) and sodium hydroxide (0.04 g in 10 ml of water) in 40 ml of methanol by the method of Description 9(d). This gave (2,3-dihydrobenzothiophene-2-carbonyl)-(S)-phenylalanyl-(S)-leucine (0.36 g).
NMR (δ) ($DMSOd_6$) 0.8-1.9(9H,m), 2.8-2.95(1H,m), 3.05-3.2(1H,m), 3.3-3.6(m), 4.3-4.4(1H,m), 4.5-4.7(2H,m), 7.05-7.35(9H,m), 8.35-8.45(2H,m), 12.5(1H, b).

Description 10

a) 2-(2,3-Dihydro-1,1-dioxobenzothiophene-3-yl)malonic acid

To a solution of diethyl 2-(2,3-dihydro-1,1-dioxobenzothiophene-3-yl)malonate (11.6 g) * in ethanol (150 ml) was added sodium hydroxide (4 g) in water (30 ml). The mixture was stirred at reflux for 4 h, cooled and the ethanol was removed under reduced pressure. The residue was diluted with water (20 ml) and was extracted with ether (3x100 ml) and dichloromethane. The aqueous layer was acidified to pH 1 with 5 M hydrochloric acid and was extracted with ether (3x80 ml) and dichloromethane (3x80 ml). The combined extracts (3x ether + 3x dichloromethane) were dried over sodium sulphate and the solvent was removed under reduced pressure to give (2,3-dihydro- 1,1-dioxobenzothiophene-3-yl)malonic acid (6.2 g).
NMR (δ) ($DMSOd_6$) 3.6-3.9(2H,m), 4.1-4.3(2H,m), 7.5-7.9(4H,m), 13.2(2H,b).

b) (2,3-Dihydro-1,1-dioxobenzothiophene-3-yl)acetic acid

2-(2,3-Dihydro-1,1-dioxobenzothiophene-3-yl)malonic acid * (1.9 g) was heated in xylene at reflux for 3 h. The mixture was cooled and extracted with saturated sodium hydrogen carbonate (2x50 ml). The combined aqueous extracts were washed with ether (50 ml) and acidified to pH 2 with 5 M hydrochloric acid. The mixture was extracted with ether (2x80 ml) and dichloromethane (2x80 ml). The combined extracts were dried over sodium sulphate and the solvent was removed under reduced pressure to give (2,3-dihydro-1,1-dioxobenzothiophene-3-yl)acetic acid (1.4 g).
NMR (δ) ($DMSOd_6$) 2.6-2.8(1H,m), 2.9-3.1(1H,m), 3.4-3.6(1H,m), 3.8-4.0(2H,m), 7.5-7.9(4H,m), 12.6(1H,b).

c) (2,3-Dihydro-1,1-dioxobenzothiophene-3-yl)acetyl-(S)-phenylalanyl-(S)-leucine methyl ester

This material was formed from (2,3-dihydro-1,1-dioxobenzothiophene-3-yl)acetic acid (0.5 g) and (S)-phenylalanyl-(S)-leucine methyl ester acetate salt following the procedure of Description 8(c), but using dichloromethane as solvent. This gave (2,3-dihydro-1,1-dioxobenzothiophene-3-yl)acetyl-(S)-phenylalanyl-

* J. Am. Chem. Soc. 1950, 72, 1985.

(S)-leucine methyl ester (0.9 g).
NMR (δ) ($CDCl_3$) 0.8-1.0(6H,m), 3.6(3H,s), 7.1-8.4(11H,m).

d) (2,3-Dihydro-1,1-dioxobenzothiophene-3-yl)acetyl-(S)-phenylalanyl-(S)-leucine

This material was formed from (2,3-dihydro-1,1-dioxobenzothiophene-3-yl)acetyl-(S)-phenylalanyl-(S)-leucine methyl ester (0.9 g) following the procedure of Description 2(b) to give (2,3-dihydro-1,1-dioxobenzothiophene-3-yl)acetyl-(S)-phenylalanyl-(S)-leucine (500 mg).
NMR (δ) ($DMSOd_6$) 0.8-1.1(6H,m), 1.6-1.9(3H,m), 2.4-2.6(1H,m), 2.7-2.9(2H,m), 3.0-3.6(3H,m), 3.8(1H,m), 4.3-4.4(1H,m), 4.6-4.9(1H,m), 7.2-7.4(5H,m), 7.4-7.9(4H,m), 8.3-8.5(2H,m).

Description 11

a) Ethyl (5-formyl-2,3-dihydrobenzofuran-3-yl)acetate

To ethyl (2,3-dihydrobenzofuran-3-yl)acetate (10.55g) in dry dimethylformamide (10.5 ml) was added cautiously, with stirring, phosphoryl chloride (55 ml). This mixture was stirred at reflux for 5 h, cooled, and poured cautiously into water (750 ml) with stirring, maintaining temperature below 80°C. This solution was cooled and extracted with ether (3x150 ml), and the extract was washed with 10% sodium carbonate solution (100 ml), dried ($Na_2SO_4$) and evaporated under reduced pressure to give a brown oil (7.94g). This was chromatographed on silica gel using ether/60-80 petroleum ether (gradient, 10-50% ether), giving recovered ethyl (2,3-dihydrobenzofuran-3-yl)acetate (2.59g), and ethyl (5-formyl-2,3-dihydrobenzofuran3-yl)-acetate (5.15g), contaminated with ca.25% of an unidentified, dihydrobenzofuran-derived, impurity.
NMR (δ) ($CDCl_3$) 1.3 (3H,t), 2.5-2.9 (2H, ABX), 3.9 (1H,m), 4.2 (2H,q), 4.4 (1H,dd), 4.9 (1H,t), 6.9 (1H,d), 7.7 (2H,m), 9.85 (1H,s).

b) (5-Formyl-2,3-dihydrobenzofuran-3-yl)acetic acid

This material was formed from ethyl (5-formyl-2,3-dihydrobenzofuran-3-yl)acetate (1.52g) following the procedure of Description 8(b). This gave (5-formyl-2,3-dihydrobebnzofuran-3-yl)acetic acid, contaminated with ca.25% of an unidentified, dihydrobenzofuran-derived impurity, as a light yellow powder (1.24g).
NMR (δ) ($DMSOd_6$) 2.6-2.9 (2H, ABX), 3.85 (1H,m), 4.4 (1H,dd), 4.85 (1H,t), 6.95 (1H,d), 7.75 (1H,dd), 7.8 (1H,s), 9.8 (1H,s), 12.3 (1H,b).

c) (5-Formyl-2,3-dihydrobenzofuran-3-yl)acetyl-(S)-phenylalanyl-(S)-leucine methyl ester

This material was formed from (5-formyl-2,3-dihydrobenzofuran-3-yl)acetic acid (0.26g) following the procedure of Description 8(c). This gave crude material (0.35g), which was chromatographed on silica gel using chloroform. This gave (5-formyl-2,3-dihydrobenzofuran-3-yl)acetyl-(S)-phenylalanyl-(S)-leucine methyl ester (0.16g) as a light yellow oil.
NMR (δ) ($CDCl_3$) 0.9 (6H,d), 1.1-1.7 (m), 2.4-2.8 (2H,m), 3.0-3.2 (2H,m), 3.7 (3H,s), 3.8-4.1 (2H,m), 4.2 (0.5H,dd), 4.35 (0.5H,dd), 4.45-4.9 (2H,m), 6.0 (1H,t), 6.25 (1H,t), 6.9 (1H,dd), 7.1-7.4 (5H,m), 7.7 (2H,m), 9.8 (1H,s).

d) (5-Formyl-2,3-dihydrobenzofuran-3-yl)acetyl-(S)-phenylalanyl-(S)-leucine

This material was formed from (5-formyl-2,3-dihydrobenzofuran-3-yl)acetyl-(S)-phenylalanyl-(S)-leucine methyl ester (0.16g) following the procedure of Description 8(b). This gave (5-formyl-2,3-dihydrobenzofuran-3-yl)acetyl-(S)-phenylalanyl-(S)-leucine (0.13g) as a yellow solid.
NMR (δ) ($DMSOd_6$) 0.9 (6H,m), 1.5-1.8 (3H,m), 2.3-2.8 (2H,m), 3.8 (1H,m), 4.1 (0.5H,dd), 4.35 (0.5H,dd), 4.45 (1H,m), 4.55-4.85 (2H,m), 6.85 (1H,dd), 7.1-7.3 (5H,m), 7.4-7.9 (4H,m), 9.8 (1H,d).

Description 12

a) Ethyl (5-carboxy-2,3-dihydrobenzofuran-3-yl)acetate

To ethyl (5-formyl-2,3-dihydrobenzofuran-3-yl)acetate (2.00g) in water at 75°C was added, with vigorous stirring, potassium permanganate (2.00g) in water (50 ml), over 10 min. After stirring at 75°C for a further 30 min, 5M HCl (10 ml) was added, followed by sufficient sodium metabisulphite (solid) to give a clear solution, which was cooled and extracted with ethyl acetate (3x50 ml). These combined organic extracts were then extracted with 5% sodium hydrogen carbonate solution (3x30 ml). Acidification of these combined aqueous extracts, extraction into chloroform (3x30 ml), drying ($Na_2SO_4$) and evaporation under reduced pressure gave ethyl (5-carboxy-2,3-dihydrobenzofuran-3-yl)acetate (1.01g) as a brown solid.

NMR (δ) ($CDCl_3$) 1.3 (3H,t), 2.55-2.9 (2H,ABX), 3.9 (1H,m), 4.2 (2H,g), 4.4 (1H,dd), 4.9 (1H,t), 6.85 (1H,d), 7.9 (1H,s), 8.0 (1H,d).

b) (5-(Methoxycarbonyl)-2,3-dihydrobenzofuran-3-yl)acetic hydrazide

Ethyl (5-carboxy-2,3-dihydrobenzofuran-3-yl)acetate (0.74g) and hydrazine hydrate (5 ml) were dissolved in ethanol (50 ml) and left to stand for 10 days.

Volatiles were then removed under reduced pressure, and the residue (0.84g) was dissolved in methanol (50 ml). Conc. sulphuric acid (1 ml) was added dropwise, and the mixture was heated at reflux for 22 h, neutralised with sodium hydrogen carbonate (solid), concentrated to small volume and poured into water (200 ml). The product was extracted into chloroform (3x50 ml), dried ($Na_2SO_4$) and evaporated, giving an orange oil, which was chromatographed on silica gel using methanol/chloroform (gradient, 0-5% methanol). This gave (5-(methoxycarbonyl)-2,3-dihydrobenzofuran-3-yl)acetic hydrazide (0.23g) as a yellow foam.

NMR (δ) ($CDCl_3$) 2.4-2.7 (2H,ABX), 3.85 (3H,s), 3.95 (1H,m), 4.4 (1H,dd), 4.8 (1H,t), 6.8 (1H,d), 7.85 (1H,s), 7.9 (1H,d).

Description 13

a) 4-(S)-(tert-Butoxycarbonylamino)-5-cyclohexyl-3-(S)-hydroxypentanoic acid 3-methylbutylamide

This material was formed from 4-(S)-(tert-butoxycarbonylamino)-5-cyclohexyl-3-(S)-hydroxypentanoic acid * (0.28g), hydroxybenzotriazole (0.13g), 3-methylbutylamine (0.11 ml) and dicyclohexylcarbodiimide (0.20g) in dry dimethylformamide (5 ml), by the method of Description 9(c). The crude product was chromatographed on silica gel using methanol/chloroform (0-2% methanol, gradient). This gave 4-(S)-(tert-butoxycarbonylamino)-5-cyclohexyl-3-(S)-hydroxypentanoic acid 3-methylbutylamide (0.18g).

NMR (δ) ($CDCl_3$) 0.8-1.0 (7H,d+m), 1.05-2.0 (24H,m), 2.2-2.45 (2H, ABX), 3.25 (2H,m), 3.6 (1H,m), 3.95 (1H, m), 4.7 (1H,d), 5.9 (1H,b).

b) 4-(S)-(-Benzyloxycarbonyl)-(S)-phenylalanyl-(S)-leucyl)amino-5-cyclohexyl-3-(S)-hydroxypentanoic acid 3-methylbutylamide

4-(S)-(tert-Butoxycarbonylamino)-5-cyclohexyl-3-(S)-hydroxypentanoic acid 3-methylbutylamide (0.18g) was dissolved in dichloromethane (6 ml) and stirred at 0°C as cold trifluoroacetic acid (6 ml) was added. After 30 min, solvents were removed *in vacuo* to give an oil, which was dissolved in dry dimethylformamide (5 ml) and cooled in ice. This solution was reacted with (benzyloxycarbonyl)-(S)-phenylalanyl-(S)-leucine (0.21g) in dry dimethylformamide (5 ml), using hydroxybenzotriazole (0.07g), dicyclohexylcarbodiimide (0.11g), and diisopropylethylamine (0.20 ml), by the method of Description 8(c). The crude product was chromatographed on silica gel using methanol/chloroform (0-2% methanol, gradient). This gave 4-(S)-(benzyloxycarbonyl)-(S)-phenylalanyl-(S)-leucyl)amino-5-cyclohexyl-3-(S)-hydroxypentanoic acid 3-methyl-

*J. Med. Chem. 1985, **28**, 1779, 1790.

butylamide (0.15g).
NMR (δ) ($CDCl_3$) 0.7-1.0 (14H,m), 1.1-1.9 (17H,m), 2.2 (2H,m), 2.8-3.05 (2H,m), 3.2 (2H,m), 3.95 (2H,m), 4.2-4.45 (2H,m), 4.95-5.15 (2H,m), 7.2-7.35 (10H,m).

## Description 14

### a) Ethyl (5-cyano-2,3-dihydrobenzofuran-3-yl)acetate

Ethyl (5-formyl-2,3-dihydrobenzofuran-3-yl)acetate (0.92g) and hydroxylamine hydrochloride (0.27g) were stirred in ethanol (20 ml) for 5 h. The solution was concentrated and poured into water. The crude product was extracted into chloroform, dried ($Na_2SO_4$) and evaporated. Elution through silica gel using chloroform gave a waxy solid (0.80g). This was stirred in dry dioxan (6 ml) with triethylamine (1.8 ml), and cooled in ice. Trifluoroacetic anhydride (0.50 ml) was added over 6 min, and the mixture was stirred for 6 h, warming to ambient temperature. 2M HCl (100 ml) was added, and the product was extracted into chloroform. The extract was washed with 5% sodium hydrogen carbonate solution, dried ($Na_2SO_4$) and evaporated. Residual aldehyde was removed by treatment with sodium borohydride (0.05g) in methanol (10 ml). The crude product was chromatographed on silica gel using ether/petroleum ether (b.p. 60-80°) (10-50% ether, gradient). This gave ethyl (5-cyano-2,3-dihydrobenzofuran-3-yl)acetate as a white solid (0.52g), still contaminated with ca.30% of the unidentified impurity of Description 11(a).
NMR (δ) ($CDCl_3$) 1.3 (3H,t), 2.55-2.8 (2H, ABX), 3.9 (1H,m), 4.2 (2H,g), 4.35 (1H,dd), 4.85 (1H,t), 6.85 (1H,d). 7.4-7.5 (2H,m).

### b) (5-Cyano-2,3-dihydrobenzofuran-3-yl)acetic acid

This material was formed from the corresponding ethyl ester (0.524g) and sodium hydroxide (1.27 ml of 10% aqueous solution) in ethanol (5 ml), using the method of Description 8(b). This gave (5-cyano-2,3-dihydrobenzofuran-3-yl)acetic acid (0.433g), still contaminated with ca.30% of the unidentified impurity of Description 11(b).
NMR (δ) ($DMSOd_6$) 2.55-2.95 (2H, ABX), 3.85 (1H,m), 4.35 (1H,dd), 4.85 (1H,t), 6.95 (1H,d), 7.65 (1H,d), 7.75 (1H,s), 12.4 (1H,b).

### c) (5-Cyano-2,3-dihydrobenzofuran-3-yl)acetyl-(S)-phenylalanyl-(S)-leucine methyl ester

This material was formed from (5-cyano-2,3-dihydrobenzofuran-3-yl)acetic acid (0.43g) following the procedure of Description 8(c). The crude product was chromatographed on silica gel using chloroform. This gave (5-cyano-2,3-dihydrobenzofuran-3-yl)acetyl-(S)-phenylalanyl-(S)-leucine methyl ester (0.34g) as a semi-solid, contaminated with a little dicyclohexylurea.
NMR (δ) 0.9 (6H,d), 2.4-2.7 (2H,2xABX), 3.05 (2H,m), 3.7 (3H,2xs), 3.9 (1H,m), 4.2 and 4.3 (1H,2xdd), 4.5 (1H,m), 4.6-4.8 (2H,m), 6.0 (1H,d), 6.25 (1H,t), 6.8 (1H,2xd), 7.2-7.35 (5H,m), 7.4-7.5 (2H,m).

### d) (5-Cyano-2,3-dihydrobenzofuran-3-yl)acetyl-(S)-phenylalanyl-(S)-leucine

This material was formed from the corresponding methyl ester (0.31g) and sodium hydroxide (0.36 ml of 10% aqueous solution) in ethanol (3 ml), using the method of Description 8(b). This gave (5-cyano-2,3-dihydro benzofuran-3-yl)acetyl-(S)-phenylalanyl-(S)-leucine (0.24g) as a light yellow foam.
NMR (δ) ($DMSOd_6$) 0.9-1.05 (6H,m), 1.5-1.8 (3H,m), 2.3-2.5(1H,m), 2.7-2.85 (1H,m), 3.1 (1H,m), 3.75 (1H,m), 4.0 (0.5H, dd), 4.2-4.4 (1.5H,m), 4.45-4.75 (2H,m + 2xt), 6.95 (1H,2xd), 7.2-7.4 (5H,m), 7.5-7.7 (2H,m), 8.2-8.5 (2H,m), 12.65 (1H,b).

## Description 15

a) Ethyl (5-(benzyloxycarbonyl)-2,3-dihydrobenzofuran-3-yl)acetate

Ethyl (5-carboxy-2,3-dihydrobenzofuran-3-yl)acetate (0.39g), anhydrous potassium carbonate (0.33g) and benzyl bromide (0.21 ml) were stirred for 60 h in dry dimethylformamide (4 ml). The mixture was diluted with ethyl acetate (50 ml), washed twice with water, and with brine; dried ($Na_2SO_4$) and evaporated. The product was chromatographed on silica gel using ether/petroleum ether (b.p. 60-80°) (0-30% ether, gradient). This gave ethyl (5-(benzyloxycarbonyl)-2,3-dihydrobenzofuran-3-yl)acetate (0.48g) as a colourless oil.

NMR ($\delta$) ($CDCl_3$) 1.2 (3H,t), 2.7 (2H, ABX), 3.6-4.5 (4H,t,q,m), 4.8 (1H,t), 5.3 (2H,s), 6.7 (1H,d), 7.1-7.4 (5H,s), 7.7-8.0 (2H,m).

b) (5-(Benzyloxycarbonyl)-2,3-dihydrobenzofuran-3-yl)acetic acid

Ethyl (5-(benzyloxycarbonyl)-2,3-dihydrobenzofuran3-yl)acetate (1.54g) was stirred in dioxan (20 ml). Lithium hydroxide hydrate (0.19g) was added in water (10 ml). After stirring for 16 h, the mixture was poured into water (200 ml), acidified (5M HCl), and the product was extracted into chloroform and evaporated. The residue was dissolved in ethyl acetate, and extracted into 5% sodium hydrogen carbonate solution. This extract was acidified (5M HCl), and the product was extracted into chloroform dried ($Na_2SO_4$) and evaporated, giving (5-(benzyloxycarbonyl)-2,3-dihydrobenzofuran-3-yl)acetic acid (1.17g) as an off-white solid.

NMR ($\delta$) ($CDCl_3$) 2.65 (1H) and 2.95 (1H) (ABX), 3.9 1H,m), 4.35 (1H,dd), 4.85 (1H,t), 5.3 (2H,s), 6.8 (1H,d), 7.3-7.5 (5H,m), 7.9-8.0 (2H,m).

Description 16

a) Ethyl 2-ethoxycarbonyl-4-(methylthio)phenoxyacetate

Ethyl 5-(methylthio)salicylate * (2.71g), anhydrous potassium carbonate (3.53g) and ethyl bromoacetate (1.42 ml) were stirred at reflux in AR acetone (75 ml) for 6 h. After cooling, the mixture was filtered, and the filtrate was concentrated, diluted with ether (100 ml), washed with water and brine, dried ($Na_2SO_4$) and evaporated. This gave ethyl 2-ethoxycarbonyl-4-(methylthio)phenoxyacetate (3.71g) as a light yellow oil.

NMR ($\delta$) ($CDCl_3$) 1.3 and 1.4 (6H,2xt), 2.45 (3H,s), 4.2 and 4.35 (4H,2xq), 4.6 (2H,s), 6.8 (1H,d), 7.3 (1H,m), 7.7 (1H,m).

b) Ethyl 3-hydroxy-5-(methylthio)benzofuran-2-carboxylate

Sodium (0.43g) was dissolved in ethanol (50 ml), and the resultant solution was evaporated to dryness. Ethyl 2-ethoxycarbonyl-4-(methylthio)phenoxyacetate (6.00g) was added in dry toluene (100 ml), and the mixture was stirred at reflux under nitrogen atmosphere for 1 h. After cooling, water (100 ml) and ethyl acetate (100 ml) were added, and the mixture was acidified (5M HCl). The layers were separated, and the aqueous portion was extracted with ethyl acetate (100 ml). The combined organics were washed with brine, dried ($MgSO_4$) and evaporated, giving ethyl 3-hydroxy- 5-(methylthio)benzofuran-2-carboxylate (4.94g) as a yellow solid.

NMR ($\delta$) ($CDCl_3$) 1.45 (3H,t), 2.5 (3H,s), 4.45 (2H,g), 6.8-8.2 (4H,m).

c) 5-(Methylthio)-2,3-dihydrobenzofuran-3-one

Ethyl 3-hydroxy-5-(methylthio)benzofuran-2-carboxylate (2.08g) was stirred in a mixture of 10% aqueous sodium hydroxide (100 ml) and ethanol (50 ml) for 2 h, and left to stand at ambient temperature for 9 days. It was then poured into water (500 ml), acidified (5M HCl) and extracted with chloroform. The extract was evaporated, dissolved in ethyl acetate (100 ml), washed with 5% sodium hydrogen carbonate solution,

*C.R. Acad. Sci. Paris Serie C, t.282, 993 (1976).

dried $Na_2SO_4$) and evaporated, giving a red solid (1.04g). This was chromatographed on silica gel using ether/petroleum ether (b.p. 60-80°) (10-40% ether, gradient). This gave a 5-(methylthio)-2,3-dihydrobe nzofuran-3-one (0.83g) as a yellow solid.
NMR (δ) ($CDCl_3$) 2.5 (3H,s), 4.65 (2H,s), 7.1 (1H,d), 7.55 (2H,m).

d) 5-Methanesulphonyl-2,3-dihydrobenzofuran-3-one

5-(Methylthio)-2,3-dihydrobenzofuran-3-one (0.83g) was stirred in glacial acetic acid (50 ml) as 30% aqueous hydrogen peroxide (1.15 ml) was cautiously added. The mixture was heated gently to reflux over 15 min, maintained at reflux for 15 min, and cooled, poured into water (500 ml), and neutralised by addition of solid sodium hydrogen carbonate. The product was extracted into chloroform, dried ($Na_2SO_4$), and evaporated. This gave 5-methanesulphonyl-2,3-dihydrobenzofuran-3-one (0.58g) as an orange solid.
NMR (δ) ($CDCl_3$) 3.1 (3H,s), 4.8 (2H,s), 7.3 (1H,d), 8.2 (1H,dd), 8.3 (1H,d).

e) Ethyl (5-(methanesulphonyl)benzofuran-3-yl)acetate

5-Methanesulphonyl-2,3-dihydrobenzofuran-3-one (0.68g) and (carbethoxymethylene)-triphenylphosphorane (1.34g) were stirred at reflux in dry toluene (100 ml) for 16h, cooled, and evaporated to dryness. The product was chromatographed on silica gel using ether/petroleum (b.p. 60-80°) (50-100% ether, gradient). This gave ethyl (5-(methanesulphonyl)benzofuran-3-yl)acetate (0.28g) as a white solid, containing ca.10% of the exo-double-bond isomers.
NMR (δ) ($CDCl_3$) 1.3 (3H,t), 3.1 (3H,s), 3.75 (2H,s), 4.2 (2H,q), 7.65 (1H,d), 7.8 (1H,s), 7.9 (1H,dd), 8.25 (1H,d).

f) Ethyl (5-(methanesulphonyl)-2,3-dihydrobenzofuran-3-yl)acetate

Ethyl (5-(methanesulphonyl)benzofuran-3-yl)acetate (0.32g), (mixture with minor amounts of exo-double-bond isomers) was hydrogenated over 10% palladium on charcoal (60% aqueous paste, 0.20g) in ethyl acetate 20 ml) for 17 h. The mixture was filtered, and the filtrate was evaporated, giving ethyl (5-(methanesulphonyl)-2,3-dihydrobenzofuran-3-yl)acetate (0.31g) as a colourless oil, crystallising on addition of ethanol.
NMR (δ) ($CDCl_3$) 1.3 (3H,t), 2.7 (1H) and 2.85 (1H) (ABX), 3.05 (3H,s), 3.95 (1H,m), 4.2 (2H,q), 4.4 (1H,dd), 4.9 (1H,t), 6.9 (1H,d), 7.75 (2H,m).

g) (5-(Methanesulphonyl)-2,3-dihydrobenzofuran-3-yl)acetic acid

Ethyl (5-(methanesulphonyl)-2,3-dihydrobenzofuran-3-yl) acetate (0.30g) was stirred in ethanol (6 ml). 10% aqueous sodium hydroxide (0.60 ml) was added, and water was added as required to maintain a solution of the sodium salt so formed. After 1.5 h, the reaction was worked up using the method of Description 8(b). This gave (5-(methanesulphonyl)-2,3-dihydrobenzofuran-3-yl) acetic acid (0.20g) as a white solid.
NMR (δ) ($CDCl_3$) 2.7 (1H) and 2.9 (1H) (ABX), 3.05 (3H,s), 3.95 (1H,m), 4.4 (1H,dd), 4.9 (1H,t), 6.95 (1H,d), 7.8 (2H,m).

Description 17

4-(S)-(Benzyloxycarbonyl-(S)-phenylalanyl-(S)-histidyl)amino-5-cyclohexyl-3-(S)-hydroxypentanoic acid isobutylamide

This material was formed from benzyloxycarbonyl-(S)-phenylalanyl-(S)-histidine (0.74g) and 4-(S)-amino-5-cyclohexyl-3-(S)-hydroxypentanoic acid isobutylamide as for Example 10 except that the citric acid wash was omitted. The product was chromatographed on silica gel using chloroform containing 0 - 10%

methanol. This gave 4-(S)-(benzyloxycarbonyl-(S)-phenylalanyl-(S)-histidyl)amino-5-cyclohexyl-3-(S)-hydroxypentanoic acid isobutylamide.
NMR (δ) ($DMSOd_6$) 0.7-2.4 (22H,m), 3.8-4.0 (2H,b), 4.4 (1H,m), 4.6 (1H,m), 7.2-7.5 (10H,m).

Description 18

4-(S)-((S)-Phenylalanyl-(S)-histidyl)amino-5-cyclohexyl-3-(S)-hydroxypentanoic acid isobutylamide

4-(S)-(Benzyloxycarbonyl-(S)-phenylalanyl-(S)-histidyl)-amino-5-cyclohexyl-3-(S)-hydroxypentanoic acid isobutylamide (0.276g) was hydrogenated over 10% palladium on charcoal (60% aqueous paste, 0.20g) in ethanol (20 ml) for 5 h. A further portion of catalyst (0.20g) was then added, and hydrogenation continued for 4 h. The mixture was filtered, and the filtrate was evaporated, giving 4-(S)-((S)-phenylalanyl-(S)-histidyl)-amino- 5-cyclohexyl-3-(S)-hydroxypentanoic acid isobutylamide (0.21g) as a colourless semi-solid.

Description 19

4-(S)-(tert-Butoxycarbonyl-(S)-phenylalanyl-(S)-leucyl)amino-5-cyclohexyl-3-(S)-hydroxvoentanoic acid isobutylamide

This material was formed from tert-butoxycarbonyl-(S)-phenylalanyl-(S)-leucine (2.1g) and 4-(S)-amino-5-cyclohexyl-3-(S)-hydroxypentanoic acid isobutylamide (1.5g) as described in Description 2(c). This gave 4-(S)-(tert-butoxycarbonyl-(S)-phenylalanyl-(S)-leucyl)amino-5-cyclo-hexyl-3-(S)-hydroxypentanoic acid isobutylamide (2.32g).
NMR (δ) ($CDCl_3$) 0.7-2.3 (31H,m), 2.8-3.2 (4H,m), 3.8-4.3 (4H,m), 6.4-7.4 (8H,m).

Description 20

4-(S)-((S)-Phenylalanyl-(S)-leucyl)amino-5-cyclohexyl-3-(S)-hydroxvoentanoic acid isobutylamide trifluoroacetate

This material was formed from 4-(S)-(tert-butoxycarbonyl-(S)-phenylalanyl-(S)-leucyl)amino-5-cyclohexyl-3-(S)-hydroxypentanoic acid isobutylamide (2.32g) and trifluoroacetic acid (10 ml) as described in Description 30. This gave 4-(S)-((S)-phenylalanyl-(S)-leucyl)-amino-5-cyclohexyl-3-(S)-hydroxypentanoic acid isobutylamide trifluoroacetate.
NMR (δ) ($CDCl_3$) 0.6-2.5 (31H,m), 2.8-3.3 (4H,m), 3.7-4.4 (4H,m), 7.0-8.0 (8H,m).

Description 21

(6-Nitro-2,3-dihydro-1,1-dioxobenzothiophene-3-yl)acetic acid

(2,3-Dihydro-1,1-dioxobenzothiophene-3-yl)acetic acid (2.00g) was added portionwise to fuming nitric acid (9 ml), cooled in a water bath; stirring was continued for 4.5 h. The solution was poured into water (75 ml) and the precipitated solid filtered off. The filtrate was cooled to 0° and the filtration repeated. The combined solids were air-dried to give (6-nitro-2,3-dihydro-1,1- dioxobenzothiophene-3-yl)acetic acid (1.96g).
NMR (δ) ($DMSOd_6$) 2.8-2.9 (1H,m), 3.0-3.1 (1H,dd), 3.6-3.7 (1H,m), 3.9-4.1 (2H,m), 7.9 (1H,m), 8.5 (2H,m), 12.7 (1H,b).
MS (m/z) (EI pos) (M) = 271.

Description 22

Ethyl (6-nitro-2,3-dihydro-1,1-dioxobenzothiophene-3-yl)acetate

(6-Nitro-2,3-dihydro-1,1-dioxobenzothiophene-3-yl) acetic acid (26.57g) was taken up in anhydrous ethanol (200 ml) and concentrated sulphuric acid (15 ml) and heated at reflux for 1.5 h. After concentration of the solution to half volume, the mixture was poured into water (100 ml) and the precipitated solid filtered off. This was air-dried to give ethyl (6-nitro-2,3-dihydro-1,1-dioxobenzothiophene-3-yl)acetate (27.59g).
NMR (δ) ($CDCl_3$) 1.2-1.3 (3H,t), 2.8-3.0 (2H,m), 3.4-3.5 (1H,dd), 3.8-3.9 (1H,dd), 4.1-4.3 (3H,m), 7.6 (1H,d), 8.5 (1H,m), 8.6 (1H,d).

Description 23

Ethyl (6-amino-2,3-dihydro-1,1-dioxobenzothiophene-3-yl)acetate

A solution of ethyl (6-nitro-2,3-dihydro-1,1-dioxobenzothiophene-3-yl)acetate (1.47g) in ethanol (100 ml) was hydrogenated at room temperature and pressure for 4 h in the presence of 10% palladium on carbon. The catalyst was removed by filtration through Kieselguhr and the filtrate evaporated to give ethyl (6-amino-2,3-dihydro-1,1-dioxobenzothiophene-3-yl)acetate (1.2g).
NMR (δ) ($CDCl_3$) 1.2-1.3 (3H,t), 2.6-2.9 (2H,m), 3.2-3.4 (1H,dd), 3.6-3.7 (1H,m), 3.8-3.9 (1H,m), 4.1-4.2 (2H,g), 6.8-7.0 (2H,m), 7.1-7.2 (1H,m).

Description 24

Ethyl (6-bromo-2,3-dihydro-1,1-dioxobenzothiophene-3-yl)acetate

Ethyl (6-amino-2,3-dihydro-1,1-dioxobenzothiophene-3-yl)acetate (10.81g) was taken up in aqueous hydrobromic acid with heating to aid solution. The solution was then cooled to 0°. A solution of sodium nitrite (2.80g) in water (4.5 ml) was added dropwise (evolution of brown fumes). Once evolution had ended the mixture was allowed to warm to room temperature. Copper bronze (0.5g) was added, leading to immediate effervescence. After heating gently for 30 min the purple solution was poured into ice. Residual copper bronze was filtered off through Kieselguhr and the filtrate extracted with chloroform (3x50 ml). The combined organics were dried ($Na_2SO_4$) and the solvents removed under reduced pressure to give (6-bromo-2,3-dihydro-1,1-dioxobenzothiophene-3-yl)acetic acid (8g). This was heated to reflux in anhydrous ethanol (150 ml) and concentrated sulphuric acid (6 ml) for 1.5 h. After cooling, the solution was concentrated to half volume, poured into water and extracted with chloroform (4x50 ml). The combined organic layers were washed with sodium bicarbonate (2x30 ml), dried ($Na_2SO_4$) and the solvents removed to give ethyl (6-bromo-2,3-dihydro-1,1-dioxobenzothiophene-3-yl)acetate (8.13g).
NMR (δ) ($CDCl_3$) 1.2-1.3 (3H,t), 2.7-2.9 (2H,m), 3.3-3.4 1H,dd), 3.7-3.8 (1H,m), 3.9-4.0 (1H,m), 4.1-4.3 (2H,q), 7.2-7.3 (1H,m), 7.7 (1H,dd)7.9 (1H,d).

Description 25

Ethyl (6-cyano-2,3-dihydro-1,1-dioxobenzothiophene-3-yl)acetate

Ethyl (6-bromo-2,3-dihydro-1,1-dioxobenzothiophene-3-yl)acetate (13.11g) was taken up in dimethylformamide (120 ml) and stirred at room temperature with copper (I) cyanide (6.9g). The resulting solution was refluxed overnight under nitrogen. After cooling, the copper complex was broken down with aqueous 1,2-diaminoethane (500 ml). The resulting blue solution was extracted with ether (5x100 ml). The combined organic extracts were washed with brine (100 ml), and the solvents removed *in vacuo* to give a beige solid (5.88g). This was purified by chromatography on silica gel (150 g) using 0-30% ethyl acetate in petroleum

ether as eluent to afford ethyl (6-cyano-2,3-dihydro-1,1-dioxobenzothiophene-3-yl)acetate (4.98g).
NMR (δ) ($CDCl_3$) 1.2-1.3 (3H,t), 2.8-3.0 (2H,m), 3.3-3.5 1H,m), 3.7-3.8 (1H,m), 4.0-4.3 (3H,m), 7.6 (1H,d), 7.9 (1H,dd), 8.1 (d).

## Description 26

### (6-Cyano-2,3-dihydro-1,1-dioxobenzothiophene-3-yl)acetic acid

Ethyl (6-cyano-2,3-dihydro-1,1-dioxobenzothiophene-3-yl)acetate (300 mg) was added to ethanol at room temperature and 10% sodium hydroxide solution (0.48 ml, 1.1 eq) added dropwise with stirring. After stirring for 16 h the solvent was removed in vacuo and the residues taken up in water (100 ml). The solution was washed with chloroform (2x15 ml), acidified to pH 0 and extracted with chloroform (4x100 ml) and ether (2x50 ml). The combined organic extracts were dried over sodium sulphate and evaporated under reduced pressure to give (6-cyano-2,3-dihydro-1,1-dioxobenzothiophene-3-yl)acetic acid (256 mg).
NMR (δ) ($DMSOd_6$) 2.8-3.0 (1H,m), 3.0-3.2 (1H,m), 3.9-4.2 (1H,m), 7.7-8.4 (3H,m).

## Description 27

### 1,1-Dioxo-2-(prop-2-en-1-yl)-2,3-dihydrobenzothiophene

1,1-Dioxo-2,3-dihydrobenzothiophene (0.5g) was dissolved in THF (20 ml) and cooled to -30° under nitrogen. N-Butyllithium (1.6 M in hexane, 2 ml) was added dropwise. After 1 h allyl bromide (0.29 ml) was added in one portion and stirring continued for 3 h. The solution was diluted with dichloromethane (100 ml), dried ($Na_2SO_4$) and the solvents removed in vacuo to give 1,1-dioxo-2-(prop-2-en-1-yl)-2,3-dihydrobenzothiophene (0.47g).
NMR (δ) ($CDCl_3$) 2.3-2.4 (1H,m), 2.7-3.0 (2H,m), 3.3-3.5 (2H,m), 5.1-5.2 (2H,m), 5.7-5.9 (1H,m), 7.2-7.7 (4H,m).

## Description 28

### (1.1-Dioxo-2,3-dihydrobenzothiophene-2-yl)acetic acid

1,1-Dioxo-2-(prop-2-en-1-yl)-2,3-dihydrobenzothiophene (0.48g) was dissolved in acetone (50 ml) at 0°C. Sodium bicarbonate (0.095g) was added in one portion. Potassium permanganate (1.49g) was added over 1.5 h with vigorous stirring. After removal of the acetone under reduced pressure, the residues were taken up in water and dilute sulphuric acid and scdium sulphite added. When the solution was clear it was extracted with ether (2x100 ml) and dichloromethane (2x100 ml). The combined organic extracts were dried over sodium sulphate and evaporated under reduced pressure to give (1,1-dioxo-2,3-dihydrobenzothiophene-2-yl)acetic acid (400 mg).
NMR (δ) ($DMSOd_6$) 2.7-3.0 (2H,m), 3.0-3.2 (1H,m), 3.6-4.1 (3H,m), 7.6-8.0 (4H,m).

## Description 29

### 4-(S)-(tert-Butoxycarbonyl-(S)-β-(pyrazol-1-yl)alanyl)amino-5-cyclohexyl-3-(S)-hydroxypentanoic acid isobutylamide

This material was formed from (S)-tert-butoxycarbonyl-(S)-β-(pyrazol-1-yl)alanine * (250 mg) and 4-(S)-amino-5-cyclohexyl-3-(S)-hydroxypentanoic acid isobutylamide (265 mg) as described in Description 2(c).

*See J.A.C.S. 1988, 110, 2237-41.

This gave 4-(S)-((S)-tert-butoxycarbonyl-(S)-β-(pyrazol-1-yl)alanyl)amino-5- cyclohexyl-3-(S)-hydroxypentanoic acid isobutylamide (240 mg).
NMR (δ) ($CDCl_3$) 0.6-2.5 (28H,m), 2.8-3.2 (2H,m), 4.3-4.6 (3H,m), 6.2 (1H,m), 7.3-7.6 (4H,m).
M.S. (m/z) $C_{26}H_{45}N_5O_5$ requires 507.3420. Found 507.3421.

Description 30

4-(S)-((S)-β-(pyrazol-1-yl)alanyl)amino-5-cyclohexyl-3-(S)-hydroxypentanoic acid isobutylamide bistrifluoroacetate

To an ice cooled solution of 4-(S)-(tert-butoxycarbonyl-(S)-β-(pyrazol-1-yl)alanyl)amino-5-cyclohexyl-3-(S)-hydroxypentanoic acid isobutylamide (240 mg) was added chilled trifluoroacetic acid (3 ml) dropwise with stirring. After 1 h the reaction mixture was concentrated under reduced pressure. Trituration in diethyl ether and filtration afforded 4-(S)-((S)-β- (pyrazolyl-1-yl)alanyl)amino-5-cyclohexyl-3-(S)-hydroxypentanoic acid isobutylamide bistrifluoroacetate (248 mg).
NMR (δ) ($DMSOd_6$) 0.7-2.3 (20H,m), 2.5-4.9 (6H,m), 6.4 (1H,m), 7.5-8.6 (6H,m).
M.S. (m/z) $C_{21}H_{37}N_5O_3$ requires 407.2897. Found 407.2896.

Description 31

4-(S)-(Benzyloxycarbonyl-(S)-phenylalanyl-(S)-β-(pyrazol-1-yl)alanyl)amino-5-cyclohexyl-3-(S)-hydroxypentanoic acid isobutylamide hemihydrate

This material was formed from benzyloxycarbonyl-(S)-phenylalanine (105 mg) and 4-(S)-((S)-3-(pyrazol-1-yl)alanyl)amino-5-cyclohexyl-3-(S)-hydroxypentanoic acid isobutylamide bistrifluoroacetate (224 mg) as described in Description 8(c) except that N-ethylmorpholine was used instead of diisopropylethylamine. Evaporation of the reaction mixture under reducd pressure and chromatography on silica gel using 0-1% methanol/chloroform gave 4-(S)-(benzyloxycarbonyl-(S)-phenylalanyl-(S)-β-(pyrazol-1-yl)alanyl)amino-5-cyclohexyl-3-(S)-hydroxypentanoic acid isotutylamide hemihydrate (144 mg).
NMR (δ) ($CD_3OD$) 0.8-2.1 (20H,m), 2.30 (2H,d), 3.0-3.3 (4H,m), 4.1-5.0 (7H,m), 5.2 (2H,d), 6.4 (1H,m), 7.3-7.9 (12H,m).
Analysis: $C_{38}H_{52}N_6O_6.0.5H_2O$ requires C,65.4; H,7.7; N,12.0%. Found C,65.6; H,7.6; N,11.8%.
M.S. (m/z)(FAB) (M+1) = 689 (consistent with m.w.=688).

Description 32

4-(S)-((S)-Phenylalanyl-(S)-β-(pyrazol-1-yl)alanyl)amino-5-cyclohexyl-3-(S)-hydroxypentanoic acid isobutylamide dihydrobromide

To a stirred solution of 4-(S)-(benzyloxycarbonyl-(S)-phenylalanyl-(S)-β-(pyrazol-1-yl)alanyl)amino-5-cyclohexyl-3-(S)-hydroxypentanoic acid isobutylamide hemihydrate (78 mg) in glacial acetic acid (2 ml) was added 48% hydrogen bromide in acetic acid (1 ml) dropwise. After 1.5 h the reaction mixture was evaporated under reduced pressure. The residue was triturated in ether and the solvent decanted to afford 4-(S)-((S)-phenylalanyl-(S)-β-(pyrazol-1-yl)alanyl)amino-5- cyclohexyl-3-(S)-hydroxypentanoic acid isobutylamide dihydrobromide (78 mg).
M.S. (m/z)(FAB) (M+1)=555 (consistent with m.w.=554).

Description 33

Benzyloxycarbonyl-(S)-β-(4-methylpyrazol-1-yl)alanine

4-Methylpyrazole (0.20 ml) was added dropwise to a stirred solution of benzyloxycarbonyl-S-serine-β-lactone * (520 mg) in acetonitrile (10 ml) and the resultant solution warmed at 50°C for 42 h. Evaporation under reduced pressure afforded benzyloxycarbonyl-(S)-β-(4-methylpyrazol-1-yl)alanine (690 mg).
NMR (δ) ($CDCl_3$) 1.8-2.3 (3H,m), 3.9-4.2 (1H,m), 4.7 (2H,s), 5.1 (2H,s), 5.5-5.9 (1H,m), 7.3 (5H,s), 10.4 (1H,bs).

Description 34

4-(S)-(Benzyloxycarbonyl-(S)-β-(4-methylpyrazol-1-yl)alanyl)amino-5-cyclohexyl-3-(S)-hydroxypentanoic acid isobutylamide

This material was formed from benzyloxycarbonyl-(S)-β-(4-methylpyrazol-1-yl)alanine (500 mg) and 4-(S)-amino-5-cyclohexyl-3-(S)-hydroxypentanoic acid isobutylamide (500 mg) as described in Description 8-(c) except for the omission of diisopropylethylamine. The crude product was chromatographed on silica gel using 0-3% methanol/chloroform to afford 4-(S)-(benzyloxycarbonyl-(S)-β-(4-methylpyrazol-1-yl)alanyl)-amino-5-cyclohexyl-3-(S)-hydroxypentanoic acid isobutylamide (243 mg).
NMR (δ) ($CD_3OD$) 0.8-2.0 (20H,m), 2.1 (3H,s), 2.3 (2H,d), 3.1 (2H,d), 3.9-4.8 (6H,m), 5.2 (2H,s), 7.3-7.6 (7H,m).
M.S. (m/z)(FAB) (M + 1) = 556 (consistent with m.w. 555).

Description 35

4-(S)-((S)-β-(4-Methylpyrazol-1-yl)alanyl)amino-5-cyclohexyl-3-(S)-hydroxypentanoic acid isobutylamide dihydrobromide

This material was formed from 4-(S)-(benzyloxycarbonyl)-(S)-β-(4-methylpyrazol-1-yl)alanyl)amino-5-cyclohexyl-3-(S)-hydroxypentanoic acid isobutylamide (243 mg) and 48% hydrogen bromide in acetic acid (2 ml) as described in Description 32. This gave 4-(S)-((S)-β-(4-methylpyrazol-1-yl)alanyl)amino-5-cyclohexyl-3-(S)-hydroxypentanoic acid isobutylamide dihydrobromide (250 mg).
NMR (δ) ($CD_3OD$) 0.8-2.4 (24H,m), 3.0 (2H,d), 3.9-4.8 (6H,m), 7.3-7.6 (2H,m).
M.S. (m/z)(FAB) (M + 1) = 422 (consistent with m.w. = 421).

Description 36

a) (5-Nitro-2,3-dihydrobenzofuran-3-yl)acetyl-(S)-phenylalanine

This material was formed from (5-nitro-2,3-dihydrobenzofuran-3-yl)acetic acid (1.1g) (Description 5(b)) and (S)-phenylalanine methyl ester hydrochloride as in Description 2(a), followed by hydrolysis as in Description 2(b). This gave (5-nitro-2,3-dihydrobenzofuran-3-yl)acetyl-(S)-phenylalanine (1.05g).
NMR (δ) ($DMSOd_6$) 2.3-3.1 (4H,m), 3.8 (1H,m), 4.1 ($\frac{1}{2}$H,m), 4.3-4.8 ($2\frac{1}{2}$H,m), 6.9 (1H,m), 7.2 (5H,b) , 8.1 (2H,b), 8.4 (1H,m), 12.8 (1H,bs).

b) (5-Nitro-2,3-dihydrobenzofuran-3-yl)acetyl-(S)-phenylalanyl-(S)-histidine methyl ester

This material was formed from (5-nitro-2,3-dihydrobenzofuran-3-yl)acetyl-(S)-phenylalanine (1.05g) and (S)-histidine dihydrochloride as in Description 2(a) except that the citric acid wash was omitted. This gave (5-nitro-2,3-dihydrobenzofuran-3-yl)acetyl-(S)-phenylalanyl-(S)-histidine methyl ester (1.45g).

*See J.A.C.S. 1985, 107, 7105-9.

NMR (δ) ($DMSOd_6$) 2.3-3.3 (6H,m), 3.7 (3H,d), 4.1 (½H,m), 4.4 (½H,m), 4.6-4.9 (3H,m), 6.9-7.1 (2H,2xm), 7.4 (5H,m), 12.0 (1H,b).

c) (5-Nitro-2,3-dihydrobenzofuran-3-yl)acetyl-(S)-phenylalanyl-(S)-histidine

(5-Nitro-2,3-dihydrobenzofuran-3-yl)acetyl-(S)-phenylalanyl-(S)-histidine methyl ester (1.45g) was dissolved in methanol (40 ml) and potassium hydroxide (0.17g) in water (10 ml) was added in one portion at 0°. After two and a half hours, the mixture was filtered and the methanol was removed under reduced pressure. The solution was diluted with water (10 ml) and extracted with ethyl acetate. The aqueous layer was adjusted to pH 6.5 (1M HCl) and filtered to give (5-nitro-2,3-dihydrobenzofuran-3-yl)acetyl-(S)-pherylalanyl-(S)-histidine.
NMR (δ) ($DMSOd_6$) 2.3-3.2 (6H,m), 3.7 (1H,m), 4.0 (½H,m), 4.3-4.9 (3½H,m), 6.8-7.0 (2H,2xm), 7.2 (5H,m).

Description 37

a) Ethyl (2,3-dihydro-3-oxo-1H-isoindol-1-ylidine)acetate

(Carbethoxymethylene)triphenylphosphorane (4.15g) and phthalimide (0.81g) were heated at reflux in dry toluene (20 ml) under nitrogen for 18 h. Solvent was removed in vacuo, and the product was chromatographed on silica gel using ether/petroleum ether (b.p. 60-80°C) (50-70% ether, gradient). This gave the title compound (0.87g) as a light yellow powder. NMR indicates the presence of a single isomer. *
NMR (δ) ($CDCl_3$) 1.35 (3H, t), 4.3 (2H, g), 5.8 (1H, s), 7.6-7.9 (4H, m), 9.65 (1H, b).

b) Ethyl 2,3-dihydro-3-oxo-1H-isoindole-1-acetate

Ethyl (2,3-dihydro-3-oxo-1H-isoindol-1-ylidine)acetate hydrogenated over 10% palladium on charcoal (60% aqueous paste, 0.15g) in ethanol (25 ml) for 4h. Catalyst was filtered off, and evaporation of solvent afforded the title compound (0.17g) as a white solid.
NMR (δ) ($DMSOd_6$) 1.15 (3H, t), 2.6 and 2.95 (2H, ABX), 4.1 (2H, q), 4.9 (1H, t), 7.5-7.7 (4H, m), 8.7 (1H, b).

c) 2,3-Dihydro-3-oxo-1H-isoindole-1-acetic acid

This material was formed from ethyl 2,3-dihydro-3-oxo-1H-isoindole-1-acetate (0.17g), following the procedure of Description 8 (b). This gave the title compound (0.042g) as a white solid.
NMR (δ) ($DMSOd_6$) 2.5 and 2.8 (2H, ABX), 4.9 (1H, t), 7.4-7.7 (4H, m), 8.6 (1H, s), 12.3-12.8 (1H, b).

Description 38

a) Ethyl (5-(chlorosulphonyl)-2,3-dihydrobenzofuran-3-yl)acetate

Ethyl (2,3-dihydrobenzofuran-3-yl)acetate (6.25g) was stirred in chloroform (30 ml) at 0°C as chlorosulphonic acid (17.5 ml) was added dropwise over 5 min. The mixture was stirred for 20 min, and poured onto crushed ice. The product was extracted into ethyl acetate, washed with 5% aqueous sodium hydrogen carbonate solution and brine, and dried ($Na_2SO_4$). Evaporation gave the title compound (8.16g) as a light yellow oil which solidified on standing.
NMR (δ) ($CDCl_3$) 1.3 (3H,t), 2.7 (1H) and 2.8 (1H)(ABX), 4.0 (1H,m), 4.2 (2H,q), 4.45 (1H,dd), 4.95 (1H,t), 6.95 (1H,d), 7.8-7.95 (2H,m).

* W. Flitsch, S.R. Schindler, Synthesis 1975, 685.

b) Ethyl (5-mercapto-2,3-dihydrobenzofuran-3-yl)acetate

Ethyl (5-(chlorosulphonyl)-2,3-dihydrobenzofuran-3-yl)acetate (1.05g) was stirred in ethanol (5 ml) as zinc powder (1.5g) and concentrated hydrochloric acid (6 ml) were added portionwise over 5 min. The mixture was stirred for 10 min, partitioned between water and chloroform, and filtered. The filtrate was separated, and the aqueous portion was further extracted with chloroform. The combined organic portions were dried ($Na_2SO_4$) and evaporated, giving the title compound (0.76g) as a malodorous, light yellow oil.
NMR (δ) ($CDCl_3$) 1.3 (3H,t), 2.55 (1H) and 2.75 (1H) (ABX), 3.35 (1H,s), 3.85 (1H,m), 4.2-4.5 (3Hmm), 4.75 (1H,t), 6.7 (1H,d), 7.15 (2H,m).

c) Disulphide of ethyl (5-mercapto-2,3-dihydrobenzofuran-3-yl)acetate

Ethyl (5-mercapto-2,3-dihydrobenzofuran-3-yl)acetate (0.23g) was stirred in ethanol (5 ml) as a solution of iodine (0.12g) in ethanol (10 ml) was added dropwise, until colouration persisted. The mixture was stirred for 5 min, diluted with water, and sufficient sodium thiosulphate was added to discharge the colouration. The product was extracted into ethyl acetate, dried ($Na_2SO_4$) and evaporated, giving the title compound (0.22g) as a light green semi-solid.
NMR (δ) ($CD_3OD$) 1.2 (6H,t), 2.5-2.8 (4H,m), 3.85 (2H, m), 4.1 (4H,q), 4.3 (2H,m), 4.75 (2H,m), 6.7 (2H, 2xd), 7.15-7.35 (4H,m).

d) Disulphide of (5-mercapto-2,3-dihydrobenzofuran-3-yl)acetic acid

This material was formed from the disulphide of ethyl (5-mercapto-2,3-dihydrobenzofuran-3-yl)acetate (0.217g), following the procedure of Description 8 (b), but isolating the product by extraction with ethyl acetate. This gave the title compound (0.17g) as an off-white solid.
NMR (δ) ($CD_3OD$) 2.5-2.8 (4H, 2xABX), 3.85 (2H,m), 4.3 (2H,m), 6.7 (2H,2xd), 7.2 (2H,m), 7.35 (2H,m).

Description 39

a) Ethyl (5-(tert-butoxycarbonylmethylthio)-2,3-dihydrobenzofuran-3-yl)acetate

Ethyl (5-mercapto-2,3-dihydrobenzofuran-3-yl)acetate (0.24g), tert-butyl bromoacetate (0.16 ml) and anhydrous potassium carbonate (0.17g) were stirred in dry dimethylformamide (4 ml) for 16h. Ethyl acetate (50 ml) was added, and the mixture was washed with water and brine, dried ($Na_2SO_4$) and evaporated. This gave the title compound (0.35g) as a colourless oil.
NMR (δ) ($CDCl_3$) 1.3 (3H,t), 1.4 (9H,s), 2.5-2.9 (2H, ABX), 3.4 (2H,s), 3.85 (1H,m), 4.2 (2H,q), 4.3 (1H,m), 4.8 (1H,t), 6.75 (1H,d), 7.3 (2H,m).

b) (5-(tert-Butoxycarbonylmethylthio)-2,3-dihydrobenzofuran-3-yl)acetic acid

This material was formed from ethyl (E-(tert-butoxycarbonylmethylthio)-2,3-dihydrobenzofuran-3-yl)-acetate (0.345g), following the procedure of Description 15 (b). This gave the title compound (0.218g) as a colourless oil, contaminated with ca.15% of the product of hydrolysis of the tert-butyl ester.
NMR (δ) ($CDCl_3$) 1.4 (9H,s), 2.6-2.9 (2H, ABX), 3.4 (2H, s), 3.85 (1H,m), 4.3 (1H,dd), 4.8 (1H,t), 6.75 (1H,d), 7.3 (2H,m).

c) (5-(tert-Butoxycarbonylmethylthio) -2,3-dihydrobenzofuran-3-yl)acetyl-(S)-phenylalanyl-(S)-leucine benzyl ester

This material was formed from (5-(tert-butoxycarbonylmethylthio)-2,3-dihydrobenzofuran-3-yl)acetic acid (0.214g) and (S)-phenylalanyl-(S)-leucine benzyl ester trifluoroacetate salt (0.318g), following the procedure of Description 8 (c). The crude product was chromatographed on silica gel using ethyl acetate/petroleum

ether (b.p. 60-80°C) (20-50% ethyl acetate, gradient). This gave the title compound (0.328g) as a colourless oil.

NMR (δ) ($CDCl_3$) 0.9 (6H, d), 1.4 (9H, s), 1.1-1.7 (m), 2.3-2.45 (1H) and 2.55-2.7 (1H) (2xABX), 3.05 (2H, m), 3.4 (2H, s), 3.8 (1H, m), 4.1 (0.5H, dd), 4.25 (0.5H, dd), 4.5-4.7 (3H, m), 5.15 (2H, ABq), 6.0-6.2 (2H, m), 6.7 (1H, m), 7.15-7.3 (12H, m).

d) (5-(tert-Butoxycarbonylmethanesulphonyl)-2,3-dihydrobenzofuran-3-yl)acetyl-(S)-phenylalanyl-(S)-leucine benzyl ester

(5-(tert-Butoxycarbonylmethylthio)-2,3-dihydrobenzofuran-3-yl)acetyl-(S)-phenylalanyl-(S)-leucine benzyl ester (0.32g) and m-chloroperoxybenzoic acid (80-90%, 0.225g) were stirred in dichloromethane (10 ml) for 1h. The solution was washed with 5% aqueous sodium hydrogen carbonate solution, dried ($Na_2SO_4$) and evaporated, giving the title compound (0.343g) as a white foam.

NMR (δ) ($CDCl_3$) 0.9 (6H, d), 1.4 (9H, s), 1.2-1.7 (m), 2.35-2.5 (1H) and 2.65 ((1H) (2x ABX), 3.05 (2H, m), 3.9 (1H, m), 4.0 (2H, m), 4.2 (0.5H, dd), 4.35 (0.5H, dd), 4.55 (1H, m), 4.6-4.85 (2H, m), 5.15 (2H, ABq), 6.1 (1H, 2xd), 6.4 (1H, t), 6.9 (1H, 2xd), 7.15-7.45 (10H, m), 7.7 (2H, m).

e) (5-(tert-Butoxycarbonylmethanesulphonyl)-2,3-dihydrobenzofuran-3-yl)acetyl-(S)-phenylalanyl-(S)-leucine

(5-(tert-Butoxycarbonylmethanesulphonyl)-2,3-dihydrobenzofuran-3-yl)acetyl-(S)-phenylalanyl-(S)-leucine benzyl ester (0.33g) was hydrogenated over 10% palladium on charcoal (60% aqueous paste, 0.17g) in ethanol (25 ml) for 3.5h. Catalyst was filtered off, and evaporation of solvent afforded the title compound (0.282g) as a light brown foam.

NMR (δ) ($CDCl_3$) 0.9 (6H, m), 1.4 (9H, s), 1.2-1.8 (m), 2.4 (1H, m), 2.7 (1H, m), 2.9-3.15 (2H, m), 3.7-3.9 (m), 4.0 (2H, 2xs), 4.1 (0.5H, dd), 4.2 (0.5H, dd), 4.5 1H, m), 4.7 (1H, t), 4.85 (1H, m), 6.8-6.95 (2H, m), 7.1-7.3 (m), 7.75 (2H, m).

Description 40

a) 3-(Bromomethyl)benzothiophene

3-Methylbenzothiophene (3.1g, 21 mmol) in carbon tetrachloride (100 ml) was refluxed with recrystallised N-bromosuccinimide (3.8g, 21 mmol) and dibenzoyl peroxide (0.05g). After two and a half hours the mixture was filtered and the solvent removed under reduced pressure to give 3-(bromomethyl)-benzothiophene

NMR (δ) ($CCl_4$) 4.7(2H,s), 7.3-8.0(4H,m)

b) 3-(Benzothiophene-3-yl)propanoic acid

3-(Bromomethyl)benzothiophene (4.77g) was added to a solution of the sodium salt of diethyl malonate in ethanol (100 ml) (generated in situ from sodium (0.51g, 22 mmol) and diethylmalonate (3.4g, 21 mmol)). The mixture was stirred for 0.33 h at room temperature, then warmed to 50° for 0.5h. After cooling the ethanol was removed under reduced pressure and the residue was partitioned between dichloromethane and water. The aqueous phase was acidified to pH3 and the organic phase separated and dried over sodium sulphate. Removal of the solvent gave an oil that was dissolved in ethanol (100 ml) and potassium hydroxide (3.9g, 68 mmol) in water (30 ml) was added. The mixture was refluxed with stirring for 4h. After cooling the solvent was concentrated under reduced pressure. The aqueous layer was extracted with ethyl acetate (50 ml) and diethyl ether (50 ml) and acidified to pH1 with 5M HCl. Extraction of the aqueous layer with chloroform (2 x 100 ml) and diethyl ether (2 x 50 ml) and drying the combined organic phases with sodium sulphate gave a dark yellow solution. Concentration of this in vacuo gave a semi-solid that was decarboxylated as in Description (10(b) to give 3-(benzothiophene-3-yl)propanoic acid (2.5g).

NMR (δ) ($DMSOd_6$) 2.7-2.9 (2H,m), 3.1-3.2 (2H,m), 7.4-7.6 (3H,m) 7.9-8.0 (1H,m) 8.0-8.1 (1H,m) 12.4 (1H, bs).

c) 3-(2,3-Dihydro-1,1-dioxobenzothicohene-3-yl) propanoic acid

3-(Benzothiophene-3-yl) propanoic acid (1.2g) was refluxed in ethanol (30 ml) containing concentrated sulphuric acid (1 ml) for 1 h. The mixture was poured into water (200 ml) and diethyl ether (100 ml), neutralised with solid sodium bicarbonate and extracted with ether (2 x 100 ml). The organic phases were dried over sodium sulphate and the solvent removed to give ethyl (3-(benzothiophene-3-yl)propanoate (1.0g). This was taken up in acetic acid (7 ml) and refluxed with 30% hydrogen peroxide solution (3.6 ml) for 0.25h. After cooling the solution was poured into water (30 ml) and extracted with chloroform (3 x 50 ml). The combined organic phases were dried ($Na_2SO_4$) and the solvent removed to give an oil that was hydrogenated in ethyl acetate over 10% palladium on charcoal, at room temperature and pressure, for 72h. After filtration of the catalyst (Kieselguhr) the solvent was removed to give ethyl 3-(2,3-dihydro-1,1-dioxobenzothiophene-3-yl)propanoate (0.24g). This was converted to 3-(2,3-dihydro-1,1-dioxobenzothiophene-3-yl)propanoic acid (0.16g) by the method in Description 8(b).
NMR (δ) ($CDCl_3$) 2.0-2.9(3H,m), 3.1-3.9(4H,m), 7.4-8.0 (4H,m), 10.4(1H,bs). MS (EI) $M^+$ = 240

Description 41

a) Ethyl 4-(4-bromophenylthio)acetoacetate

4-Bromothiophenol (5g) was dissolved in dry pyridine (18 ml). To this was added ethyl 4-chloroacetoacetate (3.65 ml) dropwise with stirring. After 15 minutes the reaction mixture was heated at 100° on an oil bath for 0.25 h and then cooled to room temperature. Ether was added (15 ml), then the pyridine dissolved in 5M hydrochloric acid. The organic layer was set aside and the aqueous layer further extracted with ether (3 x 50 ml). The combined organic phases were washed with water (75 ml), brine (35 ml) and dried over sodium sulphate. Removal of the solvent gave ethyl 4-(4-bromo phenylthio)acetoacetate (7.60g).

b) Ethyl(5-bromobenzothiophene-3-yl)acetate

Ethyl 4-(4-bromophenylthio)acetoacetate (7.60g) was added to stirred excess polyphosphoric acid and stirring continued at 65° for 4h. After cooling, the reaction mixture was poured into water (200 ml) and extracted with ether (3 x 200 ml). The combined ethereal extracts were washed with sodium carbonate solution (2 x 100 ml), dried ($Na_2SO_4$) and the solvent removed under reduced pressure to give ethyl (5-bromobenzothiophene-3-yl)acetate (4g).
NMR (δ) ($CDCl_3$) 1.2-1.3 (3H,t), 3.8 (2H,s), 4.1-4.2 (2H,q), 7.4 (1H,s), 7.5 (1H,dd), 7.7 (1H,d), 7.9 (1H,m).

c) Ethyl(5-cyanobenzothiophene-3-yl)acetate

This material was formed from ethyl(5-bromobenzothiophene-3-yl)acetate (26.42g) and copper (I) cyanide (17.07g) following the procedure of Description 25. This gave ethyl(5-cyanobenzothiophene-3-yl)-acetate (16.63g).
NMR (δ) ($CDCl_3$) 1.2-1.3 (3H,t), 3.8-3.9 (2H,s), 4.1-4.3 (2H,q), 7.5-7.6 (3H,m), 7.9-8.0 (1H,d) 8.1 (1H,m)
IR (neat) $\nu_{max}$ = 2250cm$^{-1}$.

d) Ethyl (5-cyano-1,1-dioxobenzothiophene-3-yl)acetate

Ethyl(5-cyanobenzothiophene-3-yl)acetate (1.52g) was taken up in acetic acid (10 ml) and stirred with 30% hydrogen peroxide solution (3.8 ml) at room temperature for 5 minutes. The mixture was then heated to reflux for 15 minutes, cooled and diluted with water (20 ml). The resulting suspension was extracted with chloroform (5 x 30 ml) and the combined organic phases were dried over sodium sulphate. Removal of the solvent gave ethyl (5-cyano-1,1-dioxobenzothiophene-3-yl)acetate (1.67g).
NMR (δ) ($CDCl_3$) 1.3-1.5 (3H,t), 4.2-4.4 (2H,q), 6.7 (1H,m), 7.8-8.1 (3H,m).

e) (Ethyl (5-cyano-2,3-dihydro-1,1-dioxobenzothiophene-3-yl)acetate

Ethyl (5-cyano-1,1-dioxobenzothiophene-3-yl)acetate (0.499g) was hydrogenated at room temperature and pressure for 48h using ethyl acetate as solvent and 10% palladium on carbon as catalyst. Removal of the catalyst by filtration through Kieselguhr followed by evaporation in vacuo gave the title compound (0.478g).
NMR (δ) ($CDCl_3$) 1.2-1.4 (3H,m), 2.7-3.0 (2H,m), 3.3-3.5 (1H,dd), 3.7-3.8 (1H,dd), 4.0-4.3 (3H,m), 7.8-7.9 (3H,m).

f) (5-Cyano-2,3-dihydro-1,1-dioxobenzothiophene-3-yl)acetic acid

This material was formed from ethyl (5-cyano-2,3-dihydro-1,1-dioxobenzothiophene-3-yl)acetate (0.351g) following the procedure of Description 15(b). Removal of the solvent gave the title product (0.209g).
NMR (δ) ($CD_3OD$) 2.6-2.8 (1H,dd), 2.9-3.0 (1H,dd), 3.4-3.5 (1H,m), 3.7-3.8 (1H,m), 3.9-4.0 (1H,m), 7.7-7.8 (2H,m), 7.9-8.0 (1H,m).

Description 42

(2,3-Dihydro-3-oxo-1H-isoindol-1-ylidine)acetic acid

Ethyl (2,3-dihydro-3-oxo-1H-isoindol-1-ylidineacetate (185 mg) and sodium hydroxide (10% aqueous solution, 0.34 ml) were heated at reflux in ethanol (10 ml) for 4h. The mixture was concentrated under reduced pressure, poured into water (100 ml), and acidified (5M HCl). The precipitated solid was filtered off and dried in vacuo. This gave the title compound (56 mg) as a buff solid, contaminated with ca. 10% of ester starting material.
NMR (δ) ($CD_3OD$) 5.95 (1H,s), 7.7 (2H,m), 7.85 (1H,dd), 7.95 (1H,dd).

Description 43

a) Benzyloxycarbonyl-(S)-β-(3,5-dimethylpyrazol-1-yl)alanine

This material was formed from 3,5-dimethylpyrazole (0.23g) and benzyloxycarbonyl-(S)-serine-β-lactone (0.5g) following the procedure of Description 33. Evaporation under reduced pressure gave benzyloxycarbonyl-(S)-β-(3,5-dimethylpyrazol-1-yl) alanine (0.5g).
NMR (δ) ($CDCl_3$) 1.9-2.4 (6H,m), 4.2-4.8 (2H,m), 5.0-5.2 (2H,m), 5.5-6.0 (2H,m), 7.0-7.5 (6H,m), 11.5 (1H,bs).

b) 4-(S)-(Benzyloxycarbonyl-(S)-β-(3,5-dimethylpyrazol-1-yl)alanyl)amino-5-cyclohexyl-3-(S)-hydroxypentanoic acid isobutylamide

This material was formed from benzyloxycarbonyl-(S)-β-(3,5-dimethylpyrazol-1-yl)alanine (0.5g) and 4-(S)-amino-5-cyclohexyl-3-(S)-hydroxypentanoic acid isobutylamide (0.43g) following the procedure of Description 8(c). Purification by chromatography on silica gel using 0-1% methanol/chloroform gave the title compound (0.48g).
NMR (δ) ($CDCl_3$) 0.8-2.0 (20H,m), 2.0-2.5 (8H,m) 2.9-3.2 (2H,m), 3.5-5.0 (6H,m), 5.2 (2H,bs), 5.7 (1H,bs), 6.5-7.5 (9H,m)

c) 4-(S)-((S)-β-(3,5-Dimethylpyrazol-1-yl)alanyl)amino-5-cyclohexyl-3-(S)-hydroxypentanoic acid isobutylamide dihydrobromide

This material was formed from 4-(S)-(benzyloxycarbonyl-(S)-β-(3,5-dimethylpyrazol-1-yl)alanyl)amino-5-

cyclohexyl-3-(S)-hydroxypentanoic acid isobutylamide (0.24g) and 48% hydrogen bromide in acetic acid (2 ml) as in Description 32. This gave the title compound as an orange solid (0.28g).
NMR (δ) ($CDCl_3$) 0.6-2.0 (20H,m), 2.0-3.4 (9H,m), 3.8-5.9 (8H,m), 6.1-6.3 (1H,bs), 8.0-9.0 (4H,m)

Description 44

a) Nα-Benzyloxycarbonyl-(S)-β-(tert-butoxycarbonylamino)alanine

Di-tert-butyl dicarbonate (0.71g) was added, in one portion, to a solution of Nα-benzyloxycarbonyl-(S)-β-aminoalanine (0.705g) and 1M NaOH (2.96 ml) in dioxan (7 ml) and water (3.5 ml) at 0°. The mixture was stirred at room temperature for 2 h. The solvent was removed under reduced pressure. Ethyl acetate was added and the mixture was acidified to pH2 with dilute sodium hydrogen sulphate. The aqueous layer was further extracted with ethyl acetate and the combined extracts were washed with water and dried over sodium sulphate. Removal of the solvent under reduced pressure gave Nα-benzyloxycarbonyl-(S)-β-(tert-butoxycarbonylamino)alanine (0.6g).
NMR (δ) ($CDCl_3$) 1.5 (9H,s), 3.5-5.3 (5H,m), 7.3 (5H,s)

b) 4-(S)-(Nα-benzyloxycarbonyl)-(S)-β-(tert-butoxycarbonylamino)alanyl)amino-5-cyclohexyl-3-(S)-hydroxypentanoic acid isobutylamide

Nα-Benzyloxycarbonyl-(S)-β-(tert-butoxycarbonylamino)alanine (0.2g) and 4-(S)-amino-5-cyclohexyl-3-(S)-hydroxy-pentanoic acid isobutylamide (0.16g) were reacted following the procedure of Description 8(c). Purification by chromatography on silica gel using 0-2% methanol in chloroform gave the title compound (0.20g).
NMR (δ) ($CDCl_3$) 0.8-2.6 (32H,m), 2.8-3.7 (5H,m), 3.7-4.7 (4H,m) 5.0-5.5 (4H,m), 6.4-7.5 (8H,m)

c) 4-(S)-(S)-β-(tert-Butoxycarbonylamino)alanyl)amino-5-cyclohexyl-3-(S)-hydroxypentanoic acid isobutylamide

4-(S)-(Nα-Benzyloxycarbonyl)-(S)-β-(tert-butoxycarbonylamino)alanyl)amino-5-cyclohexyl-3-(S)-hydroxypentanoic acid isobutylamide was hydrogenated for 4 h in ethanol using 10% palladium on charcoal as catalyst. After removal of the catalyst by filtration through kieselguhr the solvent was removed to give the title compound.
NMR (δ) ($CDCl_3$) 0.8-1.0 (7H,m), 1.4-1.5 (9H,s), 2.3-2.5 (2H,m), 2.9-3.2 (2H,m), 3.9-4.1 (2H,m), 6.0-6.2 (1H,bs), 7.0-7.2 (1H,bs), 8.0-8.1 (1H,bs), 7.0-7.2 (1H,bs)
M.S. (m/z) (FAB) (M+1) = 457 (consistent with mw = 456)

Description 45

a) 2-Methyl benzothiophene

Benzothiophene (3.77g) was taken up in freshly distilled THF under nitrogen at -30°C. N-Butyllithium (1.6M, 17.50ml) was added at such a rate as to keep the temperature below -20°C. The resulting suspension was stirred for 30 minutes. Iodomethane (1.3ml) was added in one portion and the reaction stirred overnight. The solvents were removed and the oil that resulted taken up in dichloromethane. After washing with water (100ml) the organic phase was dried ($Na_2SO_4$) and the solvents removed to afford the title compound as an oil (3.46g) which crystallised on standing.
NMR (δ) ($CDCl_3$) 2.4-2.6 (3H, bs), 6.7-6.9 (1H, bs), 7.0-7.4 (2H, m), 7.4-7.8 (2H, m)

b) 3-(Benzothiophene-2-yl)propanoic acid

This material was formed from 2-methyl benzothiophene (4.66g) following the procedures in Description 40(a) and (b). Decarboxylation gave 3-(benzothiophene-2-yl)propanoic acid (0.6g).
NMR (δ) ($CD_3OD$) 2.7-2.8 (2H, m), 3.1-3.3 (2H, m), 7.0-7.1 (1H, bs), 7.2-7.3 (2H, m), 7.6-7.8 (2H, m).

(c) 3-(2,3-Dihydro-1,1-dioxobenzothiophene-2-yl) propanoic acid

This material was formed from 3-(benzothiophene-2-yl) propanoic acid (0.6g) following the procedure of Description 40(c). The title compound was isolated as a white solid (0.09g).
NMR (δ) ($CD_3OD$) 2.0-2.4 (2H, m), 2.5-2.7 (2H, m), 2.9-3.1 (1H, m), 3.4-3.7 (2H, m), 7.3-7.5 (2H, m), 7.5-7.7 (2H, m).

Description 46

a) Nα-Benzyloxycarbonyl-(S)-β-(tert-butoxycarbonylamino-(S)-alanine methyl ester

To a mixture of 50% aqueous potassium hydroxide solutien (30ml) and diethyl ether (100ml) at 0°C was added N-nitrosomethylurea (8g) portionwise with occasional swirling. When all the N-nitrosomethylurea had dissolved the ether layer was decanted onto potassium hydroxide pellets. This solution was added dropwise to a stirred solution of Nα-benzyloxycarbonyl-(S)-β-(tert-butoxycarbonylamino)alanine (6.2g) in dry dichloromethane (20ml) until a permanent yellow colouration was obtained. After 15 minutes glacial acetic acid (1ml) was added and the resultant mixture evaporated *in vacuo*. Chromatography on silica gel using 0-30% ethyl acetate/pentane afforded the title compound (5.71g).
NMR (δ) ($CDCl_3$) 1.4 (9H, s), 3.3-3.9 (5H, m), 4.1-4.6 (1H, bm), 5.1 (2H, s), 5.8-6.1 (1H, bm), 7.3 (5H, s), 8.1 (1H, bm).

b) Nα-Benzyloxycarbonyl-(S)-β-aminoalanine methyl ester trifluoroacetate

The material was formed from Nα-benzyloxycarbonyl-(S)-β- (tert-butoxycarbonylamino)alanine methyl ester (0.5g) and trifluoroacetic acid (5ml) as described in Description 30. Trituration of the crude product with diethyl ether afforded the title compound as a white solid (0.49g).
NMR (δ) ($CDCl_3$), 2.9-3.3 (2H, m), 3.7 (3H, s), 4.2-4.5 (1H, m), 5.1 (2H, s), 7.3 (5H, s), 7.5-8.3 (3H, bm).

c) Nα-Benzyloxycarbonyl-(S)-β-(N-acetylamino)alanine methyl ester

Acetyl chloride (0.1ml) in dry dichloromethane (2ml) was added dropwise to a stirred solution of Nα-benzyloxycarbonyl-(S)-β-aminoalanine methyl ester trifluoroacetate (0.49g) and triethylamine (0.37ml) in dry dichloromethane (5ml) at 0°C. After stirring at 0°C for 2 h, dichloromethane (50ml) was added and the mixture washed with 10% aqueous citric acid (2 x 15ml), saturated sodium hydrogen carbonate (30ml) and brine (30ml). Chromatography on silica gel using 0-2% methanol/chloroform afforded the title compound (0.24g).
NMR (δ)($CDCl_3$) 1.95 (3H, s), 3.6-3.7 (2H, t), 3.75 (3H, s), 4.4 (1H, m), 5.1 (2H, s), 5.7-6.0 (2H, m), 7.35 (5H, m)
M.S. (m/z) (FAB) (M+1) = 295 (consistent with m.w. = 294)
Analysis: $C_{14}H_{18}N_2O_5$ requires C, 57.1; H, 6.2; N, 9.5%. Found: C, 57.1; H, 6.3; N 9.5%.

d) Nα-Benzyloxycarbonyl-(S)-β-(acetylamino)alanine

This material was prepared from Nα-benzyloxycarbonyl-S)-β-(acetylamino)alanine methyl ester (0.24g) following the procedure described in Description 2(b). This gave Nα-benzyloxycarbonyl-(S)-β-(acetylamino)-alanine as a white solid (0.137g).
NMR (δ) ($CDCl_3$) 1.9 (3H, s), 3.6 (2H, bs), 4.25-4.45 (1H, bm), 5.1 (2H, s), 6.25 (1H, d), 6.8 (1H, bs), 7.3 (5H, s), 8.8 (1H, bs)

M.S. (m/z) (E.I.) $M^+$ = 280.

e) 4-(S)-(Nα-Benzyloxycarbonyl-(S)-β-(N-acetylamino)alanyl)amino-5-cyclohexyl-3-(S)-hydroxypentanoic acid isobutylamide

This material was prepared from Nα-benzyloxycarbonyl-(S)-β-(acetylamino)alanine (0.45g) and 4-(S)-amino-5-cyclohexyl-3-(S)-hydroxypentanoic acid isobutylamide (0.435g) following the procedure of Description 2(c). Chromatography on silica gel using 0-3% methanol/chloroform afforded the title compound (0.52g).
NMR (δ) ($CD_3OD$), 0.7-1.8 (20H, m), 1.9 (3H, s), 2.3 (2H, m), 3.0 (2H, m), 3.4-3.6 (2H, m), 3.9 (2H, m), 4.25 (1H, m), 5.1 (2H, s), 7.2 - 7.4 (5H, m).
M.S. (m/z) (FAB) (M+1) = 533 (consistent with m.w. = 532)

Description 47

a) Nα-Benzyloxycarbonyl-(S)-β-(4-(chlorobutanoylamino)alanine methyl ester

This material was prepared from Nα-benzyloxycarbonyl-(S)-β-aminoalanine methyl ester trifluoroacetate (0.98g) and 4-butanoyl chloride (0.30ml) following the procedure of Description 46(c). This gave the title compound as a colourless oil (0.91g, 95%).
NMR (δ) ($CDCl_3$) 1.7-2.5 (4H, m), 3.3-3.8 (7H, m), 4.1-4.5 (1H, m), 5.0 (2H, s), 5.8-6.1 (1H, m), 6.1-6.4 (1H, m) 7.25 (5H, s).
M.S. (m/z) (E.I.) ($M^+$) = 356, $(M\text{-}HCl)^+$ = 320.
$C_{16}H_{21}N_2O_5Cl$ requires $M^+$ 356.1139. Found: 356.1139

b) N-Benzyloxycarbonyl-(S)-β-(2-oxopyrrolidin-1-yl)alanine methyl ester

To a solution of Nα-benzyloxycarbonyl-(S)-β-(4- chlorobutanoylamino)alanine methyl ester (0.91g) in dry dimethylformamide (10ml) at 0°C was added sodium hydride (0.078g of 80% dispersion in mineral oil) portionwise over 0.2 h. Cooling was removed and the reaction mixture stirred at room temperature for 18 h and at 40°C for a further 30 h. Water was added and the mixture extracted with ethyl acetate (2 x 100ml). Combined organics were washed with brine, dried ($Na_2SO_4$) and the solvent removed in vacuo to afford the title compound (0.6g).
NMR (δ) ($CDCl_3$) 2.7-3.5 (4H, m), 3.1-3.8 (7H, m), 4.2-4.7 (1H, m), 5.1 (2H, s), 5.8-6.2 (1H, m), 7.3 (5H, s)
M.S. (m/z) (FAB) (M+1) = 321 (consistent with m.w. = 320).

c) N-Benzyloxycarbonyl-(S)-β-(2-oxopyrrolidin-1-yl) alanine

This material was prepared from N-benzyloxycarbonyl-(S)-β-(2-oxopyrrolidin-1-yl)alanine methyl ester (0.6g) following the procedure of Description 2(b). This gave the title compound (0.322g).
NMR (δ) ($DMSOd_6$) 1.85-2.05 (2H, m), 2.1-2.3 (2H, t), 3.7 (1H, dd), 4.3 (1H, m), 5.1 (2H, m), 7.4 (5H, m), 7.7 (1H, d)
M.S. (m/z) (FAB) (M+1) = 307 (consistent with m.w. = 306).

d) 4-(S)-(N-Benzyloxycarbonyl-(S)-β-(2-oxopyrrolidin-1-yl)alanyl)amino-5-cyclohexyl-3-(S)-hydroxypentanoic acid isobutylamide

This material was prepared from N-benzyloxycarbonyl-(S)-β-(2-oxopyrrolidin-1-yl)alanine (0.44g) and 4-(S)-amino-5-cyclohexyl-3-(S)-hydroxypentanoic acid isobutylamide (0.39g) following the procedure of Description 2(a). This gave the title compound as a white foam (0.575g).
NMR (δ) ($CDCl_3$) 0.8-2.5 (26H, m), 2.9-4.1 (9H, m) 4.2-4.5 (1H, m), 5.0-5.2 (2H, m), 6.4-6.95 (3H, m), 7.35 (5H, s).

e) 4-(S)-((S)-$\beta$-(2-Oxopyrrolidin-1-yl)alanyl)amino-5-cyclohexyl-3-(S)-hydroxypentanoic acid isobutylamide

A solution of 4-(S)-(N-benzyloxycarbonyl-(S)-$\beta$-(2-oxopyrrolidin-1-yl)alanyl)amino-5-cyclohexyl-3-(S)-hydroxypentanoic acid isobutylamide (0.4g) in ethanol (25ml) was hydrogenated in the presence of 10% palladium on charcoal for 4 h. Filtration of the catalyst and removal of the solvent in vacuo afforded the title compound (0.295g).
NMR ($\delta$) ($CDCl_3$) 0.8-2.0 (18H, m), 2.0-2.2 (2H, m), 2.3-2.6 (4H, m), 2.9-3.2 (2H, m), 3.4-4.7 (10H, m).
M.S. $C_{22}H_{40}N_4O_4$ requires $M^+$ 424.3050. Found 424.3048.

Description 48

a) (5-Cyano-2,3-dihydrobenzofuran-3-yl)acetyl-(S)-phenylalanyl-(S)-histidine methyl ester

This material was formed from (5-cyano-2,3-dihydrobenzofuran-3-yl)acetic acid (3.5g) and (S)-phenylalanyl-(S)-histidine methyl ester trifluoroacetate (9.2g) following the procedure of Description 8(c). The crude product was chromatographed on silica gel using 0-4% methanol/chloroform as eluent. This gave (5-cyano-2,3-dihydrobenzofuran-3-yl)acetyl-(S)-phenylalanyl-(S)-histidine methyl ester as a light green foam (3.7g).
M.S. (m/z) (FAB) (M + 1) = 502 (consistent with m.w. = 501).

b) (5-Cyano-2,3-dihydrobenzofuran-3-yl)acetyl-(S)-phenylalanyl-(S)-histidine

This material was formed from (5-cyano-2,3-dihydrobenzofuran-3-yl)acetyl-(S)-phenylalanyl-(S)-histidine methyl ester (3.7g) following the procedure of Description 2(b). This gave (5-cyano-2,3-dihydrobenzofuran-3-yl)acetyl-(S)-phenylalanyl-(S)-histidine (1.37g)
NMR ($\delta$) ($CDCl_3$) 2.2-2.6 (2H, m), 2.65-2.8 (1H, m), 2.95-3.15 (3H, m), 4.1-4.6 (5H, m), 6.7-7.4 (9H, m), 7.9 (1H, s).

Description 49

a) Ethyl (6-(tert-butoxycarbonylaminomethyl)-2,3-dihydro-1,1-dioxobenzothiophene-3-yl)acetate

Ethyl (6-cyano-2,3-dihydro-1,1-dioxobenzothiophene-3-yl)acetate (1.63g) and cobalt (II) chloride hexahydrate (2.78g) were stirred in methanol (30ml). Sodium borohydride (0.67g) was added portionwise, and the black suspension so formed was stirred for 1 h, diluted with water (200ml), acidified (5M hydrochloric acid), and then basified with excess aqueous ammonia. This solution was extracted with chloroform, and the extract was dried ($Na_2SO_4$) and evaporated to a brown oil (1.78g). This was dissolved in dichloromethane (30ml), and di-tert-butyl dicarbonate (1.27g) and triethylamine (0.81ml) were added. The solution was stirred for 16 h, diluted with chloroform (50ml), washed with 0.5M hydrochloric acid, dried ($Na_2SO_4$) and evaporated. This crude product was purified by chromatography on silica gel using chloroform. This gave the title compound (1.42g) as a colourless gum.
NMR ($\delta$) ($CDCl_3$) 1.3 (3H, t), 1.45 (9H, s), 2.75 (1H) and 2.95 (1H) (ABX), 3.35 (1H) and 3.75 (1H) (ABX), 4.0 (1H, m), 4.2 (2H, g), 4.4 (2H, d), 5.0 (1H, b), 7.4 (1H, d), 7.55 (1H, d), 7.65 (1H, s).

b) (6-(tert-Butoxycarbonylaminomethyl)-2,3-dihydro-1,1-dioxobenzothiophene-3-yl)acetic acid

This material was prepared from ethyl (6-(tert-butoxycarbonylaminomethyl)-2,3-dihydro-1,1-dioxobenzothiophene-3-yl)acetate (1.37g), following the procedure of Description 8(b). This gave the title compound (1.25g) as a white foam.
NMR ($\delta$) ($CDCl_3$) 1.5 (9H, s), 2.8-3.05 (2H, ABX), 3.4 (1H, dd), 3.75 (1H, dd), 4.0 (1H, b), 4.4 (2H, bd), 5.0 (b), 7.4 (1H, d), 7.55 (1H, d), 7.65 (1H, s).

Description 50

a) Ethyl (5-(2-phthalimidoethylthio)-2,3-dihydrobenzofuran-3-yl)acetate

This material was formed from ethyl (5-mercapto-2,3-dihydrobenzofuran-3-yl)acetate (1.07g) and N-(2-bromoethyl)phthalimide (1.14g), following the procedure of Description 39(a). This gave the title compound (1.68g) as a light green syrup.
NMR (δ) ($CDCl_3$) 1.3 (3H, t), 2.55 (1H) and 2.8 (1H) ABX), 3.1 (2H, dt), 3.8 (1H, m), 3.9 (2H, t), 4.1-4.3 (3H, m), 4.7 (1H, t), 6.7 (1H, d), 7.25 (2H, d), 7.7 (2H, m), 7.8 (2H, m).

b) Ethyl (5-(2-phthalimidoethanesulphonyl)-2,3-dihydrobenzofuran-3-yl)acetate

This material was formed from ethyl (5-(2-phthalimidoethylthio)-2,3-dihydrobenzofuran-3-yl)acetate (1.64g), following the procedure of Description 16(d). This gave the title compound (1.33g) as a light brown gum.
NMR (δ) ($CDCl_3$) 1.3 (3H, t), 2.6 (1H) and 2.85 (1H) (ABX), 3.35-3.55 (2H, m), 3.85 (1H, m), 4.05 (2H, m), 4.2 (2H, m), 4.35 (1H, dd), 4.8 (1H, t), 6.8 (1H, d), 7.6-7.9 (6H, m).

c) Ethyl (5-(2-aminoethanesulphonyl)-2,3-dihydrobenzofuran-3-yl)acetate hydrochloride

Ethyl (5-(2-phthalimidoethanesulphonyl)-2,3-dihydrobenzofuran-3-yl)acetate (1.16g) and hydrazine hydrate (0.25ml) were stirred in methanol (25ml) for 16 h. The mixture was evaporated to dryness, and 0.5M hydrochloric acid (50ml) was added. The solid so formed was filtered off, and the filtrate was basified with 10% aqueous sodium carbonate solution, and extracted with chloroform and ethyl acetate. The combined extracts were dried ($Na_2SO_4$) and evaporated to an oil. This was dissolved in ethanol (30ml), and 5M hydrochloric acid (1ml) was added. Evaporation then gave the title compound (0.80g) as a gum.
NMR (δ) ($D_2O$) 1.2 (3H, t), 2.8 (2H, ABX), 3.35 (2H, t), 3.65 (2H, t), 3.95 (1H, m), 4.1 (2H, g), 4.5 (1H, dd), 4.9 (1H, t), 7.0 (1H, d), 7.8 (2H, m).

d) Ethyl (5-(2-(benzyloxycarbonylamino)ethanesulphonyl)-2,3-dihydrobenzofuran-3-yl)acetate

Ethyl (5-(2-aminoethanesulphonyl)-2,3-dihydrobenzofuran-3-yl)acetate hydrochloride (0.85g), triethylamine (0.74ml) and benzyl chloroformate (0.38ml) were stirred in dichloromethane (20ml) for 3 h, and left to stand for 64 h. The solution was diluted with chloroform (50ml), washed with 0.5M hydrochloric acid, dried ($Na_2SO_4$) and evaporated. The crude product was chromatographed on silica using chloroform. This gave the title compound (0.84g) as a light yellow oil.
NMR (δ) ($CDCl_3$) 1.3 (3H, t), 2.6 (1H) and 2.8 (1H) (ABX), 3.3 (2H, m), 3.6 (2H, m), 3.9 (1H, m), 4.2 (2H, q), 4.4 (1H, dd), 4.9 (1H, t), 5.05 (2H, s), 5.5 (1H, bt), 6.9 (1H, d), 7.3-7.4 (5H, m), 7.6-7.8 (2H, m).

e) (5-(2-(Benzyloxycarbonylamino)ethanesulphonyl)-2,3-dihydrobenzofuran-3-yl)acetic acid

This material was formed from ethyl (5-(2-(benzyloxycarbonylamino)ethanesulphonyl)-2,3-dihydrobenzofuran-3-yl)acetate (0.80g), following the procedure of Description 8(b). This gave the title compound (0.64g) as a white foam.
NMR (δ) ($CDCl_3$) 2.75 (2H, m), 3.3 (2H, m), 3.6 (2H, m), 3.9 (1H, m), 4.4 (1H, dd), 4.9 (1H, t), 5.1 (2H, s), 5.5 (1H, m), 6.9 (1H, d), 7.2-7.4 (5H, m), 7.7 (2H, m).

Description 51

a) Ethyl 2,3-dihydro-2-oxo-1H-benzimidazole-1-acetate

2-Hydroxybenzimidazole (2.05g), anhydrous potassium carbonate (3.17g), and ethyl bromoacetate (1.7ml) were stirred in dry dimethylformamide (25ml) for 64 h. Ethyl acetate (200ml) was added. and the mixture was washed with water and brine, dried ($Na_2SO_4$) and evaporated, giving a white solid (2.74g). Chromatography on silica gel using 2% methanol/chloroform gave pure dialkylated product (1.00g), and fractions containing mixtures of mono- and di-alkylated products. Repeated recrystallisation of the latter (chloroform/petroleum ether (bp. 60⁻80°C)) gave the title compound (0.36g) as white needles, m.p. 176.5-177.5°C.
NMR (δ) ($DMSOd_6$) 1.2 (3H, t), 4.15 (2H, g), 4.6 (2H, s), 6.95 (3H, m), 7.05 (1H, m), 10.95 (1H, b).

b) 2,3-Dihydro-2-oxo-1H-benzimidazole-1-acetic acid

This material was formed from ethyl 2,3-dihydro-2-oxo-1H-benzimidazole-1-acetate (0.36g), following the procedure of Description 8(b). This gave the title compound (0.23g) as a white powder.
NMR (δ) ($DMSOd_6$) 4.5 (2H, s), 6.95 (3H, m), 7.05 (1H, m), 10.9 (1H, s), 13.0 (1H, bs).

Description 52

a) Ethyl 2,3-dihydro-2-oxo-3-H-benzoxazole-3-acetate

This material was formed from 2(3H)-benzoxazolinone 2.09g), following the procedure of Description 51-(a). Recrystallisation from ethyl acetate/petroleum ether (bp 60-80°C) gave the title compound (2.84g) as straw-coloured needles.
NMR (δ) ($CDCl_3$) 1.3 (3H, t), 4.25 (2H, q), 4.55 (2H, s), 6.9 (1H, dd), 7.1-7.3 (3H, m).

b) 2,3-Dihydro-2-oxo-3-H-benzoxazole-3-acetic acid

This material was formed from ethyl 2,3-dihydro-2-oxo-3H-benzoxazole-3-acetate), following the procedure of Description 18(b). This gave the title compound (0.46g) as a grey powder.
NMS (δ) ($CD_3OD$) 4.6 (2H, s), 7.1-7.3 (4H, m).

Description 53

a) Ethyl (2,3-dihydro-1,1-dioxobenzothiophene-3-yl)acetate

This material was formed from (2,3-dihydro-1,1-dioxobenzothiophene-3-yl)acetic acid (1.99g) following the procedure of Description 22. This gave the title compound (2.24g) as a colourless oil.
NMR (δ) ($CDCl_3$) 1.3 (3H,t), 2.7-3.0 (2H,m), 3.4 1H,dd), 3.7-3.8 (1H,m), 4.0-4.1 (1H,m), 4.2 (2H,q), 7.4-7.8 (4H,m).

b) (2,3-Dihydro-1,1-dioxobenzothiophene-3-yl)acetaldehyde

Ethyl (2,3-dihydro-1,1-dioxobenzothiophene-3-yl)acetate (2.24g) was stirred in toluene (40 ml) under nitrogen at -78°. Diisobutylaluminium hydride (1.5M in toluene, 11.7 ml) was added dropwise maintaining the temperature below -65°. Stirring was continued for $\frac{1}{2}$ h at this temperature; methanol (6 ml) and saturated potassium sodium tartrate solution (12 ml) were then added dropwise. The solution was allowed to warm to room temperature, diluted with ether (100 ml) and filtered through Kieselguhr. Evaporation then gave the title product (1.80g).
NMR (δ) ($CDCl_3$) 3.1-3.3 (3H,m), 3.7-3.8 (1H,m), 4.1-4.2 (1H,m), 7.4 (1H,d), 7.5-7.6 (2H,m), 7.7 (1H,d) and 9.8 (1H,s).

c) Ethyl 4-(2,3-dihydro-1,1-dioxobenzothiophene-3-yl)but-2-enoate

(2,3-Dihydro-1,1-dioxobenzothiophene-3-yl)acetaldehyde (1.80g) in dry dichloromethane (15 ml) was added to a solution of (carbethoxymethylene)triphenylphosphorane (3.71g) in dichloromethane (15 ml). Stirring was continued overnight under nitrogen. The solvent was removed under reduced pressure and the residues triturated with ether. Filtration, evaporation of the filtrate and purification by column chromatography using chloroform-methanol gave the title compound (0.50g).
NMR (δ) ($CDCl_3$) 1.3 (3H,t), 2.6-2.9 (1H,m), 3.2 (1H,dd), 3.6 (1H,dd), 3.6 (1H,m), 3.7-3.8 (1H,m), 4.2 (2H,g), 5.9-6.0 (1H,d), 6.8-7.0 (1H,m), 7.4-7.7 (4H,m).

d) Ethyl 4-(2,3-dihydro-1,1-dioxobenzothiophene-3-yl)butanoate

This material was formed from ethyl 4-(2,3-dihydro-1,1-dioxobenzothiophene-3-yl)but-2-enoate (0.50g) following the procedure of Description 23. This gave the title compound (0.50g) as an orange oil.
NMR (δ) ($CDCl_3$) 1.4 (3H,t), 1.7-1.8 (3H,m), 2.0-2.1 1H,m), 2.4 (2H,m), 3.2 (1H,d), 3.6-3.7 (2H,m), 4.1-4.2 (2H,q), 7.4-7.8 (4H,m).

e) 4-(2,3-Dihydro-1,1-dioxobenzothiophene-3-yl)butanoic acid

This material was formed from ethyl 4-(2,3-dihydro-1,1-dioxobenzothiophene-3-yl)butanoate (0.50g) following the procedure of Description 26. This gave the title compound (0.39g) as a white solid.
NMR (δ) ($CD_3OD$) 1.7-1.8 (3H,m), 2.0 (1H,m), 2.4-2.5 (2H,m), 3.7-3.8 (2H,m), 3.9 (1H,m), 7.4-7.6 (2H,m), 7.6-7.7 (2H,m).

Description 54

a) Methyl 3-(2,3-dihydro-2-oxo-3H-benzoxazol-3-yl)propanoate

This material was formed from 2(3H)-benzoxazolinone (1.97g) and methyl acrylate (1.31 ml), following the procedure of Description 51(a). This gave the title compound (2.44g) as a dark brown oil.
NMR (δ) ($CDCl_3$) 2.85 (2H,t), 3.7 (3H,s), 4.15 (2H,t), 7.05-7.25 (4H,m).

b) 3-(2,3-Dihydro-2-oxo-3H-benzoxazol-3-yl)propanoic acid

This material was formed from methyl 3-(2,3-dihydro-2-oxo-3H-benzoxazol-3-yl)propanoate (2.42g), following the procedure of Description 15(b). Recrystallisation from ethyl acetate/petroleum ether (b.p. 60-80°C) then gave the title compound (0.91g) as a red-brown powder.
NMR (δ) ($CDCl_3$) 2.9 (2H,t), 4.1 (2H,t), 7.05-7.25 (4H,m).

Description 55

5-Cyano-2,3-dihydro-2-oxo-1H-indole-3-acetic acid

To a stirred mixture of 5-cyanoisatin * (1.01g) in dry dichloromethane (30 ml) was added (carbobenzyloxymethylene)triphenylphosphorane (2.41g). The resultant orange solution was stirred at room temperature for 20h. Evaporation to dryness and chromatography on silica gel using 0-1% methanol/chloroform afforded an orange solid (1.8g) which was dissolved in ethanol 100 ml) and hydrogenated at room temperature and atmospheric pressure in the presence of 10% palladium on carbon for 5h. Catalyst was removed by filtration and the filtrate evaporated in vacuo. The residue was dissolved in ethyl

*See J. Org. Chem. 42 (8), 1344-8 (1977).

acetate and extracted with saturated sodium hydrogen carbonate (3x100 ml). The combined extracts were acidified with solid citric acid and extracted with ethyl acetate (3x100 ml). The combined organic extracts were washed with saturated brine, dried ($Na_2SO_4$) and evaporated to dryness in vacuo to afford the title compound (0.32g) as a light yellow solid.
NMR (δ) (DMSOd$_6$) 2.8-3.15 (2H,m), 3.7 (1H,m), 6.9 (1H,d), 7.6 (1H,d), 7.65 (1H,s), 10.85 (1H,s), 12.4 (1H,s).

Description 56

a) (5-Cyano-2,3-dihydrobenzofuran-3-yl)acetyl-(S)-phenylalanine methyl ester

This material was formed from (5-cyano-2,3-dihydrobenzofuran-3-yl)acetic acid (1.66g) and (S)-phenylalanine methyl ester hydrochloride (1.5g) following the procedure of Description 2(a). This gave the title compound (2.33g) contaminated with approximately 10% dicyclohexylurea.
NMR (δ) ($CDCl_3$) 2.4-2.7 (2H,m), 3.0-3.3 (2H,m), 3.75 3H,d), 3.8-4.0 (1H,m), 4.2-4.4 (1H,m), 4.7-5.0 (2H,m), 5.9 (1H,bd),d 6.8-7.5 (8H,m).

b) (5-Aminomethyl-2,3-dihydrobenzofuran-3-yl)acetyl-(S)-phenylalanine methyl ester

To a stirred solution of (5-cyano-2,3-dihydrobenzofuran-3-yl)acetyl-(S)-phenylalanine methyl ester (2.33g) in methanol (80 ml) was added cobalt (II) chloride hexahydrate (3.04g), followed by sodium borohydride (1.21g) portionwise. After complete borohydride addition the reaction mixture was stirred for 1h. The mixture was concentrated to approximately 50 ml, poured into water and extracted into chloroform (4x100 ml). Combined chloroform extracts were extracted with 2N HCl. The acid layer was basified with solid sodium hydrogen carbonate and extracted with chloroform (3x100 ml). These extracts were combined, washed with brine, dried ($Na_2SO_4$) and evaporated to dryness in vacuo to afford the title compound (1.08g).
NMR (δ) ($CDCl_3$) 2.3-2,7 (2H,m), 3.0-3.2 (2H,m), 3.6-3.9 (6H,m), 4.1-4.3 (1H,m), 4.6-4.7 (1H,m), 4.85-5.0 (1H,m), 5.95 (1H,bd), 6.7 (1H,m), 7.0-7.4 (9H, m). M.S. (m/z) (E.I.) ($M^+$) = 368. H.R.M.S. (m/z) $C_{21}H_{24}N_2O_4$ requires 368.1736. Found: 368.1727.

c) (5-(tert-Butoxycarbonylaminomethyl)-2,3-dihydrobenzofuran-3-yl)acetyl-(S)-phenylalanine methyl ester

Di-tert-butyldicarbonate (0.66g) was added in one portion to a stirred solution of (5-aminomethyl-2,3-dihydrobenzofuran-3-yl)acetyl-(S)-phenylalanine methyl ester (1.02g) in chloroform (25 ml) at 0°C. After 18h at room temperature the reaction mixture was diluted with chloroform and washed with water (3x50 ml), 10% citric acid and brine. The organics were dried ($Na_2SO_4$) and the solvent removed in vacuo to afford the title compound (1.28g).
NMR (δ) ($CDCl_3$) 1.45 (9H,s), 2.35-2.65 (2H,m), 3.0-3.2 2H,m), 3.7-3.95 (4H,m including d at 3.75), 4.1-4.3 (3H,m), 4.6-5.0 (3H,m), 5.8-5.9 (1H,m), 6.7 (1H,m), 7.0-7.4 (8H,m).
H.R.M.S. (m/z) $C_{26}H_{30}N_2O_6$ requires 468.2261. Found: 468.2266.

d) (5-(tert-Butoxycarbonylaminomethyl)-2,3-dihydrobenzofuran-3-yl)acetyl-(S)-phenylalanine

This material was formed from (5-(tert-butoxycarbonylaminomethyl)-2,3-dihydrobenzofuran-3-yl)acetyl-(S)-phenylalanine methyl ester (1.1g) following the procedure of Description 2(b). This gave the title compound (0.28g).
NMR (δ) ($CDCl_3$) 1.50 (9H,s), 2.3-2.65 (2H,m), 2.9-3.3 (2H,m), 3.7-4.3 (4H,m), 4.5-5.0 (2H,m), 6.6-7.3 (9H,m).

e) 4-(S)-((5-(tert-Butoxycarbonylaminomethyl)-2,3-dihydrobenzofuran-3-yl)acetyl-(S)-phenylalanyl-(S)-β-(pyrazol-1-yl)alanyl)amino-5-cyclohexyl-3-(S)-hydroxypentanoic acid isobutylamide hydrate

This material was formed from (5-(tert-butoxycarbonylaminomethyl)-2,3-dihydrobenzofuran-3-yl)acetyl-(S)-phenylalanine (0.24g) and 4-(S)-((S)-β-(pyrazol-1-yl)alanyl)amino-5-cyclohexyl-3-(S)-hydroxypentanoic

acid isobutylamide dihydrobromide (0.3g) following the procedure of Description 8(c). This gave the title compound (0.25g).
NMR (δ) ($CD_3OD$) 0.7-2.0 (29H, m), 2.15-2.8 (4H,m), 2.85-3.10 (4H,m), 3.1-3.25 (1H,m), 3.5-4.2 (6H,m), 4.3-4.8 (5H,m), 6.3 (1H,m), 6.6 (1H,m), 6.9-7.1 (2H,m), 7.2 (H,m), 7.5 (1H,m), 7.6 (1H,m).
Analysis: $C_{46}H_{65}N_7O_8.H_2O$ requires C,64.1; H,7.8; N,11.4%. Found: C,63.9; H,7.6; N,11.1%.
M.S (m/z) (F.A.B) (M+1) = 844 (consistent with m.w. = 843).

## Description 57

### a) (5-Formyl-2,3-dihydrobenzofuran-3-yl)acetyl-(S)-phenylalanyl-(S)-histidine methyl ester

This material was formed from (5-formyl-2,3-dihydrobenzofuran-3-yl)acetic acid (1.82g) and (S)-phenylalanyl-(S)-histidine methyl ester bis(trifluoroacetate) (4.3g) following the procedure of Description 8(c) but omitting the citric acid wash. This gave the title compound (1.87g).
NMR (δ) ($CDCl_3$) 2.2-3.4 (6H,m), 3.6-4.0 (4H,m), 4.0-5.0 (4H,m), 6.6 (1H,m), 6.8-6.9 (1H,m), 7.3 (7H,m), 7.5-7.8 (3H,m).

### b) (5-Formyl-2,3-dihydrobenzofuran-3-yl)acetyl-(S)-phenylalanyl-(S)-histidine

To a stirred solution of (5-formyl-2,3-dihydrobenzofuran-3-yl)acetyl-(S)-phenylalanyl-(S)-histidine methyl ester (1.8g) in methanol at 0°C was added 10% aqueous sodium hydroxide (1.4 ml). After 18h at room temperature the reaction mixture was concentrated in vacuo and the residue taken up in water. The solution was washed with ethyl acetate and the pH of the aqueous layer adjusted to pH 7 with 5N HCl. Freeze-drying afforded a white solid which was triturated with methanol. Filtration and evaporation of the filtrate in vacuo afforded the title acid (1.45g).
NMR (δ) ($CD_3OD$) 2.3-2.9 (3H,m), 3.0-3.3 (3H,m), 3.7-3.9 (1H,m), 4.0-4.7 (4H,m), 6.8-6.9 (2H,m), 7.3 (6H,m), 7.7 (4H,m).

## Description 58

### a) 4-(tert-Butoxycarbonylamino)pyrazole

A solution of 4-nitropyrazole (0.94g) in ethanol was hydrogenated at room temperature and pressure in the presence of 10% palladium on carbon for 3h. Filtration and evaporation of the filtrate afforded a residue that was dissolved in chloroform (200 ml) and triethylamine (0.84g), cooled in ice, and treated with di-tert-butyl dicarbonate (1.81g). After stirring at room temperature for 18h, the reaction mixture was washed with water (3x100 ml) and brine (100 ml). The chloroform layer was dried ($Na_2SO_4$), filtered and the filtrate evaporated in vacuo to afford the title compound (0.36g). A further 1.1g was obtained by extraction of the aqueous portions with ethyl acetate.
NMR (δ) ($CDCl_3/CD_3OD$) 1.5 (9H,s), 7.5 (2H,s).

### b) Benzyloxycarbonyl-(S)-β-(4-(tert-butoxycarbonylamino)pyrazol-1-yl)alanine

A solution of 4-(tert-butoxycarbonylamino)pyrazole (0.41g) and benzyloxycarbonylserine-(S)-β-lactone (0.5g) in acetonitrile (20 ml) and dimethylformamide 2 ml) was heated at 60-70°C for 42h. Evaporation to dryness and recrystallisation from ethyl acetate/petroleum ether (b.p. 60-80°C) afforded the title compound (0.42g), m.p. 157.5-9°C.
NMR (δ) ($DMSOd_6$) 1.5 (9H,s), 4.4 (2H,m), 5.1 (2H,m), 7.3 (5H,m).
M.S. (m/z) (FAB) (M+1) = 405 (consistent with m.w.=404).

### c) 4-(S)-(Benzyloxycarbonyl-(S)-β-(4-(tert-butoxycarbonylaminopyrazol-1-yl)alanyl)amino-5-cyclohexyl-3-(S)-hydroxypentanoic acidisobutylamide

This material was formed from benzyloxycarbonyl-(S)-β-(4-tert-butoxycarbonylamino)pyrazol-1-yl)-alanine (0.41g) and 4-(S)-amino-5-cyclohexyl-3-(S)-hydroxypentanoic acid isobutylamide (0.27g) following the procedure of Description 8(c). This gave the title compound (0.30g).
NMR (δ) ($DMSOd_6$) 0.7-2.0 (29H,m), 2.2 (2H,d), 2.95 (2H,m), 3.9 (2H,m), 4.1-4.6 (3H,m), 5.0 (2H,m), 7.2-8.0 (12H,m).
M.S. (m/z) (FAB) (M+1) = 657 (consistent with m.w. = 656).

d) 4-(S)-((S)-β-(4-tert-Butoxycarbonylamino)pyrazol-1-yl)alanyl)amino-5-cyclohexyl-3-(S)-hydroxypentanoic acid isobutylamide

A solution of 4-(S)-((benzyloxycarbonyl)-(S)-β-(4-(tert-butoxy-carbonylamino)pyrazol-1-yl)alanyl)amino-5-cyclohexyl-3-(S)-hydroxypentanoic acid isobutylamide (0.19g) in ethanol (25 ml) was hydrogenated at room temperature and pressure in the presence of 10% palladium on carbon for 22h. Filtration and evaporation of the filtrate in vacuo afforded the title compound (0.13g).
M.S. (m/z) (FAB) (M+1) = 523 (consistent with m.w. = 522).

e) 4-(S)-((2,3-Dihydro-1,1-dioxobenzothiophene-3-yl)acetyl-(S)-chenylalanyl-(S)-β-(4-(tert-butoxycarbonylamino)pyrazol-1-yl)alanyl)amino-5-cyclo-hexyl-3-(S)-hydroxypentanoic acid isobutylamide

This material was formed from (2,3-dihydro-1,1-dioxobenzothiophene-3-yl)acetyl-(S)-phenylalanine (0.085g) and 4-(S)-((S)-β-(4-(tert-butoxycarbonylamino)pyrazol1-yl)-alanyl)amino-5-cyclohexyl-3-(S)-hydroxypentanoic acid isobutylamide (0.132g) following the procedure of Description 2(a). This gave the title compound (0.077g).
NMR (δ) ($CDCl_3$) 0.8-2.0 (29H,m), 2.2-2.4 (2H,m), 2.5-2.9 (2H,m), 3.8-4.1 (3H,bm), 4.3-4.8 (3H,m), 7.1-7.9 (15H,m).
M.S. (m/z) (FAB) (M+1) = 878 (consistent with m.w. = 877).

## Description 59

(5-Carboxy-2,3-dihydrobenzofuran-3-yl)acetic acid

Ethyl (5-carboxy-2,3-dihydrobenzofuran-3-yl)acetate (0.30g) and sodium hydroxide (0.10g) were dissolved in water (10 ml), and left to stand for 16h. The solution was acidified (5M hydrochloric acid), and extracted with ethyl acetate. The extract was dried ($Na_2SO_4$) and evaporated, giving the title compound (0.26g) as a light yellow powder.
NMR (δ) ($DMSOd_6$) 2.6(1H) and 2.8(1H)(ABX), 3.8(1H,m), 4.3(1H,dd), 4.8(1H,t), 6.8(1H,d), 7.75(1H,dd), 7.85-(1H,d), 12.5(1H,bs).

## Description 60

a) 3-(Benzyloxycarbonylmethylidene)phthalide

Triphenylphosphine (8.27g) and benzyl bromoacetate (5.1 ml) were stirred at gentle reflux in dry toluene (100 ml) for 15 min. Triethylamine (4.4 ml) and phthalic anhydride (4.67g) were added, and reflux was continued for 16h. The mixture was evaporated, dissolved in chloroform (50 ml), and poured into ether (350 ml). After decantation, and washing of the black residue with ether, the combined solutions were evaporated to a brown oil. This was purified by chromatography on silica gel using ether/petroleum ether (b.p. 60-80°C) (20-50% ether, gradient). Recrystallisation from ether/petroleum ether (b.p. 60-80°C) then gave the title compound (1.64g) as straw-coloured needles, m.p. 113-114°C.
NMR (δ) ($CDCl_3$) 5.3 (2H, s), 6.2 (1H, s), 7.4 (5H, m), 7.7 (1H, t), 7.8 (1H, t), 8.0 (1H, d), 9.05 (1H, d).

b) 1,3-Dihydro-3-oxobenzo[c]furan-1-ylacetic acid

3-(Benzyloxycarbonylmethylidene)phthalide (1.22g) was hydrogenated over 10% palladium on charcoal (0.51g) in ethanol for 15 min (hydrogen uptake: 200 ml). Filtration and evaporation then gave the title compound (0.83g) as a white powder.
NMR (δ) ($DMSOd_6$) 2.8 (1H) and 3.25 (1H)(ABX), 6.0 (1H,dd), 7.7 (1H,t), 7.75-8.0 (3H,m), 12.8 (1H,b).

## Example 1

4-(S)-((2,3-Dihydrobenzofuran-2-carbonyl)-(S)-phenylalanyl-(S)-leucyllamino-5-cyclohexyl-3-(S)-hydroxypentanoic acid isobutylamide hemihydrate

To a stirred solution of (2,3-dihydrobenzofuran-2-carbonyl)-(S)-phenylalanyl-(S)-leucine (0.34g), 4(S)-amino-5-cyclohexyl-3-(S)-hydroxypentanoic acid isobutylamide * (0.22 g) and hydroxybenzotriazole (0.11g) in dry tetrahydrofuran at 0° was added dicyclohexylcarbcdiimide. After a few minutes, the reaction was allowed to come to room temperature and stirred overnight. The mixture was filtered and evaporated to dryness. The residue was filtered through silica (in 2% methanol/chloroform). The product was then purified by chromatotron using 0-2% methanol/chloroform to give 4-(S)-((2,3-dihydrobenzofuran-2-carbonyl)-(S)-phenylalanyl-(S)-leucyl)amino-5-cyclohexyl-3-(S)-hydroxypentanoic acid isobutylamide hemihydrate (150 mg).
Analysis: $C_{39}H_{56}N_4O_6$ $0.5H_2O$ requires C,68.3; H,8.4; N,8.2%. Found: C,68.1; H,8.6: N,7.8%.
M.S. (FAB) (m/z) (M+1)=677 (consistent with m.w.=676).

## Example 2

4-(S)-(3-(2,3-Dihydrobenzofuran-2-yl)propanoyl-(S)-chenylalanyl-(S)-leucyl)amino-5-cyclohexyl-3-(S)-hydroxypentanoic acid isobutylamide hemihydrate

This material was formed from 3-(2,3-dihydrobenzofuran-2-yl)propanoyl-(S)-phenylalanyl-(S)-leucine (512 mg) and 4-(S)-amino-5-cyclohexyl-3-(S)-hydroxypentanoic acid isobutylamide (300 mg) by the procedure described in Description 2(c). This gave 4-(S)-(3-(2,3-dihydrobenzofuran-2-yl)propanoyl-(S)-phenylalanyl-(S)-leucyl)amino-5-cyclohexyl-3-(S)-hydroxypentanoic acid isobutylamide hemihydrate (660 mg).
NMR (δ) ($DMSOd_6$) 0.7-0.9(12H,m), 2.0-2.2(4H,m), 3.8(2H,b), 4.2-4.9(3H,m), 6.7-8.2(12H,m).
Analysis: $C_{4}\cdot H_{60}N_4O_6.0.5H_2O$ requires C,69.0; H,8.6; N,7.9%. Found: C,68.9; H,8.4; N,8.3%.
M.S. (m/z) (FAB) (M+1) = 705 (consistent with m.w. = 704).

## Example 3

4-(S)-((7-Methoxy-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

This material was formed from (7-methoxy-2,3-dihydrobenzofuran-3-yl)acetyl-(S)-phenylalanyl-(S)-leucine (530 mg) and 4-(S)-amino-5-cyclohexyl-3-(S)-hydroxypentanoic acid isobutylamide (300 mg) as described in Description 2(c). This gave 4-(S)-((7-methoxy-2,3-dihydrobenzofuran-3-yl)acetyl-(S)-phenylalanyl-(S)-leucyl)amino-5-cyclohexyl-3-(S)-hydroxypentanoic acid isobutylamide hemihydrate (460 mg).
NMR (δ) ($DMSOd_6$) 0.7-1.0(12H,m), 2.0-2.3(4H,m), 3.7(3H,s), 6.5-6.8(3H,m), 7.1-7.3(5H,m), 7.3(1H,d), 7.7-(1H,t), 8.1-8.3(2H,m).
Analysis: $C_{41}H_{60}N_4O_7.0.5H_2O$ requires C,67.5; H,8.4; N,7.7%. Found: C,67.4; H,8.6; N,7.5%.

* J. Med. Chem. 1985, 28, 1779-1790.

M.S. (m/z) (FAB) (M + 1) = 721 (consistent with m.w. = 720).

Example 4

4-(S)-((2,3-Dihydrobenzofuran-3-yl)acetyl-(S)-phenylalanyl-(S)-leucyl)amino-5-cyclohexyl-3-(S)-hydroxypentanoic acid isobutylamide hydrate

This material was formed from (2,3-dihydrobenzofuran-3-yl)acetyl-(S)-phenylalanyl-(S)-leucine (140 mg) and 4-(S)-amino-5-cyclohexyl-3-(S)-hydroxypentanoic acid isobutylamide as described in Description 2(c). This gave 4-(S)-((2,3-dihydrobenzofuran-3-yl)acetyl-(S)-phenylalanyl-(S)-leucyl)amino-5-cyclohexyl-3-(S)-hydroxypentanoic acid isobutylamide hydrate (180 mg).
NMR (δ) ($CDCl_3$) 0.7-1.0(13H,m), 2.2-2.7(4H,m), 6.7-7.4(11H,m).
Analysis: $C_{40}H_{58}N_4O_6.H_2O$ requires C,67.8; H,8.5; N,7.9%. Found C,67.8; H,8.6; N,8.3%.
M.S. (m/z) (FAB) (M + 1) = 691 (consistent with m.w. = 690).

Example 5

4-(S)-((5-Nitro-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

To a stirred, ice cooled mixture of (5-nitro-2,3-dihydrobenzofuran-3-yl)acetyl-(S)-phenylalanyl-(S)-leucine (0.55 g, 1.14 mmoles) and 1-hydroxybenzotriazole (0.154 g; 1.14 mmoles) in dry dichloromethane 20 ml) was added 1-(3-dimethylaminopropyl)-3-ethylcarbodiimide hydrochloride (0.22 g, 1.14 mmoles) in one portion. After stirring for 10 min a solution of 4-(S)-amino-5-cyclohexyl-3-(S)-hydroxypentanoic acid isobutylamide (0.31 g, 1.14 mmoles) in dry dichloromethane (10 ml) was added and stirring continued for a further 18 h at room temperature. Dichloromethane (100 ml) was added and the solution was washed with water (2x100 ml), 5 M hydrochloric acid (50 ml), saturated sodium hydrogen carbonate (50 ml) and saturated brine (100 ml). Organics were dried over anhydrous sodium sulphate and evaporated to dryness in vacuo. The residue was chromatographed on silica gel using 0-1% methanol/chloroform to afford 4-(S)-((5-nitro-2,3-dihydrobenzofuran-3-yl)acetyl-(S)-phenylalanyl-(S)-leucyl)amino-5-cyclohexyl-3-(S)-hydroxypentanoic acid isobutylamide hemihydrate (0.29 g, 34%).
NMR (δ) ($CDCl_3$) 0.7-2.0(29H,m), 2.2-3.2(7H,m), 3.6-5.2(7H,m), 6.6-8.1(11H,m).
Analysis: $C_{40}H_{57}N_5O_8$ $0.5H_2O$ requires C,64.5; H,7.9; N,9.4%. Found: C,64.4; H,7.6; N,9.2%.
M.S. (m/z) (FAB) (M + 1) = 736 (consistent with m.w. = 735).

Example 6

4-(S)-((5-Amino-2,3-dihydrobenzofuran-3-yl)acetyl-(S)-phenylalanyl-(S)-leucyl)amino-5-cyclohexyl-3-(S)-hydroxypentanoic acid isobutylamide trifluoroacetate hemihydrate

A solution of 4-(S)-((5-nitro-2,3-dihydrobenzofuran-3-yl)acetyl-(S)-phenylalanyl-(S)-leucyl)amino-5-cyclohexyl-3-(S)-hydroxypentanoic acid isobutylamide (0.195 g, 0.26 mmoles) in ethanol (20 ml) and trifluoroacetic acid (0.09 g, 0.78 mmoles) was hydrogenated at room temperature and atmospheric pressure in the presence of 10% palladium on carbon for 3 h. The catalyst was removed by filtration through kieselguhr and the filtrate evaporated to dryness in vacuo. The residue was dissolved in ethanol and precipitation by addition of ether afforded 4-(S)-((5-amino-2,3-dihydrobenzofuran-3-yl)acetyl-(S)-phenylalanyl-(S)-leucyl)amino-5-cyclohexyl-3-(S)-hydroxypentanoic acid isobutylamide trifluoroacetate hemihydrate (0.15 g).
NMR (δ) ($CDCl_3$) 0.8-2.0(29H,m), 2.2-3.3(9H,m), 3.6-4.8(7H,m), 6.7-7.4(8H,m), 8.3(1H,m).
Analysis: $C_{40}H_{59}N_5O_6.CF_3CO_2H$ $0.5H_2O$ requires C,60.9; H,7.4; N,8.5%. Found: C,60.6; H,7.5; N,8.3%.
M.S. (m/z) (FAB) (M + 1) = 706 (consistent with m.w. of free base = 705).

Example 7

4-(S)-((5-Ureido-2,3-dihydrobenzofuran-3-yl)acetyl-(S)-phenylalanyl-(S)-leucyl)amino-5-cyclohexyl-3-(S)-hydroxypentanoic acid isobutylamide dihydrate

Prepared as described in Example 5 from (5-ureido2,3-dihydrobenzofuran-3-yl)acetyl-(S)-phenylalanyl-(S)-leucine (0.367 g, 0.74 mmoles) and 4-(S)-amino-5-cyclohexyl-3-(S)-hydroxypentanoic acid isobutylamide (0.20 g, 0.74 mmoles). This gave 4-(S)-((5-ureido-2,3-dihydrobenzofuran-3-yl)acetyl-(S)-phenylalanyl-(S)-leucyl)amino-5-cyclohexyl-3-(S)-hydroxypentanoic acid isobutylamide dihydrate (0.077 g).
NMR (δ) (CD30D) 0.6-2.0(30H,m), 2.2-3.3(7H,m), 3.6-4.8(6H,m), 6.5-7.4(8H,m).
Analysis: $C_{41}H_{60}N_6O_7.2H_2O$ requires C,62.7; H,8.2; N,10.7%. Found C,63.0; H,7.8; N,10.7%.
M.S. (m/z) (FAB) (M+1)=749 (consistent with m.w.=748).

Example 8

4-(S)-((7-Nitro-2,3-dihydrobenzofuran-3-yl)acetyl-(S)-phenylalanyl-(S)-leucyl)amino-5-cyclohexyl-3-(S)-hydroxypentanoic acid isobutylamide

Prepared as described in Example 5 from (7-nitro-2,3-dihydrobenzofuran-3-yl)acetyl-(S)-phenylalanyl-(S)-leucine (0.36 g, 0.74 mmoles). This gave 4-(S)-((7-nitro-2,3-dihydrobenzofuran-3-yl)acetyl-(S)-phenylalanyl-(S)-leucyl)amino-5-cyclohexyl-3-(S)-hydroxypentanoic acid isobutylamide (0.2 g).
NMR (δ) ($DMSOd_6$) 0.7-2.0(29H,m), 2.0-3.1(8H,m), 3.8-5.0(8H,m), 6.9-8.2(12H,m).

Example 9

4 (S)-((5-(Methylaminosulphonyl)-2,3-dihydrobenzofuran-3-yl)acetyl-(S)-phenylalanyl-(S)-leucyl)amino-5-cyclohexyl-3-(S)-hydroxypentanoic acid isobutylamide

This material was formed from (5-(methylaminosulphonyl)-2,3-dihydrobenzofuran-3-yl)acetyl-(S)-phenylalanyl-(S)-leucine (0.203 g), hydroxybenzotriazole (0.057 g), dicyclohexylcarbodiimide (D.C.C.) (0.087 g) and 4-(S)-amino-5-cyclohexyl-3-(S)-hydroxypentanoic acid isobutylamide (0.103 g) in dry dimethylformamide (6 ml), using the preparative procedure of Description 8(c), but omitting addition of diisopropylethylamine. The crude product so obtained (0.155 g) was purified by chromatography on silica gel using methanol/chloroform (gradient elution, 0-2% methanol), to give 4-(S)-((5-(methylaminosulphonyl)-2,3-dihydrobenzofuran-3-yl)acetyl-(S)-phenylalanyl-(S)-leucyl)amino-5-cyclohexyl-3-(S)-hydroxypentanoic acid isobutylamide as a white solid (0.101 g) after trituration with ether.
NMR (δ) ($CD_3OD$) 0.7-1.1(12H,m), 1.1-2.0(17H,m), 2.27(2H,m), 2.4-3.25(m), 3.7-4.8(6H,m), 6.85(1H,m), 7.15-7.35(6H,m), 7.6(2H,m).
Analysis: $C_{41}H_{61}N_5O_8S$ requires C,62.8; H,7.8; N,8.9%.
Found: C,62.6; H,8.0; N,9.2%.
M.S. (m/z) (FAB) (M+1)=784 (consistent with m.w.=783).

Example 10

4-(S)-((2,3-Dihydrobenzothiophene-2-carbonyl)-(S)-phenylalanyl-(S)-leucyl)amino-5-cyclohexyl-3-(S)-hydroxypentanoic acid isobutylamide hemihydrate

This material was formed from (2,3-dihydrobenzothiophene-2-carbonyl)-(S)-phenylalanyl-(S)-leucine 0.36 g), 4-(S)-amino-5-cyclohexyl-3-(S)-hydroxypentanoic acid isobutylamide (0.22 g), and hydroxybenzotriazole (0.12 g) using 1-(3-dimethylaminopropyl)-3-ethylcarbodiimide hydrochloride (0.17 g), in place of dicyclohexylcarbodiimide, in dry dimethylformamide (10 ml) using the method of Description 8(c). The solid was

chromatographed on silica gel using chloroform to give 4-(S)-((2,3-dihydrobenzothiophene-2-carbonyl)-(S)-phenylalanyl-(S)-leucyl)amino-5-cyclohexyl-3-(S)-hydroxypentanoic acid isobutylamide hemihydrate (271 mg).

NMR (δ) ($CDCl_3$) 0.7-1.0(14H,m), 1.1-1.3(5H,m), 1.4-1.7(9H,m), 1.7-1.9(3H,m), 2.2-2.4(2H,m), 2.9-3.2(4H,m), 3.4-3.7(2H,m), 3.9-4.1(2H,m), 4.1-4.2(1H,m), 4.2-4.4(1H,m), 4.4-4.7(1H,m), 6.3-6.9(3H,m), 7.0-7.2(7H,m), 7.2-7.3(2H,m).

Analysis: $C_{39}H_{56}N_4O_5S.0.5H_2O$ requires C,66.7; H,8.2; N,8.0%. Found: C,66.9; H,8.2; N,8.0%.

M.S. (m/z) (El Pos.) M = 692.

Example 11

4-(S)-((2,3-Dihydro-1,1-dioxobenzothiophene-2-carbonyl))-(S)-phenylalanyl-(S)-leucyl)amino-5-cyclohexyl-3-(S)-hydroxypentanoic acid isobutylamide

4-(S)-((2,3-Dihydrobenzcthiophene-2-carbonyl)-(S)-phenylalanyl-(S)-leucyl)amino-5-cyclohexyl-3-(S)-hydroxypentanoic acid isobutylamide hemihydrate (0.11 g) was dissolved in dichloromethane (4 ml) and stirred at ice bath temperature. The solution was treated portionwise with m-chloroperoxybenzoic acid (0.07 g) and the mixture was left stirring overnight. The solution was diluted with chloroform (20 ml) washed with 10% sodium carbonate (2x15 ml), 5M hydrochloric acid (15 ml) and brine (15 ml). The organic layer was dried over magnesium sulphate and the solvent removed under reduced pressure. The solid produced was chromatographed on silica gel using 2% methanol/chloroform to give 4-(S)-((2,3-dihydro-1,1-dioxobenzothiophene-2-carbonyl)-(S)-phenylalanyl-(S)-leucyl)amino-5-cyclohexyl-3-(S)-hydroxypentanoic acid isobutylamide (52 mg).

NMR (δ) ($CDCl_3$) 0.5-1.1(15H,m), 1.1-1.9(16H,m), 2.0-2.5(3H,m), 2.9-3.3(3H,m), 3.3-3.7(1H,m), 3.7-4.1(3H,m), 4.1-4.5(2H,m), 4.5-4.7(1H,m), 6.2-6.8(3H,m), 7.0-7.3(5H,m), 7.3-7.8(4H,m).

M.S. (m/z) (El Pos.) M = 724.

Example 12

4-(S)-((2,3-Dihydro-1,1-dioxobenzothiophene-3-yl)acetyl-(S)-phenylalanyl-(S)-leucyl)amino-5-cyclohexyl-3-(S)-hydroxypentanoic acid isobutylamide

This material was formed from (2,3-dihydro-1,1-dioxobenzothiophene-3-yl)acetyl-(S)-phenylalanyl-(S)-leucine (100 mg) and 4-(S)-amino-5-cyclohexyl-3-(S)-hydroxypentanoic acid isobutylamide as described in Description 2(c). This gave 4-(S)-((2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide.

NMR (δ) ($CDCl_3$) 0.7-1.0(13H,m), 6.9-7.8(12H,m), 8.0-8.3(1H,bm).

Analysis: $C_{40}H_{58}N_4O_7S$ requires C,65.0; H,7.9; N,7.6%. Found C,64.7; H,7.9; N,7.5%.

M.S. (m/z) (FAB) (M + 1) = 739 consistent with m.w. = 738).

Example 13

4-(S)-((5-Formyl-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

This material was formed from (5-formyl-2,3-dihydrobenzofuran-3-yl)acetyl-(S)-phenylalanyl-(S)-leucine (0.12g) following the procedure of Description 8(c) except that 1-(3-dimethylaminopropyl)-3-ethylcarbodiimide hydrochloride (D.E.C.) was used in place of D.C.C. The crude product (0.16g) was chromatographed on silica gel using methanol/chloroform (gradient, 0-2% methanol). This gave 4-(S)-((5-formyl-2,3-dihydrobenzofuran-3-yl)acetyl-(S)-phenylalanyl-(S)-leucyl)amino-5-cyclohexyl-3-(S)-hydroxypentanoicoic acid isobutylamide hemihydrate (72 mg).

NMR (δ) ($CDCl_3$) 0.8-1.0 (13H,m), 1.1-1.8 (m), 2.2-2.4 2H,m), 2.4-2.75 (2H,m), 2.9-3.2 (4H,m), 3.8-4.9

(8H,m), 6.4-6.8 (4H,m), 6.8-6.9 (1H,m), 7.15-7.35 (5H,m), 7.7 (2H,m), 9.8 (1H,d).
Analysis: $C_{41}H_{58}N_4O_7.0.5H_2O$ requires C,67.6; H,8.2; N,7.7%. Found: C,67.8; H,8.3; N,7.7%.
M.S. (m/z) (FAB) (M+1)=719 (consistent with m.w.=718).

## Examples 14 and 15

4-(S)-((5-(Hydroxymethyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate, less polar and more polar isomers

To 4-(S)-((5-formyl-2,3-dihydrobenzofuran-3-yl)acetyl-(S)-phenylalanyl-(S)-leucyl)amino-5-cyclohexyl-3-(S)-hydroxypentanoic acid isobutylamide (50 mg) in ethanol (4 ml) was added, with stirring, sodium borohydride (10 mg). The mixture was stirred for 30 min, poured into water (50 ml), acidified with 5 M HCl, and extracted with chloroform (3x20 ml). The combined extracts were dried ($Na_2SO_4$) and evaporated. The crude product (50 mg) was chromatographed on silica gel using methanol/chloroform (gradient, 0-4% methanol). This gave, initially, 4-(S)-((5-(hydroxymethyl)-2,3-dihydrobenzofuran-3-yl)acetyl-(S)-phenylalanyl-(S)-leucyl) amino-5-cyclohexyl-3-(S)-hydroxypentanoic acid isobutylamide hemihydrate, less polar isomer (Example 14) (25 mg).
NMR (δ) ($CD_3OD$) 0.75-1.1 (13H,m), 1.1-1.5 (6H,m), 1.5-1.9 (10H,m), 2.2 (2H,d), 2.25-2.7 (2H, ABX), 2.8-3.1 (3H,m), 3.1-3.3 (1H,m), 3.6-3.8 (1H,m), 3.95 2H,m), 4.1-4.8 (6H,m), 6.7 (1H,m), 7.05-7.4 (7H,m), 7.5-8.9 (several small m).
Analysis: $C_{41}H_{60}N_4O_7$ $0.5H_20$ requires C,67.5; H,8.4; N,7.7%. Found: C,67.2; H,7.9; N,7.3%.
M.S. (m/z) (FAB) (M+1)=721 (consistent with m.w.=720).
rf (5% MeOH/$CHCl_3$, silica gel) : 0.51.

Further elution gave 4-(S)-((5-(hydroxymethyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate, more polar isomer (Example 15) (17 mg).
NMR : identical with that of the above less polar isomer.
Analysis: $C_{41}H_{60}N_4O_7$ $0.5H_2O$ requires C,67.5; H,8.4; N,7.7%. Found: C,67.3; H,7.9; N,7.7%.
M.S. (m/z) (FAB) (M+1)=721 (consistent with m.w.=720).
rf (5% MeOH/$CHCl_3$, silica gel) : 0.43.

## Example 16

4-(S)-((5-(Methoxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

To (5-(methoxycarbonyl)-2,3-dihydrobenzofuran-3-yl)acetichydrazide (100 mg) in water (5 ml) containing 5 M HCl (0.5 ml), at 0°C, was added, with stirring, sodium nitrite (83 mg) in water (1 ml). After 10 min, the mixture was extracted into ethyl acetate (3x5 ml) at 0°C. The extract, maintained at 0°C, was dried ($Na_2SO_4$), filtered, and stirred as 4-(S)-((L-phenylalanyl-(S)-leucyl)amino)-5-cyclohexyl-3-(S)-hydroxypentanoic acid isobutylamide (200 mg) and diisopropylethylamine (0.1 ml) were added. This mixture was stirred for 20h, whilst warming to ambient temperature, washed successively with 5% citric acid solution, water, 5% sodium hydrogen carbonate solution, and brine; dried ($Na_2SO_4$) and evaporated under reduced pressure, giving a yellow solid (120 mg). This was chromatographed on silica gel using methanol/chloroform (gradient, 0-2% methanol). This gave 4-(S)-((5-(methoxycarbonyl)-2,3-dihydrobenzofuran-3-yl)acetyl-(S)-phenylalanyl-(S)-leucyl)amino-5- cyclohexyl-3-(S)-hydroxypentanoic acid isobutylamide hemihydrate (66 mg).
NMR (δ) $CDCl_3$) 0.8-1.15 (13H,m), 1.15-1.9 (m), 2.4 (2H,m), 2.45-2.8 (2H,m), 3.15 (4H,m), 3.8-4.0 (4H,m), 4.0-4.15 (2H,m), 4.2 (0.5H,dd), 4.3-4.5 (2.5H,m), 4.65-4.9 (2H,m), 6.3-6.75 (3H,m), 6.85 (1H,m), 7.2-7.45 (6H,m), 7.8-8.0 (2H,m).
Analysis: $C_{42}H_{60}N_4O_8.0.5H_2O$ requires C,66.5; H,8.1; N,7.4%. Found: C,66.7; H,8.1; N,7.7%.
M.S. (m/z) (FAB) (M+1)=749 (consistent with m.w.=748).

Example 17a

4-(S)-((5-Carboxy-2,3-dihydrobenzofuran-3-yl)acetyl-(S)-phenylalanyl-(S)-leucyl)amino-5-cyclohexyl-3-(S)-hydroxypentanoic acid isobutylamide

Method 1

This material was formed from 4-(S)-((5-(methoxycarbonyl)2,3-dihydrobenzofuran-3-yl)acetyl-(S)-phenylalanyl-(S)-leucyl)amino-5-cyclohexyl-3-(S)-hydroxypentanoic acid isobutylamide (50 mg) following the procedure of Description 8(b), but using potassium hydroxide (11 mg) and a reaction time of 3 days. This gave 4-(S)-((5-carboxy-2,3-dihydrobenzofuran-3-yl)-acetyl-(S)-phenylalanyl-(S)-leucyl)amino-5-cyclohexyl-3-(S)-hydroxypentanoic acid isobutylamide (17 mg).
NMR (δ) ($CDCl_3$) 0.7-1.05 (13H,m), 1.05-1.9 (m), 2.4-2.8 (4H,m), 2.8-3.2 (4H,m), 3.6-4.8 (8H,m), 6.1-6.9 (4H,m), 7.1-7.3 (6H,m), 7.75-7.95 (2H,m).
M.S. (m/z) (FAB) (M+1)=735 (consistent with m.w.=734).

Method 2

This material was also formed from (5-carboxy-2,3-dihydrobenzofuran-3-yl)acetic acid (0.041g) and 4-(S)-((S)-phenylalanyl-(S)-leucyl)amino-5-cyclohexyl-3-(S)-hydroxypentanoic acid isobutylamide (0.10g), following the procedure of Example 13, but omitting washing with sodium hydrogen carbonate solution during workup. The crude product was chromatographed on silica gel using methanol/chloroform (0-10% methanol, gradient), giving the title compound as sesquihydrate. NMR, M.S. as reported above.
Analysis: $C_{41}H_{58}N_4O_8.1.5H_2O$ requires C,64.6; H,8.1; N,7.3%. Found: C,64.5; H,7.8; N,7.4%.

Method 3

4-(S)-((5-Benzyloxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide (0.40g) was hydrogenated over 10% palladium on charcoal (60% aqueous paste, 0.32g) in dimethylformamide (10 ml) for 2h. Catalyst was filtered off, and the filtrate was concentrated, dissolved in methanol (5 ml), diluted with ethyl acetate (100 ml), and washed with water and brine, dried ($Na_2SO_4$) and evaporated. This gave the title compound (0.33g) as a white solid.

Example 17b

4-(S)-((5-Carboxy-2,3-dihydrobenzofuran-3-yl)acetyl-(S)-phenylalanyl-(S)-leucyl)amino-5-cyclohexyl-3-(S)-hydroxypentanoic acid isobutylamide, sodium salt

The corresponding acid (32 mg) was dissolved in methanol (2 ml), and aqueous sodium hydroxide (1.52 ml of 1.15 mg/ml solution) was added. The solution was diluted with water (30 ml) and freeze-dried immediately, giving 4-(S)-((5-carboxy-2,3-dihydrobenzofuran-3-yl)acetyl-(S)-phenylalanyl-S-leucyl)- (S)-amino-5-cyclohexyl-3-(S)-hydroxypentanoic acid isobutylamide, sodium salt (33 mg) as a fluffy white solid.
NMR (δ) ($D_2O$) 0.7-1.0 (13H, m), 1.05-1.8 (16H, m), 2.3 (2H, m), 2.45-2.8 (2H, m), 2.8-3.2 (4H, m), 3.3-4.15 (5H, b), 4.3 (2H, m), 6.8 (1H, m), 7.1-7.4 (5H, m), 7.6-7.8 (2H, m).

Example 18

4-(S)-(2,3-Dihydro-1,1-dioxobenzothiophene-3-yl)acetyl-(S)-phenylalanyl-(S)-leucyl)amino-5-cyclohexyl-3-(S)-hydroxypentanoic acid 3-methylbutylamide hemihydrate

4-(S)-(Benzyloxycarbonyl-(S)-phenylalanyl-(S)-leucyl)amino-5-cyclohexyl-3-(S)-hydroxypentanoic acid 3-methylbutylamide (0.11g) and 10% palladium/charcoal (0.05g) were stirred in ethanol (20 ml) under hydrogen atmosphere for 2h. Catalyst was filtered off, and the filtrate was evaporated in vacuo. The residue was reacted with (2,3-dihydro-1,1-dioxobenzothiophene-3-yl)acetic acid (0.04g), using hydroxybenzotriazole (0.024g) and 1-(3-dimethylaminopropyl)-3-ethylcarbodiimide hydrochloride (0.034g), in dry dimethylformamide (5 ml), by the method of Example 13. The crude product was chromatographed on silica gel using methanol/chloroform (0-10% methanol, gradient). This gave 4-(S)-(2,3-dihydro-1,1-dioxobenzothiophene3-yl)-acetyl-(S)-phenylalanyl-(S)-leucyl)amino-5-cyclohexyl-3-(S)-hydroxypentanoic acid 3-methylbutylamide hemihydrate (0.09g).

NMR (δ) ($CDCl_3$) 0.8-1.0 (13H,m), 1.0-1.9 (19H,m), 2.25 (2H,m), 2.5-2.8 (2H,m), 2.8-3.7 (6H,m), 3.8-4.1 (3H,m), 4.3-5.0 (2H,m), 6.4-7.7 (13H, m).

Analysis: $C_{41}H_{60}N_4O_7S$ $0.5H_2O$ requires C,64.6; H,8.1; N,7.3%. Found: C,64.5; H,7.9; N,7.4%.

M.S, (m/z) (FAB) (M+1)=753 (consistent with m.w.=752).

## Example 19

4-(S)-((5-Cyano-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

This material was formed from (5-cyano-2,3-dihydrobenzofuran-3-yl)acetyl-(S)-phenylalanyl-(S)-leucine (0.23g), following the procedure of Example 13. The crude product was chromatographed on silica gel using methanol/chloroform (gradient, 0-2% methanol). This gave 4-(S)-((5-cyano-2,3-dihydrobenzofuran-3-yl)-acetyl-(S)-phenylalanyl-(S)-leucyl)amino-5-cyclohexyl-3-(S)-hydroxypentanoic acid isobutylamide hemihydrate (0.24g).

NMR (δ) ($DMSOd_6$) 0.7-1.0 (13H,m), 1.0-1.8 (16H,m), 2.05-2.2 (2H,m),2.3-2.45 (1H,m), 2.5-2.6 (1H,m), 2.65-3.1 (5H,m), 3.65-3.75 (1H,m), 3.75-3.95 (2.5H,m), 4.15-4.4 ($1\frac{1}{2}$ H,m), 4.4-4.7 (2H,m+2xt), 4.8-4.95 (1H,2xm), 6.9 (1H,m), 7.15-7.3 (5H,m), 7.3-7.7 (3H,m), 7.75 (1H,m), 8.2-8.4 (2H,m).

Analysis: $C_{41}H_{57}N_5O_6.0.5H_2O$ requires C,68.0; H,7.9; N,9.7%. Found: C,67.9; H,8.1; N.9.7%.

M.S. (m/z) (FAB) (M+1)=716 (consistent with m.w.=715).

## Example 20a

4-(S)-((5-(Aminomethyl)-2,3-dihydrobenzofuran-3-yl)acetyl-(S)-phenylalanyl-(S)-leucyl)amino-5-cyclohexyl-3-(S)-hydroxypentanoic acid isobutylamide hemi(trichloromethanate)

A suspension of 4-(S)-((5-cyano-2,3-dihydrobenzofuran-3-yl)acetyl-(S)-phenylalanyl-(S)-leucyl)amino-5-cyclohexyl-3-(S)-hydroxypentanoic acid isobutylamide (1.19g) was stirred in methanol (25 ml). Cobalt (II) chloride hexahydrate (0.80g) was added, followed 5 min later by sodium borohydride (0.30g). After the black colouration was discharged, a further portion of sodium borohydride (0.30g) was added; and upon discharge of this second black colouration, the mixture was diluted with water (200 ml), acidified (5M HCl), and finally basified with aqueous ammmonia (sp.gr.0.88). The product was extracted into chloroform, dried ($Na_2SO_4$), evaporated, and chromatographed on silica gel using methanol/chloroform (0-20% methanol, gradient). This gave 4-(S)-((5-(aminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide hemi(trichloromethanate) (0.53g) as a transparent foam.

NMR (δ) ($CD_3OD$) 0.7-1.05 (13H,m), 1.1-1.9 (16H,m), 2.25 (2H,d), 2.3-2.45 (1H,m), 2.55-2.65 (1H,m), 2.85-2.95 (1H,m), 3.0 (2H,d), 3.15-3.25 (1H,m), 3.6-3.8 (3H,m+2xs), 3.9-4.0 (2.5H, m+dd), 4.15 (0.5H,dd), 4.35-4.5 (2H,m), 6.7 (1H,2xd), 7.05-7.15 (2H,m), 7.2-7.3 (5H,m).

Analysis: $C_{41}H_{61}N_5O_6.0.5(CHC_{13})$ requires C,63.9; H,7.9; N,9.0%. Found: C,64.1; H,8.1; N,9.0%. M.S. (m/z) (FAB) (M+1)=720 (consistent with m.w.=719).

## Example 20b

4-(S)-((5-(Aminomethyl)-2,3-dihydrobenzofuran-3-yl)acetyl-(S)-phenylalanyl-(S)-leucyl)amino-5-cyclohexyl-3-(S)-hydroxypentanoic acid isobutylamide hydrochloride dihydrate

The corresponding free base (Example 20(a)) (0.21g) was dissolved in methanol (2 ml), and the solution was acidified by addition of 5M HCl, diluted with water (50 ml), and freeze-dried immediately. This gave 4-(S)-((5-(aminomethyl)-2,3-dihydrobenzofuran-3-yl)-acetyl-(S)-phenylalanyl-(S)-leucyl)amino-5-cyclohexyl-3-(S)-hydroxypentanoic acid isobutylamide hydrochloride dihydrate (0.21g) as a white powder.
NMR (δ) ($CD_3OD$) 0.7-1.1 (13H,m), 1.1-1.9 (16H,m), 2.25 (2H,d), 2.3-2.7 (3H,m), 2.8-3.1 (4H,m+d), 3.65-3.8 (1H,m), 3.9-4.1 (5H,m), 4.2 (0.5H,dd), 4.3-4.55 (2.5H,m), 6.75 (1H,m), 7.15-7.35 (7H,m).
Analysis: $C_{41}H_{61}N_5O_6$.HCl.$2H_2O$ requires C,62.1; H,8.4; N,8.8%. Found: C,61.8; H,8.0; N,8.7%.

Example 21

4-(S)-((5)-(Benzyloxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide

This material was formed from (5-(benzyloxycarbonyl)-2,3-dihydrobenzofuran-3-yl)acetic acid (0.153g) and 4-(S)-((S)-phenylalanyl-(S)-leucyl)amino-5-cyclohexyl-3-(S)-hydroxypentanoic acid isobutylamide (0.307g), following the procedure of Example 13. The crude product was chromatographed on silica gel using methanol/chloroform (0-2% methanol, gradient). This gave 4-(S)-((5-(benzyloxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide (0.267g) as a yellow powder.
NMR (δ) ($CDCl_3$) 0.8-1.0 (13H,m), 1.0-1.9 (16H,m), 2.25-2.5 (3H,m), 2.65 (1H,m), 3.05 (4H,m), 3.6-4.1 (4H,2xm), 4.2-5.0 (4H,m), 5.30 (2H,m), 6.4-7.0 (4H,m), 7.0-7.5 (11H,m), 7.75-7.95 (2H,m).
Analysis: $C_{48}H_{64}N_4O_8$ requires C,69.9; H,7.8; N,6.8%. Found: C,69.6; H,8.0; N,6.9%.
M.S. (m/z) (FAB) (M+1)=825 (consistent with m.w.=824).

Example 22

4-(S)-((5-(Methanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

This material was formed from (5-(methanesulphonyl)-2,3-dihydrobenzofuran-3-yl)acetic acid (0.065g) and 4-(S)-((S)-phenylalanyl-(S)-leucyl)amino-5-cyclohexyl-3-(S)-hydroxypentanoic acid isobutylamide (0.158g), following the procedure of Example 13. The crude product was chromatographed on silica gel using methanol/chloroform (0-5% methanol, gradient). This gave 4-(S)-((5-methanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate (0.113g) as a yellow powder.
NMR (δ) ($DMSOd_6$) 0.7-1.0 (13H,m), 1.0-1.8 (16H,m), 2.1 (2H,m), 2.3 (1H,m), 2.6-2.8 (2H,m), 2.8-2.95 (2H,m), 3.05 (1H,m), 3.1 and 3.15 (3H, 2xs), 3.65-3.9 (3H,m), 4.0 (0.5H,dd), 4.2-4.4 (1.5H, m+dd), 4.45-4.7 (2H, m+2xt), 4.9 (1H,t), 6.95 (1H, 2xd), 7.15-7.4 (6H,m), 7.6-7.8 (3H,m), 8.15-8.3 (2H,m).
Analysis: $C_{41}H_{60}N_4O_8S$.0.5$H_2O$ requires C,63.3; H,7.9; N,7.2%. Found: C,63.4; H,7.9; N,7.1%.
M.S. (m/z) (FAB) (M+1)=769 (consistent with m.w.=768).

Example 23

4-(S)-((5-Benzyloxycarbonyl)-2,3-dihydrobenzofuran-3-yl)acetyl-(S)-phenylalanyl-(S)-histidyl)amino-5-cyclohexyl-3-(S)-hydroxypentanoic acid isobutylamide hemihydrate

This material was formed from (5-(benzyloxycarbonyl)-2,3-dihydrobenzofuran-3-yl)acetic acid (62 mg) and 4-(S)-((S)-phenylalanyl-(S)-histidyl)amino-5-cyclohexyl-3-(S)-hydroxypentanoic acid isobutylamide (100 mg), following the procedure of Example 13, but omitting washing with citric acid. The crude product was

chromatographed on silica gel using methanol/chloroform (0-10% methanol, gradient). This gave 4-(S)-((5-(benzyloxycarbonyl)-2,3- dihydrobenzofuran-3-yl)acetyl-(S)-phenylalanyl-(S)-histidyl)amino-5-cyclohexyl-3-(S)-hydroxypentanoic acid isobutylamide hemihydrate (97 mg) as a white solid.
NMR (δ) ($DMSOd_6$) 0.7-1.0 (7H, m), 1.1-1.4 (7H, m), 1.5-1.8 (6H, m), 2.0-2.2 (2H, m), 2.3 (1H, m), 2.6-3.1 (7H, m), 3.65 (1H, m), 3.8 (2H, m), 3.95 (0.5H, dd), 4.25 (0.5H, dd), 4.4-4.6 (3H, m), 4.8-5.1 (1H, m), 5.3 (2H, m), 6.7-7.0 (2H, m), 7.1-7.3 (6H, m), 7.3-7.55 (6H, m), 7.7-7.9 (3H, m), 8.2-8.5 (2H, m), 11.6-11.9 (1H, b).
Analysis: $C_{48}H_{60}N_6O_8.0.5H_2O$ requires C, 67.2; H, 7.2; N, 9.8%. Found: C, 67.4; H, 7.1; N, 9.5%.
M.S. (m/z) (FAB) (M+1)=849 (consistent with m.w.=848).

Example 24a

4-(S)-((5-Carboxy-2,3-dihydrobenzofuran-3-yl)acetyl-(S)-phenylalanyl-(S)-histidyl)amino-5-cyclohexyl-3-(S)-hydroxypentanoic acid isobutylamide

4-(S)-((5-(Benzyloxycarbonyl)-2,3-dihydrobenzofuran-3-yl)acetyl-(S)-phenylalanyl-(S)-histidyl)amino-5-cyclohexyl-3-(S)-hydroxypentanoic acid isobutylamide (81 mg) was hydrogenated over 10% palladium on charcoal (60% aqueous paste, 80 mg) in ethanol (20 ml) for 3h. The mixture was filtered, and the filtrate was evaporated, giving 4-(S)-((5-carboxy-2,3-dihydrobenzofuran-3-yl)acetyl-(S)-phenylalanyl-(S)-histidyl)-amino-5-cyclohexyl-3-(S)-hydroxypentanoic acid isobutylamide (64 mg) as a grey solid.
NMR (δ) ($DMSOd_6$) 0.7-0.95 (8H, m), 1.0-1.4 (6H, m), 1.5-1.8 (6H, m), 2.0-2.15 (2H, m), 2.3 (1H, m), 2.6-3.1 (8H, m), 3.65 (1H, m), 3.8 (2H, m), 3.9 (0.5H, dd), 4.2 (0.5H, dd), 4.4-4.65 (3H, m), 6.75-6.9 (2H, m), 7.1-7.3 (6H, m), 7.5-7.55 (1H, m), 7.7-7.8 (3H, m), 8.3 (1H, m), 8.35-8.45 (1H, m).

Example 24b

4-((5-Carboxy-2,3-dihydrobenzofuran-3-yl)acetyl-(S)-phenylalanyl-L-histidyl)amino-5-cyclohexyl-3-hydroxypentanoic acid isobutylamide hydrochloride trihydrate

This material was formed from the corresponding free base (Example 24a) (60 mg), following the procedure of Example 20b. This gave 4-(S)-((5-carboxy-2,3-dihydrobenzofuran-3-yl)acetyl-(S)-phenylalanyl-(S)-histidyl)amino-5-cyclohexyl-3-(S)-hydroxypentanoic acid isobutylamide hydrochoride trihydrate (61 mg) as a white powder.
NMR (δ) ($DMSOd_6$) 0.7-1.0 (8H,m), 1.0-1.4 (6H,m), 1.5-1.8 (6H,m), 2.1 (2H,m), 2.3 (1H,m), 2.6-2.95 (5H,m), 2.95-3.2 (1H,m), 3.7 (1H,m), 3.85 (2H,m), 3.95 (0.5H,dd), 4.25 (0.5H,dd), 4.4-5.1 (4H,m), 6.8 (1H,2xd), 7.1-7.3 (5H,m), 7.3-7.6 (2H,m), 7.7-7.8 (3H,m), 8.3-8.45 (1H,m), 8.5-8.65 (1H,m), 9.0 (1H,s), 12.3-12.8 (b), 14.0-14.4 (2H,b).
Analysis: $C_{41}H_{54}N_6O_8.HCl.3H_2O$ requires C,58.0; H,7.2; N,9.9%. Found: C,57.8; H,6.8; N,9.5%.
M.S. (m/z)(FAB)(M+1) = 759 (consistent with m.w. = 758)

Example 25

4-(S)-((2,3-Dihydro-1,1-dioxobenzothiophene-3-yl)acetyl-(S)-phenylalanyl-(S)-histidyl)amino-5-cyclohexyl-3-(S)-hydroxypentanoic acid isobutylamide sesquihydrate

This material was formed from (2,3-dihydro-1,1-dioxo benzothiophene-3-yl)acetic acid (45 mg) and 4-(S)-((S)-phenylalanyl-(S)-histidyl)amino-5-cyclohexyl-3-(S)-hydroxypentanoic acid isobutylamide (100 mg), following the procedure of Example 23. The crude product was chromatographed on silica gel using methanol/chloroform (0-10% methanol, gradient). This gave 4-(S)-((2,3-dihydro-1,1-dioxobenzothiophene-3-yl)acetyl-(S)-phenyla lanyl-(S)-histidyl)amino-5-cyclohexyl-3-(S)-hydroxypent anoic acid isobutylamide sesquihydrate (50 mg) as a white solid.
NMR (δ) ($CD_3OD$) 0.8-1.0 (8H, m), 1.1-1.5 (6H, m), 1.55-1.85 (6H, m), 2.15-2.3 (2H, m), 2.45-2.6 (1H, m), 2.8-2.95 (3H, m), 2.95-3.15 (4H, m), 3.8-4.0 (3H, m), 4.5-4.75 (3H, m), 6.8 and 6.9 (1H), 7.2-7.7 (10H, m).

Analysis: $C_{40}H_{54}N_6O_7S.1.5H_2O$ requires C,60.8; H,7.3; N,10.6%. Found: 61.0; H,7.1; N,10.6%.
M.S. (m/z) (FAB) (M+1)=763 (consistent with m.w.=762).

## Example 26

4-(S)-((5-(N-(2-Hydroxyethyl)iminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide sesquihydrate

4-(S)-((5-Formyl-2,3-dihydrobenzofuran-3-yl)acetyl-(S)-phenylalanyl-(S)-leucyl)amino-5-cyclohexyl-3-(S)-hydroxypentanoic acid isobutylamide (0.20g), malonic acid (0.033g), and ethanolamine (0.035 ml) were stirred in ethanol (2 ml) for 16h. The gelatinous precipitate was redissolved by addition of chloroform and ethanol, and solvents were then removed to give an oil. This was dissolved in chloroform, and the product was precipitated by addition of petroleum ether (b.p. 60-80°), leaving the precipitate to solidify for 2 weeks before isolation by filtration, washing with petroleum ether (b.p. 60-80°C), and drying *in vacuo*. This gave 4-(S)-((5-(N-(2-hydroxyethyl)iminomethyl)-2,3- dihydrobenzofuran-3-yl)acetyl-(S)-phenylalanyl-(S)-leucyl)-amino-5-cyclohexyl-3-(S)-hydroxypentanoic acid isobutylamide sesquihydrate (0.113g), as a light yellow powder.
NMR ($\delta$) ($DMSOd_6$) 0.7-1.0 (13H, m), 1.0-1.8 (16H, m), 2.1 (2H, m), 2.2-2.35 (1H, m), 3.85 (2H, m), 4.1-4.7 (5H, m), 4.9 (1H, b), 6.8 (1H, 2xd), 7.1-7.3 (5H, m), 7.3-7.6 (3H, m), 7.75 (1H, m), 8.15-8.3 (3H, m). Analysis: $C_{43}H_{63}N_5O_7.1.5H_2O$ requires C, 65.5; H, 8.4; N, 8.9%. Found: C, 65.3; H, 8.3; N, 8.9%.
M.S. (m/z) (FAB) (M+1)=762 (consistent with m.w.=761).

## Example 27a

4-(S)-((5-(N-(2-Hydroxyethyl)aminomethyl)-2,3-dihydrobenzofuran-3-yl)acetyl-(S)-phenylalanyl-(S)-leucyl)-amino-5-cyclohexyl-3-(S)-hydroxvpentanoic acid isobutylamide

4-(S)-((5-(N-(2-Hydroxyethyl)iminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide (80 mg) was stirred in ethanol (4 ml) with sodium borohydride (20 mg) for 1h. The mixture was poured into water, and extracted into chloroform. The extract was dried ($Na_2SO_4$) and evaporated. The crude product was chromatographed on silica gel using methanol/chloroform (0-5% methanol, gradient). This gave 4-(S)-((5-(N-(2-hydroxyethyl)aminomethyl)-2,3-dihydrobenzofuran-3-yl)acetyl-(S)-phenylalanyl-(S)-leucyl)amino-5-cyclohexyl-3-(S)-hydroxypentanoic acid isobutylamide (20 mg) as a white solid.
NMR ($\delta$) ($DMSOd_6$) 0.7-1.0 (13H, m), 1.0-1.8 (16H, m), 2.05-2.3 (3H, m), 2.55 (1H, dd), 2.7-2.95 (4H, m), 3.05 1H, m), 3.6 (1H, m), 3.75-3.9 (2.5H, m), 4.05 (0.5H, dd), 4.25-4.4 (4H, m), 4.6 (1H, m), 4.9 (1H, t), 4.95 (1H, q), 6.65 (1H, 2xd), 7.0-7.1 (2H, 2xd), 7.1-7.4 (6H, m), 7.7 (1H, t), 8.2-8.35 (2H, m).
M.S. (m/z) (FAB) (M+1)=764 (consistent with m.w.=763).

## Example 27b

4-(S)-((5-(N-(2-Hydroxyethyl)aminomethyl)-2,3-dihydrofuran-3-yl)acetyl-(S)-phenylalanyl-(S)-leucyl)amino-5-cyclohexyl-3-hydroxypentanoic acid isobutylamide hydrochloride

This material was formed from the corresponding free base (Example 27a) (26 mg), following the procedure of Example 20(b). This gave 4-(S)-((5-(N-(2-hydroxyethyl)aminomethyl)-2,3-dihydrobenzofuran-3-yl)acetyl-(S)-phenylalanyl-(S)-leucyl)amino-5-cyclohexyl-3-hydroxypentanoic acid isobutylamide hydrochloride (18 mg) as a white powder.
NMR ($\delta$) ($DMSOd_6$) 0.7-1.0 (13H,m), 1.0-1.45 (7H,m), 1.45-1.7 (8H,m), 1.75 (1H,d), 2.1 (2H,m), 2.2-2.35 (1H m,), 2.65-3.0 (6H,m), 3.0-3.1 (1H,m), 3.65 (3H,m), 3.8 (3H,m), 3.95-4.1 (2.5H,m), 4.15 (0.5H,dd), 4.3-4.5 (2H,m), 4.6 (1H,m), 4.9 (1H,m), 5.2 (1H,m), 6.8 (1H,2xd), 7.15-7.3 (m), 7.4 (1H,d), 7.7 (1H,t), 8.15-8.35 (2H,m), 8.8 (2H,b).

M.S. (m/z) (FAB) (M + 1) = 764 (consistent with m.w. = 763)

Example 28

4-(S)-((6-Nitro-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide hydrate

This material was formed from (6-nitro-2,3-dihydro1,1-dioxobenzothiophene-3-yl)acetic acid (152 mg) and 4-(S)-((S)-phenylalanyl-(S)-leucyl)amino-5-cyclohexyl-3-(S)-hydroxypentanoic acid isobutylamide (288 mg) by the procedure described in Description 2(c). This afforded 4-(S)-((6-nitro-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide hydrate (264 mg).
NMR (δ) ($CDCl_3$) 0.7-0.9 (14H, m), 1.0-1.3 (4H, m), 1.3-1.8 (14H, m), 2.2-2.4 (2H, m), 2.5-3.7 (7H, m), 3.9-4.2 (2H, m), 4.4-5.1 (2H, m), 6.4-7.6 (10H, m), 8.2-8.6 (2H, m).
Analysis: $C_{40}H_{51}N_5O_9S.H_2O$ requires C, 59.9; H, 7.4; N, 8.7%. Found: C, 60.1; H, 7.3; N, 8.8%.
M.S. (m/z) (FAB) (M + H) = 784 (consistent with m.w. = 783).

Example 29

4-(S)-((6-Amino-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide dihydrate

A solution of 4-(S)-((6-nitro-2,3-dihydro-1,1-dioxobenzothiophene-3-yl)acetyl-(S)-phenylalanyl-(S)-leucyl)-amino-5-cyclohexyl-3-(S)-hydroxypentanoic acid isobutylamide hydrate (260 mg) in ethanol (100 ml) was hydrogenated at room temperature and pressure in the presence of 10% palladium on carbon for 4h. The catalyst was removed by filtration through Kieselguhr and the filtrate evaporated to dryness in vacuo to give 4-(S)-((6-amino-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide dihydrate (153 mg).
NMR (δ) ($CDCl_3$) 0.7-1.7 (29H, m), 1.7-2.0 (m), 2.2-2.8 (4H, m), 2.8-3.3 (5H, m), 3.4-5.0 (8H, m), 6.4-7.4 (13H, m).
Analysis: $C_{40}H_{59}N_5SO_7.2H_2O$ requires C, 60.8; H, 7.5; N, 8.9%. Found: C, 61.1; H, 7.5; N, 9.2%.
M.S. (m/z) (FAB) (M + 1) = 754 (consistent with m.w. = 753).

Example 30

4-(S)-((6-Cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide

This material was formed from (6-cyano-2,3-dihydro-1,1-dioxobenzothiophene-3-yl)acetic acid (289 mg) and 4-(S)-((S)-phenylalanyl-(S)-leucyl)amino-5-cyclohexyl-3-(S)-hydroxypentanoic acid isobutylamide (706 mg) by the procedure described in Description 2(c). This afforded 4-(S)-((6-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide (358 mg).
NMR (δ) ($CD_3OD$) 0.7-2.0 (29H, m), 2.2-2.4 (2H, m), 2.4-4.0 (11H, m), 4.3-4.4 (2H, m), 7.2-8.2 (8H, m).
M.S. (m/z) (FAB) (M + 1) = 764 (consistent with m.w. = 763).

Example 31a

4-(S)-((6-Aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide dihydrate

This compound was formed from 4-(S)-((6-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobulylamide (270 mg) by the procedure in Example 20(a) to afford 4-(S)-((6-aminomethyl-2,3-dihydro-1,1-dioxobenzothiophene-3-yl)acetyl-(S)-phenylalanyl-(S)-leucyl)amino-5-cyclohexyl- 3-(S)-hydroxypentanoic acid isobutylamide dihydrate (97 mg).

NMR (δ) ($CD_3OD$) 0.7-2.0 (29H, m), 2.2-2.3 (2H, d), 2.4-3.2 (7H, m), 3.4-3.7 (2H, m), 3.8-4.0 (4H, m), 4.4 (1H, m), 7.2-7.7 (7H, m).

Analysis: $C_{41}H_{61}N_5SO_7.2H_2O$ requires C, 61.2; H, 8.2; N, 8.7%. Found: C, 61.5; H, 7.9; N, 8.4%.

M.S. (m/z) (FAB) (M+1)=768 (consistent with m.w.=767).

Example 31b

4-(S)-((6-Aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide hydrochloride

4-(S)-((6-Aminomethyl-2,3-dihydro-1,1-dioxobenzothiophene-3-yl)acetyl-(S)-phenylalanyl-(S)-leucyl)-amino-5-cyclohexyl-3-(S)-hydroxypentanoic acid isobutylamide dihydrate (97 mg) was taken up in methanol (2 ml), acidified with hydrochloric acid (0.5M, 5 ml) and further diluted with water (100 ml). Removal of the solvent by freeze-drying afforded 4-(S)-((6-aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide hydrochloride (87 mg).

NMR (δ) ($CD_3OD$) 0.8-2.1 (29H, m), 2.2-2.4 (2H, m), 2.4-4.0 (11H, m), 4.2 (2H, bs), 4.3-4.4 (2H, m), 7.2-7.8 (8H, m).

Example 32

4-(S)-((2,3-Dihydro-1,1-dioxobenzothiophene-2-yl)acetyl-(S)-phenylalanyl-(S)-leucyl)amino-5-cyclohexyl-3-(S)-hydroxypentanoic acid isobutylamide hemihydrate

This material was formed from (1,1-dioxo-2,3-dihydrobenzothiophene-2-yl)acetic acid (79 mg) and 4-(S)-((S)-phenylalanyl-(S)-leucyl)amino-5-cyclohexyl-3-(S)-hydroxypentanoic acid isobutylamide (150 mg) by the procedure described in Description 2(c). This afforded 4-(S)-((2,3-dihydro-1,1-dioxobenzothiophene-2-yl)-acetyl-(S)-phenylalanyl-(S)-leucyl)amino-5-cyclohexyl-3-(S)-hydroxypentanoic acid isobutylamide hemihydrate (120 mg) as a white foam.

NMR (δ) ($CDCl_3$) 0.7-2.0 (33H, m), 2.2-4.6 (13H, m), 6.0-7.8 (12H, m).

Analysis: $C_{40}H_{58}N_4O_7S.\frac{1}{2}H_2O$ requires C, 64.2; H, 8.0; N, 7.5%. Found: C, 64.1; H, 7.9; N, 7.8%.

M.S. (m/z) (FAB) (M+1)=739 (consistent with m.w.=738).

Example 33

4-(S)-((2,3-Dihydro-2-oxo-1H-indol-3-yl)acetyl-(S)-phenylalanyl-(S)-leucyl)amino-5-cyclohexyl-3-(S)-hydroxypentanoic acid isobutylamide hydrate

This material was formed from 2,3-dihydro-2-oxo-1H-indole-3-acetic acid * (74 mg) and 4-(S)-((S)-phenylalanyl-(S)-leucyl)amino-5-cyclohexyl-3-(S)-hydroxypentanoic acid isobutylamide trifluoroacetate (250 mg) as described in Description 2(c). This gave 4-(S)-((2,3-dihydro-2-oxo-1H-indol-3-yl)acetyl-(S)-phenylalanyl-(S)-leucyl)amino-5-cyclohexyl-3-(S)-hydroxypentanoic acid isobutylamide hydrate (132 mg).

NMR (δ) ($CDCl_3$) 0.6-2.1 (33H, m), 2.2-3.3 (7H, m), 3.7-4.6 (4H, m), 6.6-8.9 (14H, m).

M.S. (m/z) (FAB) (M+1)=704, (M-$H_2O$+1)=686 (consistent with m.w.=703).

*See Synthesis 1979, 276-7.

Example 34

4-(S)-((5-(Ethoxycarbonylmethylaminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide hemihydrate

To a stirred solution of 4-(S)-((5-formyl-2,3-dihydrobenzofuran-3-yl)acetyl-(S)-phenylalanyl-(S)-leucyl)-amino- 5-cyclohexyl-3-(S)-hydroxypentanoic acid isobutylamide hemihydrate (83 mg), prepared as in Example 13, in methanol (3 ml) was added ethyl glycinate hydrochloride (16 mg) followed by N-ethylmorpholine (0.015 ml). After 0.25h sodium cyanoborohydride (8 mg) was added in three portions over 0.1h. After 18h concentrated hydrochloric acid (0.2 ml) was added and the reaction mixture concentrated under reduced pressure. The residue was taken up in water, washed with diethyl ether and the aqueous layer basified with 10% sodium hydroxide. The precipitated solid was extracted into chloroform (3x50 ml) and the combined extracts dried over anhydrous sodium sulphate. Removal of the solvent under reduced pressure and purification of the residue by column chromatography on silica gel using 0-3% methanol/chloroform afforded 4-(S)-((5-(ethoxycarbonylmethylaminomethyl)-2,3-dihydrobenzofuran-3-yl)-acetyl-(S)-phenylalanyl-(S)-leucyl)amino-5-cyclohexyl-3-(S)-hydropentanoic acid isobutylamide hemihydrate (48 mg).

NMR (δ) ($CDCl_3$) 0.8-2.0 (32H, m), 2.2-2.8 (4H, m), 2.9-4.9 (20H, m), 6.2-7.5 (9H, m).

Analysis: $C_{45}H_{67}N_5O_8.\frac{1}{2}H_2O$ requires C, 66.3; H, 8.4; N, 8.6%. Found: C, 66.2; H, 8.2; N, 8.2%.

M.S. (m/z) (FAB) (M+1)=806 (consistent with m.w.=805).

Example 35

4-(S)-((5-(Benzyloxycarboxylmethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide hydrate

This material was formed from 4-(S)-((5-formyl-2,3-dihydrobenzofuran-3-yl)acetyl-(S)-phenylalanyl-(S)-leucyl)amino-5-cyclohexyl-3-(S)-hydroxypentanoic acid isobutylamide hemihydrate (520 mg) and benzyl glycinate p-toluenesulphonic acid salt (470 mg) as described in Example 34 except that 4A powdered molecular sieve (4g) was added to the reaction mixture and triethylamine was used instead of N-ethylmorpholine. This gave 4-(S)-((5-(benzyloxycarbonylmethylaminomethyl)-2,3-dihydrobenzofuran-3-yl)-acetyl-(S)-phenylalanyl-(S)-leucyl)amino-5-cyclohexyl-3-(S)-hydroxypentanoic acid isobutylamide hydrate (380 mg).

NMR (δ) ($CDCl_3/CD_3OD$) 0.5-2.8 (33H, m), 2.9-4.6 (15H, m), 5.2 (2H, s), 6.5-7.9 (12H, m).

Analsis: $C_{50}H_{69}N_5O_8.H_2O$ requires C, 67.8; H, 8.1; N, 7.9%. Found: C, 67.7; H, 8.0; N, 8.0%. M.S. (m/z) (FAB) (M+1)=869, ($M-H_2NCH_2CO_2Bz+1$)=704 (consistent with m.w.868).

Example 36a

4-(S)-((5-(Carboxymethylaminomethyl)-2,3-dihydrobenzofuran-3-yl)acetyl-(S)-phenylalanyl-(S)-leucyl)amino-5-cyclohexyl-3-(S)-hydroxvoentanoic acid isobutylamide

10% Sodium hydroxide (0.054 ml) was added to a solution of 4-(S)-((5-(ethoxycarbonylmethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate (100 mg) in ethanol (5 ml) and the resultant mixture stirred at room temperature for 18h. The ethanol was removed under reduced pressure and the residue was washed with diethyl ether, neutralised with 10% citric acid and extracted into ethyl acetate (3x20 ml). Combined extracts were dried over anhydrous sodium sulphate and the solvent removed uner reduced pressure to afford 4-(S)-((5-(carboxymethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide (43 mg).

NMR (δ) ($CD_3OD$) 0.6-1.9 (29H, m), 2.1-3.1 (9H, m), 3.4-4.7 (11H, m), 6.6-7.6 (9H, m).

M.S (m/z) (FAB) (M+1)=778 (consistent with m.w.=777).

Example 36b

4-(S)-((5-(Carboxymethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide sodium salt

A mixture of 4-(S)-((5-(carboxymethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide (35 mg) and aqueous sodium hydroxide (1.56 ml of a 1.15 mg/ml solution) in water (20 ml) and methanol (2 ml) was freeze-dried to afford 4-(S)-((5-(carboxylmethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide sodium salt (35 mg).
NMR (δ) (DMSO$d_6$) 0.7-1.8 (31H, m), 2.1-2.3 (2H, m), 2.7-3.5 (10H, m), 6.8-7.5 (8H, m).

Example 37

4-(S)-((2,3-Dihydro-1,1-dioxobenzothiophene-3-yl)-acetyl-(S)-phenylalanyl-(S)-β-(pyrazol-1-yl)alanyl)amino-5-cyclohexyl-3-(S)-hydroxypentanoic acid isobutylamide hemihydrate

This material was formed from (2,3-dihydro-1,1-dioxobenzothiophene-3-yl)acetic acid (46 mg) and 4-(S)-((S)-phenylalanyl-(S)-β-(pyrazol-1-yl)alanyl)amino-5-cyclohexyl-3-(S)-hydroxypentanoic acid isobutylamide hydrobromide (73 mg) as described in Description 8(c) except that triethylamine was used instead of diisopropylethylamine. Chromatography on silica gel using 0-3% methanol/chloroform gave 4-(S)-(2,3-dihydro-1,1-dioxobenzothiophene-3-yl)acetyl-(S)-phenylalanyl(S)-β-(pyrazol-1-yl)alanyl)amino-5-cyclohexyl-3-(S)-hydroxypentanoic acid isobutylamide (32 mg).
NMR (δ) ($CD_3OD$) 0.7-1.9 (24H, m), 2.1-3.1 (8H, m), 3.8-4.75 (6H, m), 6.3 (1H, m), 7.2-7.8 (11H, m).
Analysis: $C_{40}H_{54}N_6O_7S.0.5H_2O$ requires C, 62.2; H, 7.2; N, 10.9%. Found: C, 62.0; H, 7.1; N, 10.5%.
M.S. (m/z) (FAB) (M + 1) = 763 (consistent with m.w. = 762).

Example 38

4-(S)-((2,3-Dihydro-1,1-dioxobenzothiophene-3-yl)-acetyl-(S)-phenylalanyl-(S)-β-(4-methylpyrazol-1-yl)-alanyl)amino-5-cyclohexyl-3-(S)-hydroxypentanoic acid isobutylamide hydrate

This material was formed from (2,3-dihydro-1,1-dioxobenzothiophene-3-yl)acetyl-(S)-phenylalanine (192 mg) and 4-(S)-((S)-β-(4-methylpyrazol-1-yl)alanyl)amino-5-cyclohexyl-3-(S)-hydroxypentanoic acid isobutylamide dihydrobromide as described in Description 2(a). The crude product was chromatographed on silica gel using 0-3% methanol/chloroform to afford 4-(S)-(2,3-dihydro-1,1-dioxobenzothiophene-3-yl)-acetyl-(S)-phenylalanyl-(S)-β-(4-methylpyrazol-1-yl)alanyl)amino-5-cyclohexyl-3-(S)-hydroxypentanoic acid isobutylamide hydrate (100 mg).
NMR (δ) (DMSO$d_6$) 0.6-1.8 (20H, m), 1.9-2.2 (5H, m), 2.3-3.1 (7H, m), 3.6-5.0 (8H, m), 7.0-8.6 (15H, m).
Analysis: $C_{41}H_{56}N_6O_7S.H_2O$ requires C, 62.0; H, 7.4; N, 10.6%. Found: C, 62.0; H, 7.1; N, 10.2%.
M.S. (m/z) (FAB) (M + 1) = 777 (consistent with m.w. = 776).

Example 39

4-(S)-((5-Nitro-2,3-dihydrobenzofuran-3-yl)acetyl-(S)-phenylalanyl-(S)-histidyl)amino-5-cyclohexyl-3-(S)-hydroxypentanoic acid isobutylamide hydrate

This material was formed from (5-nitro-2,3-dihydrobenzofuran-3-yl)acetyl-(S)-phenylalanyl-(S)-histidine (0.34g) and 4-(S)-amino-5-cyclohexyl-3-(S)-hydroxypentanoic acid iscbutylamide as in Example 13 except that the citric acid wash was omitted. Chromatography was performed using chloroform with 0 - 8% methanol as eluent. This gave 4-(S)-((5-nitro-2,3-dihydrobenzofuran-3-yl)acetyl-(S)-phenylalanyl-(S)-histidyl)-

amino-5-cyclohexyl-3-(S)-hydroxypentanoic acid isobutylamide hydrate (0.42 g).
NMR (δ) (DMSOd$_6$) 0.7-2.0 (22H, m), 3.8-4.0 (4H, b), 4.0-5.2 (7H).
Analysis: $C_{40}H_{53}N_7O_8.H_2O$ requires C, 61.8; H, 7.1; N, 12.6%. Found: C, 61.7; H, 6.8; N, 12.7%.
M.S. (m/z) (FAB) (M+1)=760 (consistent with m.w.=759).

Example 40

4-(S)-((5-Amino-2,3-dihydrobenzofuran-3-yl)acetyl-(S)-phenylalanyl-(S)-histidyl)amino-5-cyclohexyl-3-(S)-hydroxypentanoic acid isobutylamidehemi-(trichloromethanate)

This material was formed from 4-(S)-((5-nitro-2,3-dihydrobenzofuran-3-yl)acetyl-(S)-phenylalanyl-(S)-histidyl)amino-5-cyclohexyl-3-hydroxypentanoic acid isobutylamide (200 mg) using the procedure of Example 6, but omitting trifluoroacetic acid. This gave 4-(S)-((5-amino-2,3-dihydrobenzofuran-3-yl)acetyl-(S)-phenylalanyl-(S)-histidyl)amino-5-cyclohexyl-3-(S)-hydroxypentanoic acid isobutylamidehemi-(trichloromethanate).
NMR (δ) (DMSOd$_6$) 0.7-2.0 (22H, m), 3.8 (2H, b), 6.3-6.5 (3H, m), 6.8 (1H, m), 7.1-7.4 (6H, m), 7.5 (1H, s), 7.8 (1H, b), 8.3-8.5 (2H, m).
Analysis: $C_{40}H_{55}N_7O_6$ 0.5 ($CHCl_3$) requires C, 61.5 ; H, 7.0; N, 12.4%. Found: C, 61.5; H, 7.2; N, 12.2%.
M.S. (m/z) (FAB) (M+1)=730 (consistent with m.w.=729).

Example 41

4-(S)-(2,3-Dihydro-3-oxo-1H-isoindol-1-acetyl)-(S)-phenylalanyl-(S)-leucyl)amino-5-cyclohexyl-3-(S)-hydroxypentanoic acid isobutylamide hydrate

This material was formed from 2,3-dihydro-3-oxo-1H-isoindole-1-acetic acid (48 mg), following the procedure of Example 21. The crude product was chromatographed on silica gel using methanol/chloroform (0-5% methanol, gradient). This gave the title compound (92 mg) as a white solid.
NMR (δ) (DMSOd$_6$) 0.7-1.0 (14H,m), 1.0-1.45 (6H,m), 1.45-1.85 (9H,m), 2.1 (2H,m), 2.3-2.5 (2H,m), 2.7-2.95 (3H,m), 2.95-3.1 (1H,m), 3.75-3.9 (2H,m), 4.35 (1H,q), 4.6-4.95 (3H,m), 7.05-7.85 (11H,m), 8.2-8.5 (3H,m).
Analysis: $C_{40}H_{57}N_5O_6.H_2O$ requires C,66.6; H,8.2; N,9.7%. Found: C,66.8; H,8.1; N,9.4%.
M.S. (m/z) (FAB)(M+1) = 704 (consistent with m.w. = 703)

Example 42

Disulphide of 4-(S)-((5-mercapto-2,3-dihydrobenzofuran-3-yl)acetyl-(S)-phenylalanyl-(S)-leucyl)amino-5-cyclohexyl-3-(S)-hydroxypentanoic acid isobutylamide

This material was formed from the disulphide of (5-mercapto-2,3-dihydrobenzofuran-3-yl)acetic acid (60 mg), following the procedure of Example 21. The crude product was chromatographed on silica gel using methanol/chloroform (0-5% methanol, gradient). This gave the title compound (64 mg) as a white solid.
NMR (δ) ($CD_3OD$) 0.75-1.0 (26H,m), 1.05-1.85 (32H,m), 2.3 (4H,d), 2.3-2.5 (2H,m), 2.5-2.65 (2H,m), 2.85-3.25 8H,m), 3.7 (2H,m), 3.85-4.0 (5H,m), 4.2 (1H,m), 4.3-4.45 (4H,m), 4.55 (q), 6.65 (2H,m), 7.15-7.35 (14H, m).
M.S. (m/z) (FAB) (M+1) = 1443 (consistent with m.w. = 1442).

Example 43

4-(S)-((5-(tert-Butoxycarbonylmethanesulphonyl)-2,3-dihydrobenzofuran-3-yl)acetyl)-(S)-phenylalanyl-(S)-leucyl)amino-5-cyclohexyl-3-(S)-hydroxypentanoic acid isobutylamide hemihydrate

This material was formed from (5-(tert-butoxycarbonylmethanesulphonyl)-2,3-dihydrobenzofuran-3-yl)-acetyl-(S)-phenylalanyl-(S)-leucine (260 mg), following the procedure of Example 13. The crude product was chromatographed on silica gel using methanol/chloroform (0-5% methanol, gradient). This gave the title compound (272 mg) as a white solid.

NMR (δ) ($DMSOd_6$) 0.7-1.0 (14H,m), 1.05-1.9 (24H,m), 2.0-2.2 (2H,m), 2.2-2.4 (1H,m), 2.6-3.2 (5H,m), 3.6-4.0 (4H,m), 4.2-4.8 (5H,m), 4.8-5.0 (1H,m), 6.95-7.1 (1H,m), 7.15-7.5 (6H,m), 7.6-7.9 (3H,m), 8.1-8.4 (2H,m).

Analysis: $C_{46}H_{68}N_4O_{10}S.0.5H_2O$ requires C,62.9; H,7.9; N,6.4%. Found: C,62.8; H,7.8; N,6.3%.

M.S. (m/z) (FAB) (M+1) = 869 (consistent with m.w. = 868).

## Example 44

**4-(S)-(3-(2,3-Dihydro-1,1-dioxobenzothiophene-3-yl)-propanoyl-(S)-phenylalanyl-(S)-leucyl)amino-5-cyclohexyl-3-(S)-hydroxypentanoic acid isobutylamide hemihydrate**

This material was formed from 3-(2,3-dihydro-1,1-dioxobenzothiophene-3-yl)propanoic acid (145 mg) and 4-(S)-((S)-phenylalanyl-(S)-leucyl)amino-5-cyclohexyl-3-(S)-hydroxypentanoic acid isobutylamide (350 mg) by the procedure in Description 2(c). This gave the title compound (190 mg).

NMR (δ) ($CD_3OD$) 0.7-1.4 (21H,m), 1.4-1.9 (11H,m), 2.1-2.4 (5H,m), 2.8-3.6 (7H,m), 3.9-4.0 (2H,m), 4.3-4.4 (1H,m), 7.1-7.7 (9H,m).

Analysis $C_{41}H_{60}N_4SO_7.0.5H_2O$ requires C,64.6; H,8.1; N,7.3%, Found: C,64.5; H,8.0; N,7.3%.

M.S. (m/z) (FAB) (M+1) = (753) (consistent with m.w. = 752)

## Example 45

**4-(S)-((5-Cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide**

This material was formed from (5-cyano-2,3-dihydro-1,1-dioxobenzothiophene-3-yl)acetic acid (80 mg) and 4-(S)-((S)-phenylalanyl-(S)-leucyl)amino-5-cyclohexyl-3-(S)-hydroxypentanoic acid isobutylamide (200 mg) by the procedure in Description 2(c). This gave the title compound (109 mg).

NMR (δ) ($CD_3OD$) 0.7-2.3 (31H,m), 2.8-3.1 (4H,m), 3.8-4.0 (2H,m), 7.2-7.4 (5H,m), 7.8-8.2 (3H,m).

M.S. (m/z) (FAB) (M+1) = 764 (consistent with mw = 763)

## Example 46

**4-(S)-((2,3-Dihydro-3-oxo-1H-isoindol-1-ylidine)acetyl-(S)-phenylalanyl-(S)-leucyl)amino-5-cyclohexyl-3-(S)-hydroxypentanoic acid isobutylamide hemihydrate**

This material was formed from (2,3-dihydro-3-oxo-1H-isoindol-1-ylidine)acetic acid (32 mg), following the procedure of Example 21. The crude product was chromatographed on silica gel using methanol/chloroform (0-5% methanol, gradient). This gave the title compound (82 mg) as a straw-coloured solid.

NMR (δ) ($DMSOd_6$) 0.7-1.0 (14H,m), 1.0-1.4 (6H,m), 1.4-1.8 (9H,m), 2.1 (2H,m), 2.75-2.95 (3H,m), 3.1 (1H, dd), 3.85 (2H,m), 4.3 (1H,g), 4.7 (1H,m), 4.9 (1H,m), 6.1 (1H,s), 7.15 (1H,m), 7.2-7.35 (4H,m), 7.4 (1H,d), 7.6-7.85 (5H,m), 8.3 (1H,d), 8.55 (1H,d), 10.25 (1H,s).

Analysis: $C_{40}H_{55}N_5O_6.0.5H_2O$ requires C,67.6; H, 7.9; N, 9.8%. Found: C, 67.6; H8.0; N, 9.8%. M.S. (m/z) (FAB) (M+1) = 702 (consistent with m.w. = 701)

## Example 47

4-(S)-((2,3-Dihydro-1,1-dioxobenzothiophene-3-yl)-acetyl-(S)-phenylalanyl-(S)-β-(3,5-dimethylpyrazol-1-yl)-alanyl)amino-5-cyclohexyl-3-(S)-hydroxypentanoic acid isobutylamide dihydrate

This material was formed from (2,3-dihydro-1,1-dioxobenzothiophene-3-yl)acetyl-(S)-phenylalanine (0.214g) and 4-(S)-((S)-β-(3,5-dimethylpyrazol-1-yl)alanyl) amino-5-cyclohexyl-3-(S)-hydroxypentanoic acid isobutylamide dihydrobromide (0.25g) as in Description 2(a). Purification by chromatography on silica gel using 0-2% methanol in chloroform gave the title compound (0.17g).
NMR (δ) ($CD_3OD$) 0.7-2.3 (23H,m), 2.7-3.0 (4H,m), 3.8-4.4 (3H,m), 5.8-5.9 (1H,m), 7.1-7.7 (9H,m).
Analysis $C_{42}H_{58}N_6O_7S.2H_2O$ requires C,61.0; H, 7.6; N, 10.1%. Found: C,61.3; H,7.2; N,10.1%.
M.S. (m/z) (FAB) (M+1) = 791 (consistent with mw = 790)

Example 48

4-(S)-((2,3-Dihydro-1,1-dioxobenzothiophene-3-yl)-acetyl-(S)-phenylalanyl-(S)-β-(tert-butoxycarbonylamino)-alanyl) amino-5-cyclohexyl-3-(S)-hydroxypentanoic acid isobutylamide sesquihydrate

This material was formed from (2,3-dihydro-1,1-dioxobenzothiophene-3-yl)acetyl-(S)-phenylalanine (0.098g) and 4-(S)-((S)-β-(tertbutoxycarbonylamino)alanyl)amino-5-cyclohexyl-3-(S)-hydroxypentanoic acid isobutylamide (0.120g) as in Description 2(a) using dimethylformamide as solvent. Purification by chromatography on silica gel using 0-2% methanol in chloroform gave the title compound (0.120g).
NMR (δ) ($CD_3OD$) 0.7-1.0 (7H,bd), 1.45 (9H,s), 2.3 (2H,m), 3.0 (2H,m), 3.8-4.1 (3H,m), 7.1-7.8 (9H,m).
Analysis: $C_{42}H_{61}N_5O_9S.1.5H_2O$ requires C,60.1; H,7.7; N,8.4%. Found: C,60.0; H,7.3; N,8.4%.
M.S. (m/z) (FAB) (M+1) = 812 (consistent with m.w. 811)

Example 49

4-(S)-((2,3-Dihydro-1,1-dioxobenzothiophene-3-yl)-acetyl-(S)-phenylalanyl-(S)-β-aminoalanyl)amino-5-cyclohexyl-3-(S)-hydroxypentanoic acid isobutylamide trifluoroacetate hydrate

To a solution of 4-(S)-((2,3-dihydro-1,1-dioxobenzothiophene-3-yl)acetyl-(S)-phenylalanyl-(S)-β-(tertbutoxycarbonylamino)alanyl)amino-5-cyclohexyl-3-(S)-hydroxypentanoic acid isobutylamide (80 mg) in dry dichloromethane at 0°C was added pre-cooled trifluoroacetic acid dropwise. Stirring was continued at 0° for 1 h. The solvent was removed under reduced pressure and the product triturated with ether to give the title compound as a white solid (70 mg).
NMR (δ) ($CD_3OD$) 0.9-1.9 (20H,m), 2.3 (2H,d), 2.6 (1H,dd), 3.1 (2H,m), 3.9-4.1 (2H,m), 7.2-7.8 (8H,m).
Analysis $C_{39}H_{54}N_5O_9SF_3.H_2O$ requires C,55.5; H,6.7; N,8.3%. Found C,55.4; H,6.6; N,8.3%.
M.S. (m/z) (FAB) (M+1) = 712 (consistent with m.w. of free base = 711).

Example 50a

4-(S)-((5-Aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide

This material was formed from 4-(S)-((5-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide (0.300g) following the procedure of Example 20(a). Purification by column chromatography gave the title compound as a slightly coloured solid (0.202g).
NMR (δ) ($CD_3OD$) 0.8-2.0 (29H, m) 2.2-2.3 (2H, d) 3.8-4.0 (4H, m) 7.2-7.4 (5H, m) 7.5 (2H, m) 7.6-7.7 (1H, m).

Example 50b

4-(S)-((5-Aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide hydrochloride trihydrate

This material was formed from 4-(S)-((5-aminomethyl-2,3-dihydro-1,1-dioxobenzothiophene-3-yl)-acetyl-(S)-phenylalanyl-(S)-leucyl)amino-5-cyclohexyl-3-(S)-hydroxypentanoic acid isobutylamide (0.200g) following the procedure of Example 31(b). This gave the title compound as a white solid (0.192g).
NMR (δ) ($CD_3OD$) 0.7-1.9 (29H, m), 2.2-2.3 (2H, m), 2.7-3.1 (5H, m), 4.2 (2H, m), 4.3-4.4 (2H, m), 7.2-7.3 (5H, m), 7.5-7.6 (2H, m), 7.6-7.7 (1H, m).
Analysis: $C_{41}H_{61}N_5SO_7$. HCl. $3H_2O$ requires C, 57.4; H, 7.9; N, 8.2%. Found: C, 57.4; H, 7.6; N, 8.0%.
M.S. (m/z) (FAB) (M+1) = 769 (consistent with m.w. of free base = 768).

## Example 51

4-(S)-(3-(2,3-Dihydro-1,1-dioxobenzothiophene-2-yl)propanoyl-(S)-phenylalanyl-(S)-leucyl)amino-5-cyclohexyl-3-(S)-hydroxypentanoic acid isobutylamide hemihydrate.

This material was formed from 3-(2,3-dihydro-1,1-dioxobenzothiophene-2-yl)propanoic acid (0.09g) and 4-(S)-((S)-phenylalanyl-(S)-leucyl)amino-5-cyclohexyl-3-(S)-hydroxypentanoic acid isobutylamide (0.195g) followingthe procedure of Description 2(c). This gave the title compound (0.095g).
NMR (δ) ($CD_3OD$) 0.7-1.8 (31H, m), 1.8-2.9 (11H, m), 6.9-7.3 (5H, m), 7.3-7.6 (4H, m).
Analysis: $C_{41}H_{60}N_4SO_7.0.5H_2O$ requires C, 64.6; H, 8.1; N, 7.3%. Found: C, 64.6; H, 8.0; N, 6.9%.
M.S. (m/z) (FAB) (M+1) = 753 (consistent with m.w. = 752).

## Example 52

4 (S)-((2,3-Dihydro-1,1-dioxobenzothiophene-3-yl)acetyl-(S)-phenylalanyl-(S)-β-(acetylamino)alanyl))amino-5-cyclohexyl-3-(S)-hydroxypentanoic acid isobutylamide

A solution of 4-(S)-(Nα-benzyloxycarbonyl-(S)-β-(acetylamino)alanyl)amino-5-cyclohexyl-3-(S)-hydroxypentanoic acid isobutylamide (0.5g) in ethanol (25ml) and acetic acid (1ml) was hydrogenated in the presence of 10% palladium on charcoal for 4 h. The catalyst was filtered off and the solvent evaporated in vacuo. The residue was dissolved in dry dimethylformamide (10ml) and (2,3-dihydro-1,1-dioxobenzothiophene-3-yl)acetyl-(S)-phenylalanine (0.29g) was added followed by 1-hydroxybenzotriazole (0.106g) and triethylamine (0.217ml). The mixture was cooled to 0°C and 1-(3-dimethylaminopropyl)-3-ethylcarbodiimide hydrochloride (0.15g) was added. After 20 h ethyl acetate (100ml) was added and the solution washed with saturated sodium hydrogen carbonate and brine. The organic layer was dried ($Na_2SO_4$) and evaporated in vacuo. The residue was purified by chromatography on silica gel using 0-3% methanol/chloroform to afford the title compound (0.03g).
NMR (δ) ($CD_3OD$) 0.8-2.0 (25H, m), 2.2-3.1 (6H, m), 3.4-3.6 (2H, m), 3.8-4.1 (3H, m), 4.1-4.7 (3H, m), 7.1-7.7 (9H, m)
M.S. (m/z) (FAB) (M+1) = 754 (consistent with m.w. = 753)

## Example 53

4-(S)-((2,3-Dihydro-1,1-dioxobenzothiophene-3-yl)acetyl-(S)-phenylalanyl-(S)-β-(2-oxopyrrolidin-1-yl)alanyl)-amino-5-cyclohexyl-3-(S)-hydroxypentanoic acid isobutylamide

This material was prepared from 4-(S)-(benzyloxycarbonyl-(S)-β-(2-oxopyrrolidin-1-yl)alanyl)amino-5-cyclohexyl-3-(S)-hydroxypentanoic acid isobutylamide (0.28g) and (2,3-dihydro-1,1-dioxobenzothiophene-3-yl)acetyl-L-phenylalanine (0.246g) by the procedure of Example 52 except that DMF was used as solvent for the second step. Chromatography on silica gel using 0-3% methanol/chloroform as eluent afforded the title compound as a white foam (0.278g)

M.S. (m/z) (FAB) (M+1) = 780 (consistent with m.w. = 779)

Example 54

4-(S)-((5-Cyano-2,3-dihydrobenzofuran-3-yl)acetyl-(S)-phenylalanyl-(S)-histidyl)amino-5-cyclohexyl-3-(S)-hydroxypentanoic acid isobutylamide hydrate

This material was formed from (5-cyano-2,3-dihydrobenzofuran-3-yl)acetyl-(S)-phenylalanyl-(S)-histidine (0.68g) and 4-(S)-amino-5-cyclohexyl-3-(S)-hydroxypentanoic acid isobutylamide (0.375g) following the procedure of Example 13. Chromatography on silica gel using 0-10% methanol/chloroform as eluent afforded the title compound as a white solid (0.42g).
NMR (δ) (DMSO$d_6$) 0.6-1.0 (8H, m), 1.0-1.9 (12H, m), 1.95-3.2 (10H, m), 3.5-5.1 (8H, m), 6.8-7.8 (10H, m), 8.2-8.5 (2H, m), 11.6-11.9 (1H, m).
Analysis: $C_{41}H_{53}N_7O_5.H_2O$ requires C, 65.0; H, 7.3; N, 12.9%. Found: C, 64.7; H, 7.2; N, 12.6%.
M.S. (m/z) (FAB) (M+1) = 740 (consistent with m.w. = 739).

Example 55

4-(S)-((6-(tert-Butoxycarbonylaminomethyl)-2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide sesquihydrate

This material was formed from (6-(tert-butoxycarbonylaminomethyl)-2,3-dihydro-1,1-dioxobenzothiophene-3-yl)acetic acid (108mg), following the procedure of Example 23. The crude product was purified by chromatography on silica gel using methanol/chloroform (0-10% methanol, gradient). This gave the title compound (147mg) as an off-white powder. NMR (δ) ($CD_3OD$) 0.8-1.0 (8H, m), 1.1-1.6 (15H, m), 1.6-1.9 (6H, m), 2.15-2.3 (2H, m), 2.45-2.6 (1H, m), 2.8-3.2 (7H, m) 3.2-3.6 (m), 3.8-4.05 (3H, m), 4.3 (2H, s), 4.5-4.9 (m), 6.95 (1H, d), 7.15-7.4 (6H, m), 7.4-7.7 (3H, m).
Analysis: $C_{46}H_{65}N_7O_9S$. $1.5H_2O$ requires C, 60.1; H, 7.5; N, 10.7%. Found: C, 60.4; H, 7.2; N, 10.7%.
M.S. (m/z) (FAB) (M+1) = 892 (consistent with m.w. = 891).

Example 56

4-(S)-((5-(Carboxymethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide, sodium salt

4-(S)-((5-(tert-Butoxycarbonylmethanesulphonyl)-2,3-dihydrobenzofuran-3-yl)acetyl-(S)-phenylalanyl- (S)-leucyl)amino-5-cyclohexyl-3-hydroxypentanoic acid isobutylamide (114mg) was dissolved in dichloromethane (4ml) and trifluoroacetic acid (4ml) was added. The mixture was left to stand for 3 h, and evaporated in vacuo. The residue was dissolved in methanol (2ml), and aqueous sodium hydroxide (4.4ml of a 1.15 mg/ml solution) was added. The mixture was then diluted with water (40ml) and freeze-dried, giving the title compound (121mg) as a white powder.
NMR (δ) (DMSO$d_6$) 0.7-1.0 (14H, m), 1.0-1.8 (15H, m), 2.05-2.2 (2H, m), 2.3 (1H, m), 2.55-3.1 (6H, m), 3.6-3.9 (3H, m), 4.0-4.15 (2H, m), 4.15-4.4 (2H, m), 4.45-4.65 (2H, m), 6.9 (1H, 2 x d), 7.15-7.3 (5H, m), 7.4 (0.5H, d), 7.55 (0.5H, d), 7.6-7.9 (3H, m), 8.15-8.4 (2H, m).
M.S. (m/z) (FAB) (M+1) = 813, (M+23) = 835 (consistent with m.w. of free acid = 812).

Example 57

4-(S)-(5-(2-(Benzyloxycarbonylamino)ethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide hemihydrate

This material was formed from (5-(2-(benzyloxycarbonylamino)ethanesulphonyl)-2,3-dihydrobenzofuran-3-yl)acetic acid (72mg), following the procedure of Example 21. The crude product was purified by chromatography on silica gel using methanol/chloroform (0-5% methanol, gradient). This gave the title compound (95mg) as a white solid.
NMR (δ) (DMSO$d_6$) 0.7-1.0 (13H, m), 1.0-1.4 (7H, m), 1.4-1.8 (9H, m), 2.1 (2H, m), 2.3 (1H, m), 2.7 (2H, m), 2.8-2.95 (3H, m), 3.05 (1H, m), 3.2-3.4 (m), 3.7 (1H, m), 3.75-3.9 (2H, m), 3.95 (0.5H, dd), 4.25 (0.5H, dd), 4.3 (1H, q), 4.45 (O.5H, t), 4.55 (0.5 H, t), 4.55-4.7 1H, m), 4.9 (1H, m), 5.0 (2H, s), 6.95 (1H, m), 7.1-7.4 (11H, m), 7.6-7.8 (3H, m), 8.2 (2H, m).
Analysis: $C_{50}H_{69}N_5O_{10}S$. 0.5$H_2O$ requires C, 63.8; H, 7.5; N, 7.4%. Found: C, 64.0; H, 7.5; N, 7.5%.
M.S. (m/z) (FAB) (M+1) = 932 (consistent with m.w. = 931).

Example 58

4-(S)-(2,3-Dihydro-2-oxo-1H-benzimidazole-1-acetyl)-L-phenylalanyl-(S)-leucyl)amino-5-cyclehexyl-3-(S)-hydroxypentanoic acid isobutylamide sesquihydrate

This material was formed from 2,3-dihydro-2-oxo-1H-benzimidazole-1-acetic acid (38mg), following the procedure of Example 21. The crude product was purified by chromatography on silica gel using methanol/chloroform (0-10% methanol, gradient). This gave the title compound (56mg) as a white solid.
NMR (δ) (DMSO$d_6$) 0.7-1.0 (14H,m), 1.0-1.8 (15H,m), 2.1 2H,m), 2.7-2.95 (3H,m), 2.95-3.1 (1H,m), 3.8 (2H,m), 4.2-4.5 (3H,m), 4.6 (1H,m), 4.8-4.9 (1H,m), 6.5 (d) and 6.55 (d) (1H), 6.85 (1H,m), 6.95 (2H,m), 7.25 (5H,m), 7.35 (d) and 7.6 (d) (1H), 7.7 (1H,m), 8.2 (1H,m), 8.5 (1H,m), 10.8 (1H,s).
Analysis: $C_{39}H_{56}N_6O_6$.1.5$H_2O$ requires C,64.0; H,8.1;
Found: C,64.2; H,7.8; N,11.3%.
M.S (m/z) (FAB) (M+1) = 705 (consistent with m.w. = 704).

Example 59

4-(S)-(2,3-Dihydro-2-oxo-3H-benzoxazole-3-acetyl-(S)-phenylalanyl-(S)-leucyl)amino)-5-cyclohexyl-3-(S)-hydroxypentanoic acid isobutylamide hemihydrate

This material was formed from 2,3-dihydro-2-oxo-3H-benzoxazole-3-acetic acid (36mg), following the procedure of Example 21. The crude product was purified by chromatography on silica gel using methanol/chloroform (0-10% methanol, gradient). This gave the title compound (73mg) as a light yellow solid.
NMR (δ) (DMSO$d_6$) 0.7-1.0 (14H,m), 1.0-1.8 (15H,m), 2.1 (2H,m), 2.6-3.1 (4H,m), 3.75-3.9 (2H,m), 4.25-4.4 (2H,m), 4.5 (1H,m), 4.6 (1H,m), 4.8-4.9 (1H,m), 6.7 (1H,m), 7.1 (2H,m), 7.15-7.4 (7H,m), 7.7 (1H,t), 8.25 (1H,d), 8.65 (1H,d).
Analysis: $C_{39}H_{55}N_5O_7$.0.5$H_2O$ requires C,65.5; H,7.9; N,9.8%%. Found: C,65.8; H,8.0; N,9.8%.
M.S. (m/z) (FAB) (M+1) = 706 (consistent with m.w. = 705.

Example 60

4-(S)-((6-Aminomethyl-2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide bis(trifluoroacetate) sesquihydrate

4-(S)-((6-(tert-Butoxycarbonylaminomethyl)-2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide (130 mg) was dissolved in dichloromethane (4 ml) and trifluoroacetic acid (4 ml).After standing for 30 min, solvents were removed, and the gum was triturated with ether. Filtration then gave the title compound (131 mg) as a white solid.
NMR (δ) ($CD_3OD$) 0.8-1.05 (8H,m), 1.1-1.5 (6H,m), 1.5-1.9 (6H,m), 2.2-2.4 (2H,m), 2.6 (1H,m), 2.8-3.1

(5H,m), 3.15 (2H,m), 3.45-3.7 (1H,m), 3.85-4.05 (3H,m), 4.2 (2H,m), 4.5-4.7 (m), 7.0-7.4 (6H,m), 7.5-7.6 (1H,m), 7.7 (1H,m), 7.8 (1H,m), 8.75 (1H,s).
Analysis: $C_{41}H_{57}N_7O_7S.2(C_2HO_2F_3).1.5H_2O$ requires C,51.6; H,6.0; N,9.4%. Found: C,51.7; H,5.9; N,9.3%.
M.S. (m/z) (FAB) (M+1) = 792 (consistent with m.w. of free base = 791).

Example 61

4-(S)-(4-(2,3-Dihydro-1,1-dioxobenzothiophene-3-yl-butanoyl-(S)-phenyllanyl-(S)-leucyl)amino-5-cyclohexyl-3-(S)-hydroxvoentanoic acid isobutylamide

This material was formed from 4-(2,3-dihydro-1,1-dioxo benzothiophene-3-yl)butanoic acid (0.058g) following the procedure of Example 21. This gave the title compound (0.11g) as a white solid.
NMR (δ) ($CD_3OD$) 0.6-1.0 (13H,m), 1.0-1.3 (6H,m), 1.3-1.7 (11H,m), 2.1-2.2 (3H,m), 2.9 (2H,d), 3.9 (2H,d), 7.1-7.2 (5H,m), 7.3-7.5 (2H,m), 7.5-7.7 (2H,m).
M.S. (m/z) (FAB) (M+1) = 767 (consistent with m.w. = 766).

Example 62

4-(S)-((3-(2,3-Dihydro-2-oxo-3H-benzoxazol-3-yl)propanoyl)-(S)-phenylalanyl-(S)-leucyl)amino-5-cyclohexyl-3-(S)-hydroxypentanoic acid isobutylamide hydrate

This material was formed from 3-(2,3-dihydro-2-oxo-3H-benzoxazol-3-yl)propanoic acid (39 mg), following the procedure of Example 21. The crude product was purified by chromatography on silica gel using methanol/chloroform (0-5% methanol, gradient). This gave the title compound (93 mg) as an orange solid.
NMR (δ) ($CDCl_3$) 0.7-1.0 (14H,m), 1.0-1.8 (15H,m), 2.0-2.2 (2H,m), 2.55 (2H,t), 2.7 (1H,m), 2.8-3.0 (3H,m), 3.75-3.95 (4H,m), 4.25 (1H,m), 4.55 (1H,m), 4.8 (d) and 4.9 (d) (1H), 7.1-7.25 (8H,m), 7.3 (2H,m), 7.7 (1H,m), 8.15 (1H,d), 8.25 (d) and 8.35 (d) (1H).
Analysis: $C_{40}H_{57}N_5O_7.H_2O$ requires C,65.1; H,8.1; N,9.5%. Found: C,65.0; H,7.9; N,9.2%.
M.S. (m/z)(FAB) (M+1)=720 (consistent with m.w.=719).

Example 63

4-(S)-((5-Cyano-2,3-dihydro-2-oxo-1H-indol-3-yl)acetyl(S)-phenylalanyl-(S)-leucyl)amino-5-cyclohexyl-3-(S)-hydroxypentanoic acid isobutylamide hydrate

To a stirred, ice-cooled solution of 4-(S)-((S)-phenylalanyl-(S)-leucyl)amino-5-cyclohexyl-3-(S)-hydroxypentanoic acid isobutylamide (0.31g), 5-cyano-2,3-dihydro-2-oxo-1H-indole-3-acetic acid (0.126g) and 1-hydroxybenzotriazole (0.079g) in dry DMF (5 ml) was added 1-(3-dimethylaminopropyl)-3-ethylcarbodiimide hydrochloride (0.112g) in one portion. Cooling was removed and stirring continued for 18h. The reaction mixture was poured into water and extracted into ethyl acetate (3x75 ml). Combined extracts were washed with 10% citric acid, water, saturated sodium hydrogen carbonate solution, brine, dried ($Na_2SO_4$) and the solvent removed in vacuo. The residue was purified by chromatography on silica gel using 0-6% methanol/chloroform to afford the title compound (0.25g) as a light yellow foam.
NMR (δ) ($DMSOd_6$) 0.7-1.9 (29H,m), 2.0-2.2 (2H,m), 2.6-3.1 (5H,m), 3.5-3.9 (3H,m), 4.2-4.4 (1H,m), 4.4-4.6 (1H,m), 4.8-4.95 (1H,m), 6.8-7.5 (8H,m), 7.5-7.8 (2H,m), 8.1-8.4 (2H,m), 10.8-11.0 (1H,m).
Analysis: $C_{41}H_{56}N_6O_6.H_2O$ requires C,65.9; H,7.8; N,11.2%. Found: C,65.7; H,7.6; N,10.8%.
M.S. (m/z)(FAB) (M+1)=729 (consistent with m.w.=728).

Example 64

4-(S)-((2,3-Dihydro-1,1-dioxobenzothiophene-3-yl)acetyl-(S)-phenylalanyl-(S)-β-(4-aminopyrazol-1-yl)alanyl)-amino-5-cyclohexyl-3-(S)-hydroxypentanoic acid isobutylamide

This material was formed from 4-(S)-((2,3-dihydro-1,1-dioxobenzothiophene-3-yl)acetyl-(S)-phenylalanyl-(S)-β-(4-(tert-butoxycarbonylamino)pyrazol-1-yl)alanyl)amino-5-cytertexyl-3-(S)-hydroxypentanoic acid isobutylamide (0.05g) following the procedure of Description 30. This gave the title compound (0.04g) as a white solid.

NMR (δ) ($DMSOd_6$) 0.7-1.9 (20H,m), 2.1 (2H,d), 2.4 (1H,dd), 2.6-3.1 (6H,m), 3.7-3.9 (3H,m), 4.2-4.3 (1H,dd), 4.3-4.45 (1H,dd), 4.6-4.9 (3H,m), 7.1-7.8 (12H,m), 8.3 (1H,t), 8.5 (1H,m).

M.S. (m/z) (FAB) (M+1) = 778 (consistent with m.w. = 777).

Example 65

4-(S)-((5-Aminomethyl-2,3-dihydrobenzofuran-3-yl)acetyl-(S)-phenylalanyl-(S)-β-(pyrazol-1-yl)alanyl)amino-5-cyclohexyl-3-(S)-hydroxypentanoic acid isobutylamide trifluoroacetate sesquihydrate

This material was formed from 4-(S)-((5-(tert-butoxycarbonylaminomethyl)-2,3-dihydrobenzofuran-3-yl)-acetyl-(S)-phenylalanyl-(S)-β-(pyrazol-1-yl)alanyl)amino-5-cyclohexyl-3-(S)-hydroxypentanoic acid isobutylamide hydrate (0.126g) following the procedure of Description 30. This gave the title compound (0.098g).

NMR (δ) ($DMSOd_6$) 0.7-2,3 (24H,m), 2.5-3.1 (6H,m), 3.3-4.9 (11H,m), 6.15 (1H,m), 6.7 (1H,m), 7.0-7.2 (7H,m), 7.3 (1H,m), 7.6 (2H,m), 7.9 (3H,b), 8.2-8.4 (2H,m).

Analysis: $C_{43}H_{58}F_3N_7O_8.1.5H_2O$ requires C,58.4; H,6.9; N,11.1%. Found: C,58.2; H,6.6; N,10.9%.

M.S. (m/z) (FAB) (M+1) = 744 (consistent with m.w. of free base = 743).

Example 66

4-(S)-((5-Formyl-2,3-dihydrobenzofuran-3-yl)acetyl-(S)-phenylalanyl-(S)-histidyl)amino-5-cyclohexyl-3-(S)-hydroxypentanoic acid isobutylamide sesquihydrate

This material was formed from (5-formyl-2,3-dihydrobenzofuran-3-yl)acetyl-(S)-phenylalanyl-(S)-histidine (0.9g) and 4-(S)-amino-5-cyclohexyl-3-(S)-hydroxypentanoic acid isobutylamide (0.5g) following the procedure of Example 13. This gave the title compound (0.30g) as a white solid.

NMR (6) ($CDCl_3$) 0.7-1.9 (20H,m), 2.0-3.4 (10H,m), 3.6-4.4 (4H,m), 4.4-4.7 (3H,m), 6.65-7.7 (12H,m), 9.7 (1H,m).

Analysis: $C_{41}H_{54}N_6O_7.1.5H_2O$ requires C,64.0; H,7.5; N,10.9%. Found: C,64.1; H,7.4; N,10.8%.

M.S. (m/z) (FAB) (M+1) = 743 (consistent with m.w. = 742).

Example 67

4-(S)-((5-Aminoethyl-2,3-dihydro-2-oxo-1H-indol-3-yl)acetyl-(S)-phenylalanyl-(S)-leucyl)amino-5-cyclohexyl-3-(S)-hydroxypentanoic acid isobutylamide hydrate

This material was prepared as in E20a from the corresponding 5-cyano compound E63.

Analysis: $C_{41}H_{60}N_6O_6.2.5H_2O$ requires C, 63.3; H: 8.4, N: 10.8. Found: C, 63.2; H: 8.1, N: 10.8

Example 68

4-(S)-(1,3-Dihydro-3-oxobenzo[c]furan-1-ylacetyl)-(S)-phenylalanyl-(S)-leucyl)amino-5-cyclohexyl-3-(S)-hydroxypentanoic acid isobutylamide hydrate

This material was formed from 1,3-dihydro-1-oxobenzo[c]furan-3-yl-acetic acid (36 mg), following the procedure of Example 21. The crude product was purified by chromatography on silica gel using methanol/chloroform (0-5% methanol, gradient). This gave the title compound (83 mg) as a white solid.
NMR (δ) ($CDCl_3$) 0.7-1.0 (14H,m), 1.0-1.8 (15H,m), 2.05-2.2 (2H,m), 2.6-2.95 (5H,m), 3.05 (1H,td), 3.7-3.9 (2H,m), 4.35 (1H,m), 4.6 (0.5H, m), 4.7 (0.5H,m), 4.8-4.95 (1H,m), 5.8 (1H,t), 7.15-7.4 (6H,m), 7.4-7.6 (2H,m), 7.6-7.75 (2H,m), 7.8 (1H,m), 8.15-8.45 (2H,m).
Analysis: $C_{40}H_{56}N_4O_7.H_2O$ requires C,66.5; H,8.1; N,7.8%. Found: C,66.6; H.8.0; N,7.8%.
M.S. (m/z) (FAB) (M+1) = 705 (consistent with m.w. = 704).

Biological Data

In vitro human renin inhibition

Renin inhibitory activity was estimated as the percentage change in renin activity in human plasma in the presence and absence of compound. The source of plasma was blood taken from healthy volunteers. Renin activity was defined by the difference in angiotensin I levels between two halves of a sample, one incubated at 37° and the other at 4° for 2 h. Angiotensin I levels were measured using a $^{125}I$- angiotensin I radioimmunoassay kit (NEN/DuPont, Stevenage). Results were calculated as the mean of at least two, duplicate determinations and $IC_{50}$ values were calculated by linear regression analysis of at least three concentrations of compound.

The results were as follows:

| Compound | $IC_{50}$(x$10^{-8}$M) | Compound | $IC_{50}$(x$10^{-8}$M) |
|---|---|---|---|
| E 3 | 5 | E31a | 0.8 |
| E 5 | 6 | E36a | 10 |
| E 6 | 3 | E39 | 4 |
| E 7 | 5 | E43 | 0.8 |
| E 9 | 5 | E45 | 0.9 |
| E12 | 1 | E50 | 1 |
| E20a | 3 | E56 | 6 |
| E23 | 47 | E60 | 4 |
| E29 | 1 | | |

The remaining compounds exemplified also showed activity in this test.

## Claims

1. A compound of formula (I), or a pharmaceutically acceptable salt thereof:

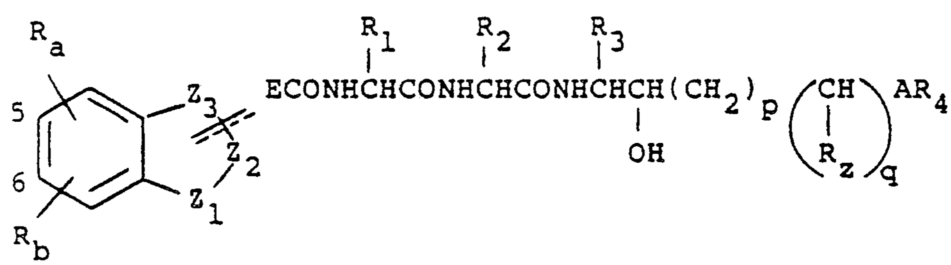

(I)

wherein
$Z_1$, $Z_2$, $Z_3$ and the carbon atoms to which $Z_1$ and $Z_3$ are attached, form a 5-membered non-aromatic heterocyclic ring;
E is absent or is $(CH_2)_n$ or $CH(CH_2)_{n-1}$ wherein n is 1 to 4;

A is -CONH-, -NHCO-, -COO-, $-S(O)_r-$ wherein r is 0, 1 or 2, or $-CH_2-$;
p is 0, 1 or 2;
q is 0 or 1;
$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl or $C_{3-8}$ cycloalkyl;
$R_4$ is $C_{1-6}$ alkyl or $C_{3-8}$ cycloalkyl; and
the dashed line represents an optional bond (when E is present).

2. A compound according to claim 1 wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, and one of $Z_2$ and $Z_3$, is $CH_2$, CH or C when the other is S or O, or is $CH_2$, CH, C, NR or N when the other is SO or $SO_2$; or $Z_2$ is SO, $SO_2$ or C=O when one of $Z_1$ and $Z_3$ is N and the other is O, S or NR; or $Z_1$ is CO, $Z_2$ is O and $Z_3$ is CH.

3. A compound according to claim 2 wherein $Z_1$ is $SO_2$ or O, $Z_2$ is $CH_2$, $Z_3$ is CH and E is attached at $Z_3$.

4. A compound according to any one of claims 1 to 3 wherein $R_a$ and $R_b$ are selected from halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy.

5. A compound according to claim 4 wherein one of $R_a$ and $R_b$ is hydrogen and the other is 5- or 6-$CH_2NH_2$ or $CO_2H$, 5-$CH_2NHCH_2CO_2H$ or 5-$SO_2CH_2CO_2H$.

6. A compound according to any one of claims 1 to 5 wherein R9 is phenyl or naphthyl.

7. A compound according to any one of claims 1 to 6 wherein the amino acid residue containing $R_2$ is Leu, β-pyrazolylalanine or His.

8. A compound according to claim 1 wherein
E is $(CH_2)_n$ wherein n is 0, 1 or 2;
$Z_1$ is O, S, SO, $SO_2$ or NR wherein R is hydrogen or $C_{1-6}$ alkyl;
$Z_2$ is $CR_xR_y$ wherein $R_x$ is hydrogen and $R_y$ is hydrogen or $R_y$ is attached to E in formula (I), or $R_x$ and $R_y$ together are an oxo group;
$Z_3$ is $CH_2$ or is CH attached to E in formula (I);
$R_{11}$ is $C_{1-6}$ alkyl, $C_{3-8}$ cycloalkyl, or optionally substituted aryl or heteroaryl; and
the remaining variables are as defined in respect of formula (I).

9. A compound according to claim 1 for the preparation of formula (IA) or a pharmaceutically acceptable salt thereof:

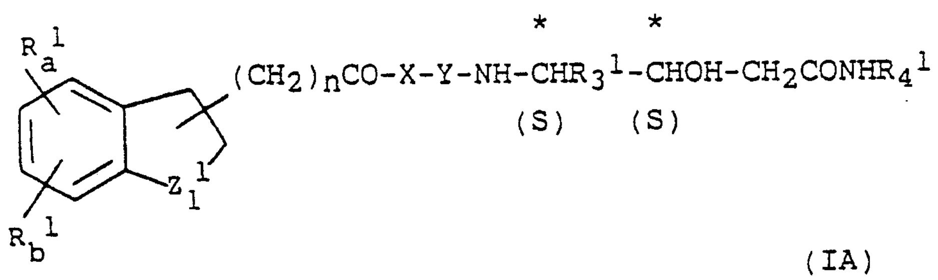

(IA)

wherein
X is Phe or NAla;
Y is Leu, Ile, Nle or His;
$Z_1^1$ is O, S, SO or $SO_2$;

n is 0, 1 or 2;
$R_a'$ and $R_b'$ are independently selected from hydrogen, halogen, $C_{1-6}$ alkyl, $C_{1-6}$ alkoxy, nitro, cyano, $SO_3H$, CHO, $C_{1-6}$ alkyl substituted by hydroxy or amino optionally substituted by a $C_{1-7}$ alkanoyl group or by one or two $C_{1-6}$ alkyl groups, or a group $NR_5{}^1R_6{}^1$, $SO_2NR_5{}^1R_6{}^1$, $S(O)_mR_5{}^1$, $CO_2R_5{}^1$, $NHCONR_5{}^1R_6{}^1$ or $NHCOR_5{}^1$ wherein $R_5{}^1$ and $R_6{}^1$ are independently selected from hydrogen or $C_{1-6}$ alkyl and m is 0, 1 or 2;
$R_3{}^1$ is cyclohexylmethyl; and
$R_4{}^1$ is $C_{4-5}$ alkyl.

10. A compound according to any one of claims 1 to 9, in which the right hand sequence in formulae (I) and (IA) is 4-(S)-amino-5-cyclohexyl-3-(S)-hydroxypentanoic acid isobutylamide (ACHPAA).

11. A compound according to any one of claims 1 to 10 wherein E is $(CH_2)_n$ wherein n is 1, 2 or 3 and E is attached at the 3-position with respect to $Z_1$.

12. A compound selected from the group consisting of:
4-(S)-((2,3-dihydrobenzofuran-2-carbonyl)-(S)-phenylalanyl-(S)-leucyl)amino-5-cyclohexyl-3-(S)-hydroxypentanoic acid isobutylamide,
4-(S)-(3-(2,3-dihydrobenzofuran-2-yl)propanoyl-(S)-phenylalanyl-(S)-leucyl)amino-5-cyclohexyl-3-(S)-hydroxypentanoic acid isobutylamide,
4-(S)-((7-methoxy-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-nitro-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-amino-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-ureido-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((7-nitro-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(methylaminosulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydrobenzothiophene-2-carbonyl)-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-2-carbonyl))-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((5-formyl-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(hydroxymethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(methoxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-carboxy-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-(2,3-dihydro-1,1-dioxobenzothiophene-3-yl)acetyl-(S)-phenylalanyl-(S)-leucyl)amino-5-cyclohexyl-3-(S)-hydroxypentanoic acid 3-methylbutylamide,
4-(S)-((5-cyano-2,3-dihydrobenzofuran-3-yl)acetyl-(S)phenylalanyl-(S)-leucyl)amino-5-cyclohexyl-3-(S)-hydroxypentanoic acid isobutylamide,
4-(S)-((5-(aminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(benzyloxycarbonyl)-2,3-dihydrobenzofuran-3-yl)acetyl-(S)-phenylalanyl-(S)-leucyl) amino-5-cyclohexyl-3-(S)-hydroxypentanoic acid isobutylamide,
4-(S)-((5-(methanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(benzyloxycarbonyl)-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((5-carboxy-2,3-dihydrobenzofuran-3-yl)acetyl-(S)-phenylalanyl-(S)-histidyl)amino-5-cyclohexyl-3-(S)-hydroxypentanoic acid isobutylamide,

4-(S)-((2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide,
4-(S)-((5-(N-(2-hydroxyethyl)iminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((5-(N-(2-hydroxyethyl)aminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((6-nitro-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-amino-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-2-yl)acetyl-(S)-phenylalanyl-(S)-leucyl)amino-5-cyclohexyl-3-(S)-hydroxypentanoic acid isobutylamide,
4-(S)-((2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-((5-(ethoxycarbonylmethylaminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((5-(benzyloxycarbonylmethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(carboxymethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(pyrazol-1-yl)alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(4-methylpyrazol-1-yl)alanyl)-amino-5-cyclohexyl-3-(S)-hydroxypentanoic acid isobutylamide,
4-(S)-((5-nitro-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((5-amino-2,3-dihydrobenzofuran-3-yl)acetyl-(S)phenylalanyl-(S)-histidyl)amino-5-cyclohexyl-3-(S)-hydroxypentanoic acid isobutylamide,
4-(S)-(2,3-dihydro-3-oxo-1H-isoindol-1-acetyl)-(S)-phenylalanyl-(S)-leucyl)amino-5-cyclohexyl-3-(S)-hydroxypentanoic acid isobutylamide,
disulphide of 4-(S)-((5-mercapto-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(tert-butoxycarbonylmethanesulphonyl)-2,3-dihydrobenzofuran-3-yl)acetyl)-(S)-phenylalanyl-(S)-leucyl)amino-5-cyclohexyl-3-(S)-hydroxypentanoic acid isobutylamide,
4-(S)-(3-(2,3-dihydro-1,1-dioxobenzothiophene-3-yl)propanoyl-(S)-phenylalanyl-(S)-leucyl)amino-5-cyclohexyl-3-(S)-hydroxypentanoic acid isobutylamide,
4-(S)-((5-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((2,3-dihydro-3-oxo-1H-isoindol-1-ylidine)-acetyl-(S)-phenylalanyl-(S)-leucyl)amino-5-cyclohexyl -3-(S)-hydroxypentanoic acid isobutylamide,
4-(S)-((2,3-dihydro-1,1-dioxobenzothiophene-3-yl)acetyl-(S)-phenylalanyl-(S)-β-(3,5-dimethylpyrazol-1-yl)-alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(tert-butoxycarbonylamino)-alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-aminoalanyl)amino-5-cyclohexyl-3-(S)-hydroxypentanoic acid isobutylamide,
4-(S)-((5-aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-(3-(2,3-dihydro-1,1-dioxobenzothiophene-2-yl)propanoyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(acetylamino)alanyl))amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(2-oxopyrrolidin-1-yl)alanyl)-amino-5-cyclohexyl-3-(S)-hydroxypentanoic acid isobutylamide,

4-(S)-((5-cyano-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((6-(tert-butoxycarbonylaminomethyl)-2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide,
4-(S)-((5-(carboxymethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-(5-(2-(benzyloxycarbonylamino)ethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-(2,3-dihydro-2-oxo-1H-benzimidazole-1-acetyl)-L-phenylalanyl-(S)-leucyl)amino-5-cyclohexyl-3-(S)-hydroxypentanoic acid isobutylamide,
4-(S)-(2,3-dihydro-2-oxo-3H-benzoxazole-3-acetyl-(S)-phenylalanyl-(S)-leucyl)amino)-5-cyclohexyl-3-(S)-hydroxypentanoic acid isobutylamide,
4-(S)-((6-aminomethyl-2,3-dihydro-1,1-dioxobenzothio-phene-3-yl)acetyl-(S)-phenylalanyl-(S)-histidyl)amino-5-cyclohexyl-3-(S)-hydroxypentanoic acid isobutylamide,
4-(S)-(4-(2,3-dihydro-1,1-dioxobenzothiophene-3-yl-butanoyl-(S)-phenylalanyl-(S)-leucyl)amino-5-cyclohexyl-3-(S)-hydroxypentanoic acid isobutylamide,
4-(S)-((3-(2,3-dihydro-2-oxo-3H-benzoxazol-3-yl)propanoyl)-(S)-phenylalanyl-(S)-leucyl)amino-5-cyclohexyl-3-(S)-hydroxypentanoic acid isobutylamide,
4-(S)-((5-cyano-2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(4-aminopyrazol-1-yl)alanyl)-amino-5-cyclohexyl-3-(S)-hydroxypentanoic acid isobutylamide,
4-(S)-((5-aminomethyl-2,3-dihydrobenzofuran-3-yl)-acetyl-(S)-phenylalanyl-(S)-β-(pyrazol-1-yl)alanyl)amino-5-cyclohexyl-3-(S)-hydroxypentanoic acid isobutylamide,
4-(S)-((5-formyl-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((5-aminoethyl-2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-(1,3-dihydro-3-oxobenzo[c]furan-1-yl-acetyl)-(S)-phenylalanyl-(S)-leucyl)amino-5-cyclohexyl-3-(S)-hydroxypentanoic acid isobutylamide,
and pharmaceutically acceptable salts of any one of the foregoing compounds.

13. A process for the preparation of a compound of formula (I) or a pharmaceutically acceptable salt thereof which comprises reacting a reagent of the formula (III):

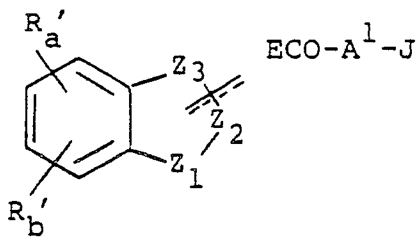

(III)

wherein $R_a'$ and $R_b'$ are $R_a$ and $R_b$ respectively or groups or atoms convertible thereto; $A^1$ is absent or represents an appropriate amino acid or dipeptide unit; J is OH or a leaving group; and the remaining variables are as hereinbefore defined; with a reagent of the formula (IV):

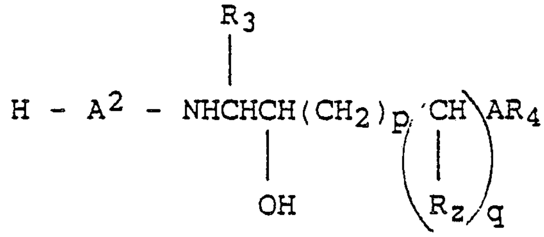

(IV)

$A^2$ is absent or represents an appropriate amino acid or dipeptide unit, such that $A^1 + A^2$ is $-NHCHR_1CONHCHR_2CO-$, and the remaining variables are as defined in claim 1; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'$ $R_b'$ to $R_a$ and/or $R_b$ and/or forming a pharmaceutically acceptable salt thereof.

14. A pharmaceutical composition comprising a compound according to any one of claims 1 to 12, and a pharmaceutically acceptable carrier.

15. A compound according to any one of claims 1 to 12 for use as an active therapeutic substance.

16. A compound according to any one of claims 1 to 12 for use in the treatment of hypertension.

17. Use of a compound according to any one of claims 1 to 12 in the manufacture of a medicament for use in the treatment of hypertension.

Claims for the following Contracting State: ES

1. A process for the preparation of a compound of formula (I), or a pharmaceutically acceptable salt thereof:

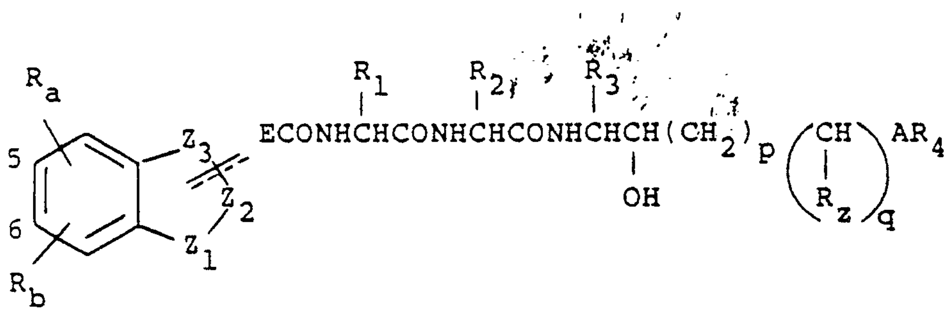

(I)

wherein

$Z_1$, $Z_2$, $Z_3$ and the carbon atoms to which $Z_1$ and $Z_3$ are attached, form a 5-membered non-aromatic heterocyclic ring;

E is absent or is $(CH_2)_n$ or $CH(CH_2)_{n-1}$ wherein n is 1 to 4;

A is -CONH-, -NHCO-, -COO-, $-S(O)_r-$ wherein r is 0, 1 or 2, or $-CH_2-$;

p is 0, 1 or 2;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino; $R_3$ is $CH_2R_{12}$ wherein $R_{12}$ is $C_{1-6}$ alkyl or $C_{3-8}$ cycloalkyl;

$R_4$ is $C_{1-6}$ alkyl or $C_{3-8}$ cycloalkyl; and

the dashed line represents an optional bond (when E is present); which comprises reacting a reagent of the formula (III):

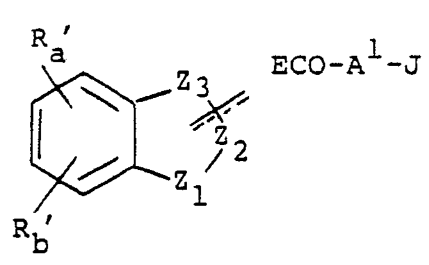

(III)

wherein $R_a'$ and $R_b'$ are $R_a$ and $R_b$ respectively or groups or atoms convertible thereto; $A^1$ is absent or represents an appropriate amino acid or dipeptide unit; J is OH or a leaving group; and the remaining variables are as hereinbefore defined; with a reagent of the formula (IV):

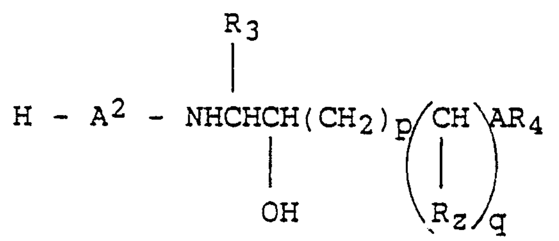

(IV)

$A^2$ is absent or represents an appropriate amino acid or dipeptide unit, such that $A^1$ + $A^2$ is $-NHCHR_1CONHCHR_2CO-$, and the remaining variables are as defined in claim 1; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'$ $R_b'$ to $R_a$ and/or $R_b$ and/or forming a pharmaceutically acceptable salt thereof.

2. A process according to claim 1 wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, and one of $Z_2$ and $Z_3$, is $CH_2$, CH or C when the other is S or O, or is $CH_2$, CH, C, NR or N when the other is SO or $SO_2$; or $Z_2$ is SO, $SO_2$ or C=O when one of $Z_1$ and $Z_3$ is N and the other is O, S or NR; or Z is CO, $Z_2$ is O and $Z_3$ is CH.

3. A process according to claim 2 wherein $Z_1$ is $SO_2$ or O, $Z_2$ is $CH_2$, $Z_3$ is CH and E is attached at $Z_3$.

4. A process according to any one of claims 1 to 3 wherein $R_a$ and $R_b$ are selected from halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy.

5. A process according to claim 4 wherein one of $R_a$ and $R_b$ is hydrogen and the other is 5- or 6-$CH_2NH_2$ or $CO_2H$, 5-$CH_2NHCH_2CO_2H$ or 5-$SO_2CH_2CO_2H$.

6. A process according to any one claims 1 to 5 wherein $R_9$ is phenyl or naphthyl.

7. A process according to any one of claims 1 to 6 wherein the amino acid residue containing $R_2$ is Leu, β-pyrazolylalanine or His.

8. A process according to claim 1 wherein
E is $(CH_2)_n$ wherein n is 0, 1 or 2;
$Z_1$ is O, S, SO, $SO_2$ or NR wherein R is hydrogen or $C_{1-6}$ alkyl;
$Z_2$ is $CR_xR_y$ wherein $R_x$ is hydrogen and $R_y$ is hydrogen or $R_y$ is attached to E in formula (I), or $R_x$ and $R_y$ together are an oxo group;
$Z_3$ is $CH_2$ or is CH attached to E in formula (I);
$R_{11}$ is $C_{1-6}$ alkyl, $C_{3-8}$ cycloalkyl, or optionally substituted aryl or heteroaryl; and
the remaining variables are as defined in respect of formula (I).

9. A process according to claim 1 for the preparation of formula (IA) or a pharmaceutically acceptable salt thereof:

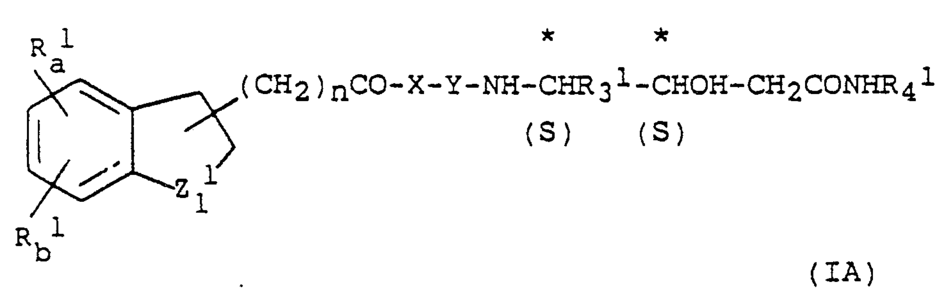

(IA)

wherein
X is Phe or NAla;

Y is Leu, Ile, Nle or His;
$Z_1$ is O, S, SO or $SO_2$;
n is 0, 1 or 2;
$R_a^1$ and $R_b^1$ are independently selected from hydrogen, halogen, $C_{1-6}$ alkyl, $C_{1-6}$ alkoxy, nitro, cyano, $SO_3H$, CHO, $C_{1-6}$ alkyl substituted by hydroxy or amino optionally substituted by a $C_{1-7}$ alkanoyl group or by one or two $C_{1-6}$ alkyl groups, or a group $NR_5^1R_6^1$, $SO_2NR_5^1R_6^1$, $S(O)_mR_5^1$, $CO_2R_5^1$, $NHCONR_5^1R_6^1$ or $NHCOR_5^1$ wherein $R_5^1$ and $R_6^1$ are independently selected from hydrogen or $C_{1-6}$ alkyl and m is 0, 1 or 2;
$R_3^1$ is cyclohexylmethyl; and
$R_4^1$ is $C_{4-5}$ alkyl.

10. A process according to any one of claims 1 to 9, which the right hand sequence in formulae (I) and (IA) is 4-(S)-amino-5-cyclohexyl-3-(S)-hydroxypentanoic acid isobutylamide (ACHPAA).

11. A process according to any one of claims 1 to 10 wherein E is $(CH_2)_n$ wherein n is 1, 2 or 3 and E is attached at the 3-position with respect to $Z_1$.

12. A process according to claim 1 for the preparation of:
4-(S)-((2,3-dihydrobenzofuran-2-carbonyl)-(S)-phenylalanyl-(S)-leucyl)amino-5-cyclohexyl-3-(S)-hydroxypentanoic acid isobutylamide,
4-(S)-(3-(2,3-dihydrobenzofuran-2-yl)propanoyl-(S)-phenylalanyl-(S)-leucyl)amino-5-cyclohexyl-3-(S)-hydroxypentanoic acid isobutylamide,
4-(S)-((7-methoxy-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-nitro-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-amino-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-ureido-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((7-nitro-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(methylaminosulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydrobenzothiophene-2-carbonyl)-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-2-carbonyl))-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene3-yl)acetyl-(S)-phenylalanyl-(S)-leucyl)amino-5-cyclohexyl-3-(S)-hydroxypentanoic acid isobutylamide,
4-(S)-((5-formyl-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(hydroxymethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(methoxycarbonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-carboxy-2,3-dihydrobenzofuran-3-yl)acetyl-(S)-phenylalanyl-(S)-leucyl)amino-5-cyclohexyl-3-(S)-hydroxypentanoic acid iscbutylamide,
4-(S)-(2,3-dihydro-1,1-dioxobenzothiophene-3-yl)acetyl-(S)-phenylalanyl-(S)-leucyl)amino-5-cyclohexyl-3-(S)-hydroxypentanoic acid 3-methylbutylamide,
4-(S)-((5-cyano-2,3-dihydrobenzofuran-3-yl)acetyl-(S)phenylalanyl-(S)-leucyl)amino-5-cyclohexyl-3-(S)-hydroxypentanoic acid isobutylamide,
4-(S)-((5-(aminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(benzyloxycarbonyl)-2,3-dihydrobenzofuran-3-yl)acetyl-(S)-phenylalanyl-(S)-leucyl) amino-5-cyclohexyl-3-(S)-hydroxypentanoic acid isobutylamide,
4-(S)-((5-(methanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(benzyloxycarbonyl)-2,3-dihydrobenzofuran-3-yl)acetyl-(S)-phenylalanyl-(S)-histidyl)amino-5-cyclohexyl-3-(S)-hydroxypentanoic acid isobutylamide,

4-(S)-((5-carboxy-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide,
4-(S)-((5-(N-(2-hydroxyethyl)iminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((5-(N-(2-hydroxyethyl)aminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((6-nitro-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-amino-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((6-aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-2-yl)acetyl-(S)-phenylalanyl-(S)-leucyl)amino-5-cyclohexyl-3-(S)-hydroxypentanoic acid isobutylamide,
4-(S)-((2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-((5-(ethoxycarbonylmethylaminomethyl)-2,3-dihydrobenzofuran-3-yl)acetyl-(S)-phenylalanyl-(S)-leucyl)-amino-5-cyclohexyl-3-(S)-hydroxypentanoic acid isobutylamide,
4-(S)-((5-(benzyloxycarbonylmethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(carboxymethylaminomethyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(pyrazol-1-yl)alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(4-methylpyrazol-1-yl)alanyl)-amino-5-cyclohexyl-3-(S)-hydroxypentanoic acid isobutylamide,
4-(S)-((5-nitro-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((5-amino-2,3-dihydrobenzofuran-3-yl)acetyl-(S)phenylalanyl-(S)-histidyl)amino-5-cyclohexyl-3-(S)-hydroxypentanoic acid isobutylamide,
4-(S)-(2,3-dihydro-3-oxo-1H-isoindol-1-acetyl)-(S)-phenylalanyl-(S)-leucyl)amino-5-cyclohexyl-3-(S)-hydroxypentanoic acid isobutylamide,
disulphide of 4-(S)-((5-mercapto-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-((5-(tert-butoxycarbonylmethanesulphonyl)-2,3-dihydrobenzofuran-3-yl)acetyl)-(S)-phenylalanyl-(S)-leucyl)amino-5-cyclohexyl-3-(S)-hydroxypentanoic acid isobutylamide,
4-(S)-(3-(2,3-dihydro-1,1-dioxobenzothiophene-3-yl)propanoyl-(S)-phenylalanyl-(S)-leucyl)amino-5-cyclohexyl-3-(S)-hydroxypentanoic acid isobutylamide,
4-(S)-((5-cyano-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-((2,3-dihydro-3-oxo-1H-isoindol-1-ylidine)-acetyl-(S)-phenylalanyl-(S)-leucyl)amino-5-cyclohexyl -3-(S)-hydroxypentanoic acid isobutylamide,
4-(S)-((2,3-dihydro-1,1-dioxobenzothiophene-3-yl)acetyl-(S)-phenylalanyl-(S)-β-(3,5-dimethylpyrazol-1-yl)-alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(tert-butoxycarbonylamino)-alanyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-aminoalanyl)amino-5-cyclohexyl-3-(S)-hydroxypentanoic acid isobutylamide,
4-(S)-((5-aminomethyl-2,3-dihydro-1,1-dioxobenzothiophene-3-yl)acetyl-(S)-phenylalanyl-(S)-leucyl)amino-5-cyclohexyl-3-(S)-hydroxypentanoic acid isobutylamide,
4-(S)-(3-(2,3-dihydro-1,1-dioxobenzothiophene-2-yl)propanoyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(acetylamino)alanyl))amino-5-cyclohexyl-3-(S)-hydroxypentanoic acid isobutylamide,

4-(S)-((2,3-dihydro-1,1-dioxobenzothiophene-3-yl)acetyl-(S)-phenylalanyl-(S)-β-(2-oxopyrrolidin-1-yl)alanyl)-amino-5-cyclohexyl-3-(S)-hydroxypentanoic acid isobutylamide,
4-(S)-((5-cyano-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((6-(tert-butoxycarbonylaminomethyl)-2,3-dihydro-1,1-dioxobenzothiophene-3-yl)acetyl-(S)-phenylalanyl-(S)-histidyl)amino-5-cyclohexyl-3-(S)-hydroxypentanoic acid isobutylamide,
4-(S)-((5-(carboxymethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-(5-(2-(benzyloxycarbonylamino)ethanesulphonyl)-2,3-dihydrobenzofuran-3-yl)acetyl-(S)-phenylalanyl-(S)-leucyl)amino-5-cyclohexyl-3-(S)-hydroxypentanoic acid isobutylamide,
4-(S)-(2,3-dihydro-2-oxo-1H-benzimidazole-1-acetyl)-L-phenylalanyl-(S)-leucyl)amino-5-cyclohexyl-3-(S)-hydroxypentanoic acid isobutylamide,
4-(S)-(2,3-dihydro-2-oxo-3H-benzoxazole-3-acetyl-(S)-phenylalanyl-(S)-leucyl)amino)-5-cyclohexyl-3-(S)-hydroxypentanoic acid isobutylamide,
4-(S)-((6-aminomethyl-2,3-dihydro-1,1-dioxobenzothio-phene-3-yl)acetyl-(S)-phenylalanyl-(S)-histidyl)amino-5-cyclohexyl-3-(S)-hydroxypentanoic acid isobutylamide,
4-(S)-(4-(2,3-dihydro-1,1-dioxobenzothiophene-3-yl-butanoyl-(S)-phenylalanyl-(S)-leucyl)amino-5-cyclohexyl-3-(S)-hydroxypentanoic acid isobutylamide,
4-(S)-((3-(2,3-dihydro-2-oxo-3H-benzoxazol-3-yl)propanoyl)-(S)-phenylalanyl-(S)-leucyl)amino-5-cyclohexyl-3-(S)-hydroxypentanoic acid isobutylamide,
4-(S)-((5-cyano-2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-((2,3-dihydro-1,1-dioxobenzothiophene-3-yl)acetyl-(S)-phenylalanyl-(S)-β-(4-aminopyrazol-1-yl)alanyl)-amino-5-cyclohexyl-3-(S)-hydroxypentanoic acid isobutylamide,
4-(S)-((5-aminomethyl-2,3-dihydrobenzofuran-3-yl)-acetyl-(S)-phenylalanyl-(S)-β-(pyrazol-1-yl)alanyl)amino-5-cyclohexyl-3-(S)-hydroxypentanoic acid isobutylamide,
4-(S)-((5-formyl-2,3-dihydrobenzofuran-3-yl)acetyl-(S)-phenylalanyl-(S)-histidyl)amino-5-cyclohexyl-3-(S)-hydroxypentanoic acid isobutylamide,
4-(S)-((5-aminoethyl-2,3-dihydro-2-oxo-1H-indol-3-yl)acetyl-(S)-phenylalanyl-(S)-leucyl)amino-5-cyclohexyl-3-(S)-hydroxypentanoic acid isobutylamide,
4-(S)-(1,3-dihydro-3-oxobenzo[c]furan-1-ylacetyl)-(S)-phenylalanyl-(S)-leucyl)amino-5-cyclohexyl-3-(S)-hydroxypentanoic acid isobutylamide,
or a pharmaceutically acceptable salt of any one of the foregoing compounds.

13. Use of a compound according to any one of claims 1 to 12 in the manufacture of a medicament for use in the treatment of hypertension.